(19) Europäisches Patentamt
European Patent Office
Office européen des brevets

(11) EP 4 259 286 B1

(12) EUROPEAN PATENT SPECIFICATION

(45) Date of publication and mention of the grant of the patent:
**15.07.2026 Bulletin 2026/29**

(21) Application number: **21839801.4**

(22) Date of filing: **07.12.2021**

(51) International Patent Classification (IPC):
***A61Q 13/00*** *(2006.01)* ***A61K 8/899*** *(2006.01)*
***A61K 8/898*** *(2006.01)* ***C11D 3/37*** *(2006.01)*
***C11D 3/50*** *(2006.01)*

(52) Cooperative Patent Classification (CPC):
**A61Q 13/00; A61K 8/898; A61K 8/899; C11D 3/3742; C11D 3/507;** C11D 2111/12

(86) International application number:
**PCT/US2021/072770**

(87) International publication number:
**WO 2022/126093 (16.06.2022 Gazette 2022/24)**

# (54) TREATMENT COMPOSITIONS WITH PRO-FRAGRANCE SILICONE POLYMERS THAT COMPRISE HETEROCYCLIC MOIETIES

BEHANDLUNGSZUSAMMENSETZUNGEN MIT DUFTSTOFFFÖRDERNDEN SILIKONPOLYMEREN MIT HETEROCYCLISCHEN EINHEITEN

COMPOSITIONS DE TRAITEMENT AVEC DES POLYMÈRES DE SILICONE PRO-PARFUM QUI COMPRENNENT DES FRAGMENTS HÉTÉROCYCLIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.12.2020 US 202063122960 P**
**25.03.2021 US 202163165841 P**

(43) Date of publication of application:
**18.10.2023 Bulletin 2023/42**

(73) Proprietor: **The Procter & Gamble Company**
**Cincinnati, OH 45202 (US)**

(72) Inventors:
- **NATOLI, Sean, Nicholas**
  **Cincinnati, Ohio 45202 (US)**
- **KLUESENER, Bernard, William**
  **Cincinnati, Ohio 45202 (US)**
- **PANANDIKER, Rajan, Keshav**
  **Cincinnati, Ohio 45202 (US)**
- **MIRACLE, Gregory, Scot**
  **Cincinnati, Ohio 45202 (US)**

(74) Representative: **P&G Patent Belgium UK**
**Temselaan 100**
**1853 Strombeek-Bever (BE)**

(56) References cited:
**WO-A1-2010/094356** **WO-A1-2012/084292**
**WO-A1-99/46318** **DE-A1- 102007 012 909**

- **"Delivery System Handbook for Personal Care and Cosmetic Products - Technology, Applications, and Formulations", 1 January 2005, WILLIAM ANDREW PUBLISHING, article ROBERT J PERRY: ""Pro-fragrant" Silicone Delivery Polymers", pages: 667 - 682, XP055390102, DOI: 10.1016/ B978-081551504-3.50037-7**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 259 286 B1

## Description

FIELD OF THE INVENTION

The present disclosure relates to treatment compositions that include pro-fragrance silicone polymers that include one or more heterocyclic moieties, where the heterocyclic moieties include a residue of an aldehyde-containing perfume raw material, a ketone-containing perfume raw material, or a mixture thereof. The present disclosure also relates to related premix compositions, pro-fragrance silicone polymers, and precursor silicone polymers. The present disclosure also relates to related methods of making and using such silicone polymers, premix compositions, and treatment compositions.

BACKGROUND OF THE INVENTION

Consumer product manufacturers and consumers alike desire treatment compositions that provide freshness benefits. To address this need, neat perfume raw materials (PRMs) have been utilized as a means to deliver freshness in consumer products such as is in the case of a laundry treatment application, where the delivery of such freshness may be achieved during pre-treatment, laundry cycle, rinse cycle, tumbling cycle or combinations thereof. While such applications do yield scented fabrics, they do not exhibit sustained delivery of scents or deposit with high efficiencies. As such, there is an effort to develop a composition capable of overcoming these challenges.

Pro-fragrance compounds can be useful to manufacturers of various treatment compounds, such as consumer products like liquid fabric enhancers, detergents, or dryer sheets. A "pro-fragrance compound" is a compound which may or may not be odoriferous in itself but which, upon external stimulation (e.g. light, temperature, or moisture), produces an odor which is characteristic of one or more of its released fragrance materials. Such compounds typically release fragrance materials (i.e., perfume raw materials) upon hydrolysis of a chemical bond, which can result in a desirable release profile, for example by delaying the release of the fragrance materials and with a combined scent character that may not be readily obtained from only a neat perfume oil.

Various silicon-based compounds are known in the art for being useful as pro-fragrance carriers, but they typically present certain challenges. For example, silicic acid esters are known for use as base molecules for pro-fragrance materials. However, the silicic acid ester bonds are often hydrolytically unstable, resulting in premature perfume release. Amino-modified silicones are also known to be useful as the backbones of pro-fragrance materials, but such materials may form imine bonds with the perfume raw materials, thereby leading to color and/or physical instabilities. WO99/46318A1 describes a pro-fragrance silicone polymers.

Therefore, there is still a need for improved silicone-based pro-fragrance materials, as well as stable and effective treatment compositions and related methods that employ them.

SUMMARY OF THE INVENTION

The present disclosure relates to pro-fragrance silicone polymers. The polymers include one or more heterocyclic moieties that include the residue of a perfume raw material. The pro-fragrance silicone polymer as defined in claim 1 herein includes a silicone backbone, an organic linker group that includes a carbon atom bonded to a silicon atom of the silicone backbone, and a heterocyclic moiety bonded to the organic linker group, where the heterocyclic moiety comprises five ring members, the ring members including: a first ring member that is a nitrogen atom; a second ring member that is a carbon atom, where the second ring member is part of a residue of a perfume raw material ("PRM"), where the PRM that formed the residue includes a moiety selected from an aldehyde moiety, a ketone moiety, and a combination thereof; a third ring member selected from the group consisting of an oxygen atom or a sulfur atom, preferably an oxygen atom, where the second ring member is directly bonded to the first ring member and to the third ring member. The present disclosure also relates to related precursor silicone polymers that are capable of forming such pro-fragrance silicone polymers.

The present disclosure provides treatment compositions as defined in claim 1 herein having a treatment adjunct and a pro-fragrance silicone polymer.

The present disclosure also provides liquid premix compositions, where the premix includes (a) a pro-fragrance silicone polymer as defined herein and optionally one or more free perfume raw materials, and/or (b) a precursor silicone polymer and a perfume raw material that comprises a moiety selected from an aldehyde moiety, a ketone moiety, or a combination thereof, where the precursor silicone polymer and the perfume raw material are capable of condensing to form a pro-fragrance silicone polymer as defined herein.

The present disclosure further relates to methods of making such treatment compositions and liquid premix compositions. The present disclosure provides a method of making a treatment composition, which method comprises the steps of: providing a base composition comprising a treatment adjunct; combining the base composition with one or more of the following: (a) a pro-fragrance silicone polymer as defined herein; (b) a precursor silicone polymer as defined herein

and a perfume raw material that comprises a moiety selected from the group consisting of an aldehyde moiety, a ketone moiety, and a combination thereof; or (c) a mixture thereof.

The present disclosure further relates to methods of treating a surface or an article with such treatment compositions, as well as surfaces or articles that include the pro-fragrance silicone polymer.

The present disclosure also provides the use of such pro-fragrance silicone polymers as a perfuming agent.

## DETAILED DESCRIPTION OF THE INVENTION

The present disclosure relates to pro-fragrance silicone polymers and certain precursor silicone polymers, as well as related premixes, treatment compositions, and methods of making and using such materials and compositions.

The pro-fragrance silicone polymers of the present disclosure comprise a heterocyclic moiety, such as an oxazolidine, that includes the residue of a perfume raw material. Such polymers may be formed by combining certain nitrogen-containing precursor silicone polymers with certain perfume raw materials, for example in a premix, and then treating an article or surface with a composition that includes the combination.

Without wishing to be bound by theory, it is believed that the heterocyclic moiety formed when a perfume raw material condenses with certain silicone precursors tend to result in improved color and chemical stability compared to pro-fragrance materials formed from known amino-modified silicones or silicic acid esters. For example, compared to imine bonds derived from such amino-modified silicones, the heterocyclic pro-fragrance materials described herein are less likely to form conjugated systems and lead to undesirable color changes. Further, it is believed that the heterocyclic moieties covalently appended to a silicone polymer (e.g., Si-CR') result in improved chemical stability upon storage, for example compared to the silicic acid ester bonds (e.g., Si-OR'), resulting in a longer release profile and more perfume released at later touchpoints. Furthermore, greater hydrophobicity of the precursor silicone polymers and resulting pro-fragrance silicones may result in improved PRM association and/or deposition. Additionally, it is believed that the perfume release profile can be tuned by the manufacturer by adjusting the polymer size and/or the substituent groups on the ring of the heterocyclic moiety. Additionally, it is believed that the heterocyclic moieties of the present disclosure are relatively stable under acidic conditions, for example a pH of from about 2 to about 4, such as those that are typical of liquid fabric softeners/conditioners.

The materials, compositions, and methods are discussed in more detail below.

As used herein, the articles "a" and "an" when used in a claim, are understood to mean one or more of what is claimed or described. As used herein, the terms "include," "includes," and "including" are meant to be non-limiting. The compositions of the present disclosure can comprise, consist essentially of, or consist of, the components of the present disclosure.

The terms "substantially free of" or "substantially free from" may be used herein. This means that the indicated material is at the very minimum not deliberately added to the composition to form part of it, or, preferably, is not present at analytically detectable levels. It is meant to include compositions whereby the indicated material is present only as an impurity in one of the other materials deliberately included. The indicated material may be present, if at all, at a level of less than 1%, or less than 0.1%, or less than 0.01%, or even 0%, by weight of the composition.

As used herein "consumer product" means baby care, personal care, fabric & home care, family care, feminine care, health care, snack and/or beverage products or devices intended to be used or consumed in the form in which it is sold, and not intended for subsequent commercial manufacture or modification. Such products include but are not limited to diapers, bibs, wipes; products for and/or methods relating to treating hair (human, dog, and/or cat), including, bleaching, coloring, dyeing, conditioning, shampooing, styling; deodorants and antiperspirants; personal cleansing; cosmetics; skin care including application of creams, lotions, and other topically applied products for consumer use; and shaving products, products for and/or methods relating to treating fabrics, hard surfaces and any other surfaces in the area of fabric and home care, including: air care, car care, dishwashing, fabric conditioning (including softening), laundry detergency, laundry and rinse additive and/or care, hard surface cleaning and/or treatment, and other cleaning for consumer or institutional use; products and/or methods relating to bath tissue, facial tissue, paper handkerchiefs, and/or paper towels; tampons, feminine napkins; products and/or methods relating to oral care including toothpastes, tooth gels, tooth rinses, denture adhesives, tooth whitening; over-the-counter health care including cough and cold remedies, pain relievers, RX pharmaceuticals, pet health and nutrition, and water purification.

As used herein the phrase "fabric care composition" includes compositions and formulations designed for treating fabric. Such compositions include but are not limited to, laundry cleaning compositions and detergents, fabric softening compositions, fabric enhancing compositions, fabric freshening compositions, laundry prewash, laundry pretreat, laundry additives, spray products, dry cleaning agent or composition, laundry rinse additive, wash additive, post-rinse fabric treatment, ironing aid, unit dose formulation, delayed delivery formulation, detergent contained on or in a porous substrate or nonwoven sheet, and other suitable forms that may be apparent to one skilled in the art in view of the teachings herein. Such compositions may be used as a pre-laundering treatment, a post-laundering treatment, or may be added during the rinse or wash cycle of the laundering operation.

As used herein, "fragrance premix composition," "premix composition," and "premix" are used interchangeably, unless otherwise indicated.

As used herein, "amine content," "amine value," and "amine content values" are used interchangeably unless indicated otherwise and can be determined according to the method provided in the Test Method section. Weight percent of nitrogen can be determined from the total amine value as provided in the Test Method Section.

As used in the present disclosure, the term "polymer" includes homopolymers, i.e. obtained by polymerization of one type of monomer with appropriate end cap, as well as copolymers, i.e. obtained by the polymerization of two or more types of monomers. It is understood that in the present invention the term "polymers" includes higher order oligomers (such as trimers, tetramers etc.). Unless otherwise noted, all component or composition levels are in reference to the active portion of that component or composition, and are exclusive of impurities, for example, residual solvents or by-products, which may be present in commercially available sources of such components or compositions.

All temperatures herein are in degrees Celsius (°C) unless otherwise indicated. Unless otherwise specified, all measurements herein are conducted at 20°C and under the atmospheric pressure.

In all embodiments of the present disclosure, all percentages are by weight of the total composition, unless specifically stated otherwise. All ratios are weight ratios, unless specifically stated otherwise.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

Pro-Fragrance Silicone Polymer

The present disclosure relates to pro-fragrance silicone polymers. Such polymers are useful in delivering a fragrance, for example to a target surface. In particular, the pro-fragrance silicone polymers of the present disclosure include a heterocyclic moiety, where the heterocyclic moiety includes a fragment of a perfume raw material. The heterocyclic moiety may be formed by a condensation reaction between, for example, an aldehyde- or ketone-containing perfume raw material and a portion of a silicone precursor that includes an alkanolamine or thiol amine moiety. The perfume raw material is then released upon hydrolysis.

It has been found that by associating the perfume raw materials with the silicone polymer precursors in the fashion described herein, desirable benefits associated with color stability, perfume deposition, and/or perfume release profiles can be achieved.

The present disclosure relates to a pro-fragrance silicone polymer comprising a silicone backbone, an organic linker group, and a heterocyclic moiety bonded to the organic linker group. The organic linker group comprises a carbon atom bonded to a silicon atom of the silicone backbone. As described in more detail below, the heterocyclic moiety comprises a residue of a perfume raw material.

The pro-fragrance silicone polymer comprises a silicone backbone. Typically, the silicone backbone is a siloxane backbone. By "backbone" it is meant the silicone polymer that provides the scaffolding to which the organic linker group is attached. The silicone backbone is typically formed from the precursor silicone polymer, described in more detail below. The silicone backbone is functionalized with X groups and Y or Z groups, as described in more detail below. The silicone backbone may be linear or branched.

It may be preferred that the organic linker group and the heterocyclic moiety, together, form a pendant group that is attached to the silicone backbone. The pendant group, specifically a terminal carbon atom of the linker group, may be bonded to a silicon atom located at a terminal position of the silicone backbone; such silicon atoms are referred to herein as a "terminal silicon atom." The pendant group, specifically a terminal carbon atom of the linker group may be bonded to a silicone atom located at a non-terminal position of the silicone backbone; such silicon atoms are referred to herein as "non-terminal silicon atoms." It may be preferred for the pro-fragrance silicone polymer to comprise one or more pendant groups bonded to one or more non-terminal silicon atoms; this configuration may be preferred because it may allow for greater PRM loading opportunities, given that for many silicone polymers, there are more non-terminal silicon atoms than there are terminal silicon atoms. A pro-fragrance silicone polymer may have pendant groups bonded to terminal silicon atoms and pendant groups bonded to non-terminal silicon atoms. It may even be that one pendant group is bonded to another pendant group.

A pendant group may comprise one or more residues of a perfume raw material, for example at least two residues. At least one of these residues, e.g. a first PRM residue, is part of the heterocyclic moiety, e.g., a first heterocyclic moiety. A second PRM residue may be part of a second heterocyclic moiety on the same pendant group. A second PRM residue may be located on the same pendant group but may not be part of a second heterocyclic moiety; for example, a second PRM residue may be bonded to the pendant group by way of an imine bond. A second PRM residue may be bonded

to the organic linker group. A second PRM residue may be located in a relatively terminal position on the pendant group relative to the first PRM residue. Put another way, the second PRM residue may be located in a distal position relative to both the first PRM residue and the silicone backbone.

The organic linker group and the heterocyclic moiety may be connected to two or more silicone backbones. The organic linker group and the heterocyclic moiety may effectively cross-link two silicone polymer fragments. In an alternative configuration, the organic linker group and the heterocyclic moiety may link two silicone polymer fragments in a linear fashion, such is the case illustrated by Synthetic Example 18, below.

The heterocyclic moiety comprises five ring members. It is understood that the heterocyclic moiety may comprise additional atoms, for example as substituents; the five ring members are only those atoms that form the "ring" of the heterocyclic moiety.

Several of the ring members, namely first, second, and third ring members, have particular identities. For example, the ring members comprise a first ring member that is a nitrogen atom.

The second ring member is a carbon atom. Furthermore, the second ring members (i.e., the carbon atom) is part of a residue of a perfume raw material ("PRM", or a parent PRM), where the PRM that formed the residue comprises a moiety selected from the group consisting of an aldehyde moiety, a ketone moiety, and combinations thereof. More specifically, the carbon atom is the carbon of an aldehyde moiety or ketone moiety of the parent PRM. The heterocyclic moiety forms when a condensation reaction occurs between an amine-containing portion (for example, an alkanolamine or a thiol amine) of a silicone precursor polymer and the parent PRM. Such PRMs are discussed in more detail below. Although transient in low amounts, open-chain tautomers may be present during the formation of the described heterocycles.

The third ring member is an oxygen atom or a sulfur atom, preferably an oxygen atom. It may be preferred for the third ring member to be an oxygen atom as it is believed to have faster rates of PRM hydrolysis, higher PRM loading, improved viscosity profiles, and improved stability.

The second ring member is bonded directed to the first ring member. The second ring member is also bonded directly to the third ring member. When the third ring member is an oxygen atom, and when the fourth and fifth ring members are carbon atoms, the heterocyclic moiety is an oxazolidine-type structure. When the third ring member is a sulfur atom, and when the fourth and fifth ring members are carbon atoms, the heterocyclic moiety is a thiazolidine-type structure.

The general structure of an oxazolidine ring is shown below. The structure has five ring members, each labeled with a number (1-5). It is understood that in the present disclosure, such rings will be substituted, at least at the second ring member, which, as described above, is the residue of a PRM. Furthermore, such ring members will be bonded, directly or indirectly, to the organic linker group and ultimately to the silicone backbone.

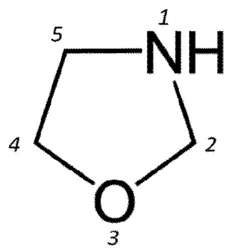

The pro-fragrance silicone polymers of the present disclosure comprise at least one radical selected from the group consisting of Formula I, Formula II, and mixtures thereof, where the radicals of Formula I and Formula II have the following structures:

(Si) - X - Z Formula I;

(Si) - X - (Z - X)$_n$ - (Si) Formula II;

where "(Si) -" represents the bond to an Si atom, where each X group is the organic linker group and is an independently selected divalent organic moiety group comprising from 2 to 12 chain atoms, wherein the X group is bonded to a non-terminal silicon atom, where the Z group comprises the heterocyclic moiety, and where the index n in Formula II is 1 or 2, preferably 1.

The pro-fragrance silicone polymer comprises at least one radical, preferably more than one radical, from the group consisting of Formula I, Formula II, and mixtures thereof. In some cases, the pro-fragrance silicone polymer may have one radical according to Formula I, Formula II, or mixtures thereof. The molar ratio of the radicals (e.g., the -X-Z group) to silicon atoms in the pro-fragrance silicone polymer may be, on average, from 1:1 to 1:500, or from 1:2 to 1:100, or from 1:2 to 1:50, or from 1:2 to 1:20, or from 1:2 to 1:15, or from 1:2 to 1:12, or from 1:5 to 1:12. Relatively more radicals may

be preferred so that a single pro-fragrance silicone polymer has higher loading capacity with regard to PRMs and can therefore delivery greater degrees of freshness benefits more efficiently. However, ratios that are too high may result in stability and/or cross-linking challenges.

Preferably, the at least one radical of the pro-fragrance silicone polymer has the structure of Formula I. Such radicals may correspond to the pendant groups described above and may be located at terminal silicon atoms, non-terminal silicon atoms, or combinations thereof. Radicals according to Formula I may be preferred because of commercial viability and controllable physical properties in both viscosity and formulations.

The structure of Formula II indicates a linking of two polymeric silicone moieties. Such a structure may effectively cross-link two pendant silicone polymer fragments in an intra- or inter-molecular fashion (e.g., one or more X moieties are bonded to a non-terminal silicon atom). Additionally, mixtures of pendant silicone polymer fragments may be cross-linked in an intra- or inter-molecular fashion. Preferably, n is equal to 1 due to ease of synthesis leading to consistent viscosity and formulation properties. Radicals according to Formula II may be preferred for improving perfume delivery and color benefits.

As mentioned above, the "(Si) -" represents the covalent bond to a silicon atom of the silicon polymer. As discussed in more detail above, the silicon atom to which the X moiety is joined is a non-terminal silicon atom.

A carbon atom of the X moiety is bonded to the silicon atom of the backbone, e.g., via the "(Si) -" bond. It is believed that configuration is more stable than if an oxygen of a linker group were to be bonded to the silicon atom. The X group is bonded to a non-terminal silicon atom.

Each X group may independently be a divalent organic moiety having a molecular weight between about 14 and about 1000 Da, preferably between about 28 and about 495 Da, more preferably between about 42 and about 98 Da. Such molecular weights may be preferred for a variety of reasons, such as being readily commercially available and/or affordable, to maintain relatively high perfume loading by weight %, improved reactivity with the PRMs, for improved physical properties of the polymer, and/or for stability within a product or treatment composition.

As mentioned above, each X group is an organic linker group. Each X group is an independently selected divalent organic moiety group comprising from two to twelve chain atoms. By "chain atoms" it is meant the number of atoms directly between the silicon atom and the Z group, counted in a linear fashion. That being said, the X group may include substitutions along the chain; however, the atoms of these substitutions are not to be counted when determining the number of chain atoms. The majority of the chain atoms may be carbon atoms, although heteroatoms, preferably oxygen, may be present in the chain.

Each X group independently comprises from two to twelve chain atoms, preferably from two to nine chain atoms, more preferably from two to six chain atoms, even more preferably from three to five chain atoms. Even more preferably, at least one of the chain atoms may be an oxygen atom. The X group may comprise one or more oxygen atoms, preferably one oxygen atom, for example an alkoxy or an ether group. The oxygen atom may be at a non-terminal position of the X group. The X group may be a four-carbon ether group; preferably, the X group is $-CH_2-CH_2-CH_2-O-CH_2-*$, preferably where the asterisk (*) is the end of the X group that is bonded to the Z group.

The organic linker group may be independently selected from a member of the group consisting of $C_2$-$C_{32}$ substituted or unsubstituted hydrocarbon, $C_2$-$C_{32}$ substituted or unsubstituted alkoxy, $C_6$-$C_{32}$ substituted or unsubstituted aryloxy, $C_2$-$C_{32}$ substituted or unsubstituted acetoxy, $C_2$-$C_{32}$ saturated or unsaturated carbonyl, $C_2$-$C_{32}$ substituted or unsubstituted alkyl amines, $C_2$-$C_{32}$ substituted or unsubstituted hydroxy, $C_5$-$C_{32}$ substituted arenes, $C_2$-$C_{32}$ substituted or unsubstituted epoxides, $C_2$-$C_{32}$ substituted or unsubstituted episulfides, or $C_2$-$C_{32}$ substituted or unsubstituted aziridines; preferably a member independently selected from the group consisting of $C_2$-$C_{32}$ substituted or unsubstituted hydrocarbons or $C_2$-$C_{32}$ substituted or unsubstituted alkoxy; more preferably $C_2$-$C_{32}$ substituted or unsubstituted alkoxy; most preferably $C_2$-$C_{32}$ unsubstituted alkoxy.

The organic linker group may be substituted or unsubstituted. The organic linker group may be linear or branched, preferably linear. As mentioned above, the organic linker group has from two to twelve chain atoms.

In the radicals according to Formula I and/or Formula II, the Z group comprises the heterocyclic moiety. More specifically, each Z group may be a monovalent or divalent heterocyclic moiety derivable by the removal from Formula IV of a moiety selected from the group consisting of $R^1$, one or more monovalent substituents of J, or combinations thereof, where Formula IV has the following structure:

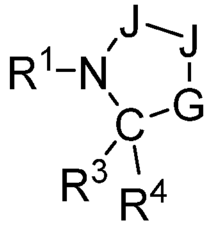

Formula IV,

where G is selected from the group consisting of oxygen or sulfur, preferably oxygen;

where the index m is 2;

where $R^1$ is selected from H, a monovalent moiety with a molecular weight between 15 and 495 Da, or a point of attachment of Z to an X group, preferably $R^1$ is H or a point of attachment of Z to an X group, or preferably $R^1$ is selected from H or a monovalent moiety with a molecular weight between 15 and 101 Da, even more preferably $R^1$ is H;

where each J is independently $C(R^2)_2$,

where each $R^2$ is independently selected from H, a monovalent moiety with a molecular weight between 14 and 990 Da or a point of attachment of Z to an X group, preferably R2 is H or a point of attachment of Z to an X group, or preferably $R^2$ is selected from H and a monovalent moiety with a molecular weight between 14 and 186 Da, even more preferably $R^2$ is H,

with the proviso that a first unit and a second unit can optionally be taken together, where feasible, as a divalent substituent, where the first unit is a first $R^2$ group, and where the second unit is selected from the group consisting of a second $R^2$ group, the $R^1$ group, and a monovalent substituent of the $R^1$ group, preferably where the divalent substituent is selected from the group consisting of a fused ring, a spirocyclic ring, an unsaturated substituent, $=N(R^1)$, =O, and =S,

where the $-C(R^3)(R^4)-$ moiety is the residue of a perfume raw material, preferably a perfume raw material that comprises from 3 to 34 carbon atoms, where the perfume raw material from which the residue is derived comprises an aldehyde moiety, a ketone moiety, or a combination thereof, preferably where $R^3$ is independently selected from a monovalent organic moiety, and preferably wherein is $R^4$ is independently selected from the group consisting of hydrogen and a monovalent organic moiety, optionally, where a second moiety selected from $R^1$, a substituent of $R^1$, one or more monovalent substituents of J, or a combination thereof is replaced with a second link to an X group, preferably thereby forming a second ring structure. To note, in the Synthesis Examples section below, Synthetic Example 15 illustrates an example of such structures where a second ring structure is formed.

As indicated above, each Z group is derivable by the removal of a moiety from Formula IV. It is important to note, however, that "derivable" in this context does not necessarily mean that the moiety is actually derived from a compound according to Formula IV. In fact, it is expected that the Z moiety will rarely if ever be derived from such a compound. Instead, the Z moiety will typically be derived from the condensation reaction of a PRM and an alkanolamine or thiol amine moiety of a silicone polymer precursor, which results in the heterocyclic moiety. Presenting Formula IV as a stand-alone compound is only intended to illustrate the general formula of the Z group, as well as the variety of ways that the Z group may be attached to the X group, in that the site of removal of a (hypothetical) moiety from Formula IV indicates where the Z group is attached to the X group, typically to a terminal portion of the X group.

Put another way, one of the substituents of Formula IV may be a point of attachment (i.e., a covalent bond) of the Z group to the X group. More specifically, an $R^1$ group, and/or any $R^2$ group as described below, may be a point of attachment of attachment of Z to an X group. Even more specifically, it may be preferred that at least one and no more than two of $R^1$, any $R^2$, or mixtures thereof are a point of attachment of Z to an X group.

As described above, a second moiety, for example one selected from $R^1$, a substituent of $R^1$, one or more monovalent substituents of J, or a combination thereof, may be removed and replaced with a second link or bond to an X group. The X group may be part of the same radical or a different radical as the Z group in question. When the X group is part of the same radical, the second bond may result in a second ring structure (e.g., in addition to the heterocyclic moiety that comprises the PRM residue). In the Synthesis Examples section below, Synthetic Examples 13 and 18 illustrate examples of such structures.

As described above, the $-C(R^3)(R^4)-$ moiety is the residue of a perfume raw material (PRM), where the parent PRM comprises an aldehyde moiety, a ketone moiety, or a combination thereof. The "C" of the $-C(R^3)(R^4)-$ moiety represents the carbon of an aldehyde or ketone moiety of the PRM, for example the aldehyde or ketone moiety that was part of the condensation reaction that resulted in the heterocyclic moiety. Thus, the parent PRM may be characterized by the formula $R^3-C(O)-R^4$. When $R^4$ is a monovalent organic moiety, the parent PRM comprises a ketone that condenses to become part of the heterocyclic moiety. When $R^4$ is a hydrogen, the parent PRM comprises an aldehyde that condenses to become part of the heterocyclic moiety. Suitable perfume raw materials are discussed in more detail below.

As described above, there are two J substituents in Formula IV because it is believed that five-membered rings are relatively more stable.

As described above, each J is independently $C(R^2)_2$ for stability, for activity with perfume raw materials, and/or for

release of perfume raw material reasons.

Each Z is a monovalent or divalent five-membered heterocyclic moiety, preferably derivable by the removal from Formula IV of a moiety selected from $R^1$, one or more monovalent substituents of J, or combinations thereof, where Formula IV has the following structure:

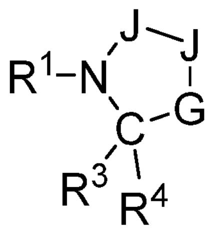

Formula IV,

where G, $R^1$, $R^3$, and $R^4$ are as described above, preferably wherein G is oxygen, and where each J is independently $C(R^2)_2$, where $R^2$ is as described above. Optionally, a second moiety selected from $R^1$, a substituent of $R^1$, one or more monovalent substituents of J, or a combination thereof is replaced with a second link to an X group, preferably thereby forming a second ring structure, more preferably this second ring structure contains a second PRM residue, where the second ring structure may be at a distal position relative to the both the first PRM residue and the silicone backbone, or is forming a bicyclic structure with the heterocyclic moiety.

The Z group may preferably be derivable by the removal of $R^1$ from Formula IV, meaning that the Z group is attached to the X group at the nitrogen atom shown in Formula IV.

The Z group may be preferably derivable by the removal of one or more monovalent substituents of J from Formula IV, preferably where J is $-C(R^2)_2-$, and one or more of the $R^2$ groups are removed, meaning that the Z group is attached to the X group at the location where the removed $R^2$ group would have been. In such cases, it may be preferred that $R^1$ is H.

In Formula IV, when $R^1$ is present as the monovalent moiety, the monovalent moiety is preferably a substituted or unsubstituted $C_1$-$C_{35}$ alkyl group, more preferably a monovalent moiety with a molecular weight of from 15 to 101 Da, even more preferably a substituted or unsubstituted $C_1$-$C_8$ alkyl. In Formula IV, when $R^2$ is present as the monovalent moiety, the monovalent moiety is preferably a substituted or unsubstituted, saturated or unsaturated $C_1$-$C_{70}$ alkyl, more preferably a monovalent moiety with a molecular weight of from 14 to 186 Da, even more preferably a substituted or unsubstituted, saturated or unsaturated $C_1$ to $C_{12}$ alkyl, preferably $R^2$ is H or a point of attachment of Z to an X group. It may be preferred that two of $R^1$, any $R^2$, or a combination thereof are points of attachment of Z to an X group, preferably forming a second ring structure.

It may be preferred that each Z is a monovalent or divalent heterocyclic moiety according to Formula IV, a five-membered heterocyclic moiety, wherein at least one $R^2$ is a point of attachment of Z to an X group, preferably wherein $R^1$ is H.

The pro-fragrance silicone polymers of the present disclosure may have a structure according to Formula V, shown below:

$$[R^5R^6R^7SiO_{1/2}]_{(q+2r+2)}\ [R^8R^9SiO_{2/2}]_p\ [R^{10}SiO_{3/2}]_q\ [SiO_{4/2}]_r \qquad \text{Formula V,}$$

where: q is an integer from 0 to 150; p is an integer from 0 to 1500; r is an integer from 0 to 150; where q+p+r equals an integer greater than or equal to 1; where each of $R^5$, $R^6$, $R^7$, $R^8$, $R^9$ and $R^{10}$ moiety is independently selected from the group consisting of H, OH, a monovalent organic moiety, a radical according to Formula I, or a radical according to Formula II, where at least one of the $R^5$-$R^{10}$ moieties is a radical according to Formula I or a radical according to Formula II, preferably a radical according to Formula I.

When any of $R^5$, $R^6$, or $R^7$ are radicals according to Formula I or Formula II, the radical is attached to a terminal silicon atom. When any of $R^8$, $R^9$, or $R^{10}$ are radicals according to Formula I or Formula II, the radical is attached to a non-terminal silicon atom.

As indicated above, each of $R^5$, $R^6$, $R^7$, $R^8$, $R^9$ and $R^{10}$ moiety may be independently selected from the group consisting of H, OH, a monovalent organic moiety, a radical according to Formula I, or a radical according to Formula II. The monovalent organic moieties, if present, may be independently selected from $C_1$-$C_{32}$ alkyl, $C_2$-$C_{32}$ alkenyl, $C_2$-$C_{32}$ alkynyl, $C_1$-$C_{32}$ substituted alkyl, $C_6$-$C_{32}$ aryl, $C_5$-$C_{32}$ substituted aryl, $C_6$-$C_{32}$ alkylaryl, $C_6$-$C_{32}$ substituted alkylaryl, $C_1$-$C_{32}$ alkoxy, $C_5$-$C_{32}$ aryloxy, $C_2$-$C_{32}$ acetoxy, $C_1$-$C_{32}$ carbonyl, $C_1$-$C_{32}$ carboxamide, $C_1$-$C_{32}$ alky amine, $C_1$-$C_{32}$ thiol amine, or $C_1$-$C_{32}$ alkanolamine.

Perfume Raw Materials

Certain perfume raw materials may be combined with the silicone polymer precursor, for example to form the pro-fragrance silicone polymers of the present disclosure. As described above, the pro-fragrance silicone polymers comprise a heterocyclic moiety, where the heterocyclic moiety comprises, among other things, a residue of a perfume raw material. This section further describes perfume raw materials (and related residues thereof) that are suitable for incorporation into such pro-fragrance silicone polymers.

The term "perfume raw material" (or "PRM") as used herein refers to compounds having a molecular weight of at least about 100 g/mol and which are useful in imparting an odor, fragrance, essence, or scent, either alone or with other perfume raw materials. Typical PRMs comprise inter alia alcohols, ketones, aldehydes, esters, ethers, nitrites, and alkenes, such as terpene. A listing of common PRMs can be found in various reference sources, for example, "Perfume and Flavor Chemicals", Vols. I and II; Steffen Arctander Allured Pub. Co. (1994) and "Perfumes: Art, Science and Technology", Miller, P. M. and Lamparsky, D., Blackie Academic and Professional (1994).

The perfume raw materials that react with the silicone precursor comprises a moiety selected from an aldehyde moiety, a ketone moiety, or combinations thereof. It is believed that PRMs having one or more of these moieties are able to effectively form the heterocyclic moieties in combination with the silicone polymer precursors described herein. Further, such PRMs in combination with the silicone precursors may result in effective release and/or longevity profiles. This is particularly advantageous given that PRMs having such moieties are common and desirable when formulating consumer-relevant olfactory experiences. Preferred PRMs may include an aldehyde moiety or a ketone moiety that is a unsaturated at the $\alpha,\beta$-position.

As mentioned above, the pro-fragrance silicone polymer, specifically the heterocyclic moiety, may include a residue of a perfume raw material that comprises an aldehyde moiety. Perfume raw materials that comprise an aldehyde moiety are provided below in Table A. It is believed that the materials provided in Table A are illustrative (but non-limiting) examples of PRMs that are suitable for use according to the present disclosure.

Table A. Aldehyde-containing perfume raw materials.

| Number | Registry Name | Trade Name |
|---|---|---|
| 1 | 3-Cyclohexene-1-carboxaldehyde, dimethyl- | Ligustral |
| 2 | 3-Cyclohexene-1-carboxaldehyde, 2,4,6-trimethyl- | Isocyclocitral |
| 3 | Cyclohexanemethanol, .alpha.,3,3-trimethyl-, formate | Aphermate |
| 4 | 3-(4-tert-butylphenyl)butanal; pt-bucinal; 3-(4-tert-butylphenyl)butanal | Lilial |
| 5 | 2-methylundecanal | Methyl Nonyl Acetaldehyde |
| 6 | 1-methyl-3-(4-methylpent-3-enyl)cyclohex-3-ene-1-carbaldehyde; myrmac aldehyde | Precyclemone B |
| 7 | Benzenepropanal, 3-(4-ethylphenyl)-2,2-dimethylpropanal | Floralozone |
| 8 | 2,4-dimethylcyclohex-3-ene-1-carbaldehyde | Ligustral/Triplal |
| 9 | Decanal | Decyl Aldehyde |
| 10 | 10-Undecen-1-al ; Undecenoic aldehyde; n-Undecenoic aldehyde; Hendecen-10-al; | Undecylenic aldehyde; Aldehyde C-11, unsaturated;Aldehyde C-11 undecylenic; |
| 11 | 8-,9 and 10-Undecenal, mixture of isomers | Intreleven aldehyde |
| 12 | Benzenepropanal, .alpha.-methyl-4-(1-methylethyl)- | Cymal |
| 13 | 2,6,10-trimethylundec-9-enal | Adoxal; Farenal |
| 14 | 4-(octahydro-4,7-methano-5H-inden-5-ylidene)butanal | Dupical |
| 15 | 3-Ethoxy-4-hydroxybenzaldehyde | Ethyl vanillin |
| 16 | tricyclo[5.2.1.02,6]decane-3-carbaldehyde | Vertral® |
| 17 | 4,7-Methano-1H-indene-2-carboxaldehyde, octahydro-5-methoxy-; 6-Methoxy dicyclopentadiene carboxaldehyde; 8-Methoxytricyclo(5.2.2.1)decane-4-carboxaldehyde; Octahydro-5-methoxy-4,7-methano-1H-indene-2-carboxaldehyde; | Scentenal® 981810 |

(continued)

| Number | Registry Name | Trade Name |
|---|---|---|
| 18 | 4-Hydroxy-3-methoxybenzaldehyde | Vanillin |
| 19 | Trans-4-decenal | Decenal-4-trans |
| 20 | α-hexyl-; α-n-Hexyl-β-phenylacrolein; 2-Hexyl-3-phenyl-2-propenal; 2-Hexyl-3-phenyl-propenal; (2Z)-2-Hexyl-3-phenyl-2-propenal; Hexyl-3-phenyl-propenal; n-Hexyl cinnamaldehyde; (2E)-2-Benzylideneoctanal; 2-[(E)-Benzylidene]octanal | α-Hexylcinnamaldehyde; α-Hexylcinnamic aldehyde; Hexyl cinnamic aldehyde; Hexylcinnamaldehyde; Cinnamaldehyde, |
| 21 | 4-Dodecenal | Tangerinal DIPG 984655 |
| 22 | 3-Cyclohexene-1-propanal,beta,4-dimethyl- | Liminal® 955374 |
| 23 | trans-2-Dodecenal | Mandarine aldehyde 10% CITR 965765 |
| 24 | 4,8-Dimethyl-4,9-decadienal | Floral Super |
| 25 | Hydroxymyrac aldehyde; 4-(4-Hydroxy-4-methyl-pentyl)-3-cyclohexen-1-carboxyaldehyde; Lyral ; Kovanol | Lyral |
| 26 | 2-Hexenal, (E)- | 2-Hexenal |
| 27 | Benzaldehyde | Benzaldehyde |
| 28 | Benzeneacetaldehyde | Phenyl Acetaldehyde |
| 29 | Benzeneacetaldehyde, .alpha.-methyl- | Hydratropic Aldehyde |
| 30 | 3-Cyclohexene-1-carboxaldehyde, 3,5-dimethyl- | Cyclal C, |
| 31 | Benzaldehyde, 4-methoxy- | Anisic Aldehyde |
| 32 | Octanal, 7-hydroxy-3,7-dimethyl- | Hydroxycitronellal |
| 33 | 3-Cyclohexene-1-carboxaldehyde, 3,6-dimethyl- | Cyclovertal |
| 34 | Octanal, 7-methoxy-3,7-dimethyl- | Methoxycitronellal Pq |
| 35 | Benzenepropanal, beta.-methyl-; 3-phenylbutanal | Trifernal |
| 36 | 4,7-Methano-1H-indenecarboxaldehyde, octahydro- | Formyltricyclodecan |
| 37 | Octanal | Octyl Aldehyde |
| 38 | 5-Heptenal, 2,6-dimethyl- | Melonal |
| 39 | Octanal, 3,7-dimethyl- | Dihydrocitronellal |
| 40 | 2-Nonenal | 2 Nonen-1-al |
| 41 | 6-Octenal, 3,7-dimethyl- | Citronellal |
| 42 | 2-Decenal | 2 Decene-1-al |
| 43 | 2,6-Octadienal, 3,7-dimethyl- | Citral |
| 44 | Undecenal | Iso C-11 Aldehyde |
| 45 | Undecanal | Undecyl Aldehyde |
| 46 | 2-Undecenal | 2-Undecene-1-Al |
| 47 | Benzaldehyde, 4-(1-methylethyl)- | Cuminic Aldehyde |
| 48 | Decanal, 2-methyl- | Methyl Octyl Acetaldehyde |
| 49 | Benzenepropanal, 4-(1,1-dimethylethyl)- | Bourgeonal |
| 50 | 2-Dodecenal | 2 Dodecene-1-al |
| 51 | Benzenepropanal, .beta.-methyl-3-(1-methylethyl)- | Florhydral |
| 52 | 1,3-Benzodioxole-5-carboxaldehyde | Heliotropin |
| 53 | 3-Cyclohexene-1-carboxaldehyde, 1-methyl-4-(4-methylpentyl)- | Vemaldehyde |

(continued)

| Number | Registry Name | Trade Name |
|---|---|---|
| 54 | Benzenepropanal, 4-methoxy-.alpha.-methyl- | Canthoxal |
| 55 | Cyclohexenebutanal, .alpha.,2,2,6-tetramethyl- | Cetonal |
| 56 | Dodecanal | Lauric Aldehyde |
| 57 | 5,9-Undecadienal, 2,6,10-trimethyl- | Oncidal |
| 58 | Bicyclo[2.2.2]oct-5-ene-2-carboxaldehyde, 6-methyl-8-(1-methylethyl)- | Maceal |
| 59 | 2-methyl-3-[4-(2-methylpropyl)phenyl]propanal | cyclamen homoaldehyde |
| 60 | 6-methoxy-2,6-dimethyloctanal | calypsone |
| 61 | 4-propan-2-ylbenzaldehyde | Cuminic Aldehyde |
| 62 | 3,6-dimethylcyclohex-3-ene-1-carbaldehyde | VERTOLIFF |
| 63 | 2-methyl-3-(4-methylphenyl)propanal | Jasmorange®; satinaldehyde |
| 64 | 3-phenylprop-2-enal | Cinnamic Aldehyde |

The perfume raw material that formed the PRM residue of the heterocyclic moiety may be selected from the group consisting of the aldehyde-containing PRMs of Table A, above. The PRM that formed the PRM residue of the heterocyclic moiety may comprise an aldehyde moiety and preferably be selected from the group consisting of: methyl nonyl acetaldehyde: benzaldehyde; floralozone; isocyclocitral; triplal (ligustral); precylcemone B; lilial; decyl aldehyde; undecylenic aldehyde; cyclamen homoaldehyde; cyclamen aldehyde; dupical; oncidal; adoxal; melonal; calypsone; anisic aldehyde; heliotropin; cuminic aldehyde; scentenal; 3,6-dimethylcyclohex-3-ene-1-carbaldehyde; satinaldehyde; canthoxal; vanillin; ethyl vanillin; cinnamic aldehyde; and mixtures thereof.

As mentioned above, the pro-fragrance silicone polymer, specifically the heterocyclic moiety, may include a residue of a perfume raw material that comprises a ketone moiety. Perfume raw materials that comprise a ketone moiety are provided below in Table B. It is believed that the materials provided in Table B are illustrative (but non-limiting) examples of PRMs that are suitable for use according to the present disclosure.

Table B. Ketone-containing perfume raw materials.

| Number | Registry Name | Trade Name |
|---|---|---|
| 1 | 2-Buten-1-one, 1-(2,6,6-trimethyl-3-cyclohexen-1-yl)- | delta-Damascone |
| 2 | (1-(2,6,6-Trimethyl-2-cyclohexen-1-yl)-2-buten-1-one); 2-Buten-1-one, 1-(2,6,6-trimethyl-2-cyclohexen-1-yl)-, (E)- | alpha-Damascone |
| 3 | (1-(2,6,6-Trimethyl-1-cyclohexen-1-yl)-2-buten-1-one); 2-Buten-1-one, 1-(2,6,6-trimethyl-1-cyclohexen-1-yl)-, (E)- | beta-Damascone |
| 4 | 2-Buten-1-one, 1-(2,6,6-trimethyl-1,3-cyclohexadien-1-yl)- | Damascenone |
| 5 | 1,1,2,3,3-pentamethyl-2,5,6,7-tetrahydroinden-4-one | Cashmeran |
| 6 | 1-(5,5-dimethyl-1-cyclohexenyl)pent-4-en-1-one | Neobutenone Alpha |
| 7 | 1-(5,5-dimethyl-1-cyclohexenyl)pent-4-en-1-one | Galbascone; Dynascone |
| 8 | 1-naphthalen-2-ylethanone | Methyl Beta-Naphthyl Ketone |

(continued)

| Number | Registry Name | Trade Name |
|---|---|---|
| 9 | 2-(2-(4-Methyl-3-cyclohexen-1-yl)propyl)cyclo-pentanone | Nectaryl |
| 10 | 2-Hexyl-2-cyclopenten-1-one (main component) | Isojasmone B 11 |
| 11 | Methyl 2,6,10-Trimethyl-2,5,9-cyclododecatrien-1-yl ketone; | Trimofix "O" |
| 12 | α-Isomethyl ionone; 5-(2,6,6-Trimethyl-2-cyclohexen-1-yl)-3-methyl-3-buten-2-one; | Methyl ionone; Methyl Ionone Alpha Iso; Methyl Ionone Gamma; Isoraldeine 70; Isoraldeine 95; Gamma Methylionone 600 UC; Alpha Daphnone; Iraldeine gamma; gamma Methyl Ionone Pure; gamma Methyl Ionone A; Gamma Methyl Ionone Coeur |
| 13 | 2-Heptylcyclopentanone; | Fleuramone; Projasmon |
| 14 | 3-(Hydroxymethyl)nonan-2-one (and isomer) | Methyl lavender ketone |
| 15 | 2-Cyclohexen-1-one, 2-methyl-5-(1-methylethenyl)-, (R)- | Laevo Carvone |
| 16 | Bicyclo[2.2.1]heptan-2-one, 1,7,7-trimethyl-, (1R)- | Camphor Gum |
| 17 | 2-Heptanone | Methyl Amyl Ketone |
| 18 | 3-Octanone | Ethyl Amyl Ketone |
| 19 | 2-Octanone | Methyl Hexyl Ketone |
| 20 | 5-Hepten-2-one, 6-methyl- | Methyl Heptenone |
| 21 | Ethanone, 1-(4-methylphenyl)- | Para Methyl Acetophenone |
| 22 | 2-Butanone, 4-phenyl- | Benzyl Acetone |
| 23 | 1,4-Methanonaphthalen-5(1H)-one, 4,4a,6,7,8,8a-hexahydro- | Tamisone |
| 24 | 2H-1-Benzopyran-2-one, 3,4-dihydro- | Dihydrocoumarin |
| 25 | Cyclohexanone, 5-methyl-2-(1-methylethyl)-, cis- | Iso Menthone |
| 26 | 2H-Pyran-2-one, 6-butyltetrahydro- | Nonalactone |
| 27 | 3-Hepten-2-one, 3,4,5,6,6-pentamethyl- | Koavone |
| 28 | Cyclopentanone, 3-methyl-2-pentyl- | Jasmylone |
| 29 | 3-Nonanone | Ethyl Hexyl Ketone |
| 30 | Ethanone, 1-(3,3-dimethylcyclohexyl)- | Herbac |
| 31 | 3-Heptanone, 5-methyl-, oxime | Stemone |
| 32 | Cyclohexanone, 2-(1-methylpropyl)- | 2-Sec-Butyl Cyclo Hexanone |
| 33 | Cyclopentanone, 2-pentyl- | Delphone |

(continued)

| Number | Registry Name | Trade Name |
|---|---|---|
| 34 | 2-Cyclopenten-1-one, 3-methyl-2-pentyl- | Dihydroj asmone |
| 35 | Cyclohexanone, 5-methyl-2-(1-methylethyl)-, trans- | Menthone Racemic |
| 36 | Cyclohexanone, 4-(1,1-dimethylpropyl)- | Orivone |
| 37 | 2-Undecanone | Methyl Nonyl Ketone |
| 38 | 1-Decanol | Rhodalione |
| 39 | 2-Cyclohexen-1-one, 3-methyl-5-propyl- | Livescone |
| 40 | 2-Cyclopenten-1-one, 2-methyl-3-(2-pentenyl)- | Iso Jasmone |
| 41 | Ionone | Ionone Ab |
| 42 | 3-Buten-2-one, 4-(2,6,6-trimethyl-2-cyclohexen-1-yl)-, (E)- | Ionone Alpha |
| 43 | 3-Buten-2-one, 4-(2,6,6-trimethyl-1-cyclohexen-1-yl)- | Ionone Beta |
| 44 | 2-Buten-1-one, 1-(2,4,4-trimethyl-2-cyclohexen-1-yl)-, (E)- | Isodamascone N |
| 45 | 2H-1-Benzopyran-2-one | Coumarin |
| 46 | Cyclopentanone, 2-heptyl- | Fleuramone |
| 47 | 3-Decanone, 1-hydroxy- | Methyl Lavender Ketone |
| 48 | 1-Propanone, 1-[2-methyl-5-(1-methylethyl)-2-cyclohexen-1-yl]- | Nerone |
| 49 | 9-Undecen-2-one, 6,10-dimethyl- | Tetra Hydro Psuedo Ionone |
| 50 | 1-phenylethanone | Acetophenone |
| 51 | 2-butan-2-ylcyclohexan-1-one | Freskomenthe |
| 52 | Ethanone, 1-(3-methyl-2-benzofuranyl)- | nerolione |
| 53 | 4-(4-methoxyphenyl)butan-2-one | Anisyl Acetone |

The perfume raw material that formed the PRM residue of the heterocyclic moiety may be selected from the group consisting of the ketone-containing PRMs of Table B, above. The PRM that formed the PRM residue of the heterocyclic moiety may comprise a ketone moiety and may preferably be selected from the group consisting of: nerolione; 4-(4-methoxyphenyl)butan-2-one; 1-naphthalen-2-ylethanone; nectaryl; trimofix O; fleuramone; delta-damascone; beta-damascone; alpha-damascone; methyl ionone; 2-hexylcyclopent-2-en-1-one; galbascone; and mixtures thereof.

The pro-fragrance silicone polymers of the present disclosure may be used in combination with other perfume raw materials, even PRMs that do not contain an aldehyde or ketone moiety. Other perfume raw materials, such as those that do not contain an aldehyde moiety or a ketone moiety, may also be mixed with the silicone precursor, as it is believed that the hydrophobic silicone droplets may facilitate deposition of certain PMRs, even if a condensation reaction does not occur. Additionally or alternatively, it is possible that the PRMs in question may react with other portions of the silicone precursor, even if the reaction does not result in a heterocyclic moiety as described herein. Additionally or alternatively, the

PRMs may react with other compounds, such as low-molecular-weight amines, that may in turn react with the silicone precursor. It may even be that a materials supplier may wish to provide reacted PRMs and unreacted PRMs in the same silicone emulsion, for example to save on packaging, shipping, or storage costs. Additionally or alternatively, other PRMs may be provided as neat or free oils to the premix composition and/or the treatment compositions according to the present disclosure, for example to provide a more well-rounded olfactory experience.

Other perfume raw materials are provided below in Table C. It is believed that the materials provided in Table C are illustrative (but non-limiting) examples of PRMs that are suitable for use according to the present disclosure. For example, these "other" PRMs may be co-formulated with the pro-fragrance silicone polymers of the present disclosure, for example by being added as neat or encapsulated perfume. It may even be that the pro-fragrance silicone polymers can further react/condense with one or more other perfumes, even if a heterocyclic moiety is not formed in the process.

Table C. Other perfume raw materials.

| Number | Registry Name | Trade Name |
|---|---|---|
| 1 | Ethyl 2 Methyl Pentanoate | Manzanate |
| 2 | 3,7,11-Trimethyl-2,6,10-dodecatrien-12-ol; 2,6,10-Dodecatrien-1-ol, 3,7,11-trimethyl-; Famesol; Farnesyl alcohol; 3,7,11-Trimethyl-2,6,10-dodecatrien-1-ol; 3,7,11-Trimethyl-2,6,10-dodecatrienol; Trimethyl-2,6,10-dodecatriene-1-ol; (2E,6E)-3,7,11-Trimethyl-2,6,10-dodecatrien-1-ol; α-Famesol; alpha-Famesol; 3,7,11-Trimethyldodeca-2,6,10-trien-1-ol; 3,7,11-Trimethyl-2,6,10-dodecatrien-1-ol (famesol); (E)-alpha-Farnesol | Farnesol |
| 3 | (1-Methyl-2-(1,2,2-trimethylbicyclo[3.1.0]-hex-3-ylmethyl)cyclopropyl)methanol (Mixture of diastereoisomers) | Javanol® |
| 4 | 2-Methyl-3-{(1,7,7-trimethylbicyclo{2.2.1}hept-2-yl)oxy}exo-1-propanol and isomers | Bornafix® |
| 5 | 2-Methyl-4-(2,2,3-trimethylcyclopent-3-en-1-yl)butan-1-ol; 2-Methyl-4-(2,2,3-trimethyl-3-cyclopenten-1-yl)-butan-1-ol | Brahmanol® |
| 6 | 2-Ethyl-4-(2,2,3-trimethyl-3-cyclo-penten-1-yl)-2-buten-1-ol; 2-Ethyl-4-(2,2,3-trimethyl-3-cyclopenten-1-yl)-2-buten-1-ol; B-Ethyl-2,2,3-trimethyl-3-Cyclopentene-1-but-2-enol; Ethyl Trimethylcyclopentene Butenol | Bacdanol® Bacdanol ; Sandranol; Bangalol; Sandolen ; Balinol; Laevo Trisandol; Levosandol; |
| 7 | Cyclohexanemethanol, 4-(1-methylethyl)-, cis-; 4-Isopropylcyclohexanemethanol; | Mayol® 957230 |
| 8 | 2-Methyl-5-phenylpentan-1-ol | Rosaphen® |
| 9 | 3-Methyl-5-phenylpentanol; 3-Methyl-5-phenyl-1-pentanol; | Phenoxanol®; Mefrosol; Phenyl hexanol |
| 10 | 9-decen-1-ol; 9-Decenol; | Rosalva; Trepanol |
| 11 | 2-Methyl-4-(2,2,3-trimethyl-3-cyclopenten-1-yl)-2-buten-1-ol;; 2-Buten-1-ol, 2-methyl-4-(2,2,3-trimethyl-3-cyclopenten-1-yl)-; 2-Buten-1-ol,2-methyl-4-(2,2,3-trimethyl-3-cyclopenten-1-yl)- | Hindinol; Sandalmysore core ; Santalaire; Madranol; Santaliff™ |
| 12 | 3-Cyclohexene-1-propanol gamma 4-dimethyl- | Cyclomethylene citronellol 937001 |
| 13 | 2,2-Dimethyl-3-(3-methylphenyl)propan-1-ol | Majantol® |
| 14 | 3,7-Dimethyl-6-octen-1-ol, (-)-Citronellol; Rhodinol | Citronellol |
| 15 | (2E,6Z)-nona-2,6-dien-1-ol | 2,6-Nonadienol |
| 16 | 2-[(1,7,7-Trimethylbicyclo[2.2.1]hept-2-yl)oxy]-ethanol | Cedanol |
| 17 | 2,4,6-Trimethyl-3-cyclohexene-1-methanol | Isocyclogeraniol |

(continued)

| Number | Registry Name | Trade Name |
|---|---|---|
| 18 | 3,7-Dimethyl-trans-2,6-octadien-1-ol; 3,7-Dimethyl-2,6-octadien-1-ol (isomers); trans-Geraniol; Guaniol; Lemonol; trans-3,7-Dimethyl-2,6-octadien-1-ol; Geraniol alcohol; Geraniol extra; Geranyl alcohol; 2,6-Dimethyl-trans-2,6-octadien-8-ol; 2,6-Octadien-1-ol, 3,7-dimethyl-, trans-; 3,7-Dimethyl-trans-2,6-octadien-1-ol; (E)-3,7-Dimethyl-2,6-octadien-1-ol; Meranol; trans-3,7-Dimethyl octa-2,6-dien-1-ol; (2E)-3,7-Dimethyl-2,6-octadien-1-ol; Nerol; Neryl alcohol; trans-3,7-Dimethyl-2,6-octadien-1-ol (geraniol); t-Geraniol; (E)-Geraniol; (E)-3,7-Dimethyl-2,6-octadien-1-ol; Geraniol (E) | Geraniol |
| 19 | Dihydrocinnamic alcohol; 3-Phenylpropanol; Benzenepropanol; 1-Propanol, 3-phenyl-; γ-Phenylpropanol; γ-Phenylpropyl alcohol; (3-Hydroxypropyl)benzene; Hydrocinnamic alcohol; Hydrocinnamyl alcohol; 3-Phenyl-n-propanol; 3-Phenyl-1-propanol; 3-Phenylpropyl alcohol; 3-Benzenepropanol; Phenylpropyl alcohol; 1-Hydroxy-3-phenylpropane; 3-Phenylpropan-1-ol; Phenylpropylic alcohol | 3-Phenylpropyl alcohol |
| 20 | Cinnamic alcohol; 3-Phenyl-2-propen-1-ol; Cinnamyl alcohol; γ-Phenylallyl alcohol; Phenyl-2-propen-1-ol; Styrone; Styryl carbinol; 3-Phenylallyl alcohol; 1-Phenyl-1-propen-3-ol; 3-Phenyl-2-propen-1-ol; 3-Phenyl-2-propenol; Alkohol skoricovy; 3-Fenyl-2-propen-1-ol; Peruvin; Phenyl-2-propenol; Phenylallyl alcohol; (2E)-3-Phenyl-2-propen-1-ol; 3-phenylprop-2-en-1-ol; 2-Propen-1-ol, 3-phenyl- | Cinnamic alcohol |
| 21 | 2-Hexen-1-ol, (E)-; trans-2-Hexen-1-Ol; trans-2-Hexenol; 2-Hexenol; 2-Hexen-1-ol, trans-; (2E)-2-Hexen-1-ol; (E)-2-Hexenol; (E)-Hex-2-en-1-ol; (E)-Hex-2-enol; (E)-2-Hexene-1-ol; Hex-2(E)-enol; t-2-Hexen-1-ol; 2-(E)-hexenol; trans-Hex-2-en-1-ol | trans-2-Hexenol |
| 22 | 4-(5,5,6-Trimethylbicyclo[2.2.1]hept-2-yl)cyclohexan-1-ol (and isomers, 85% solution in IPM) | Sandela® |
| 23 | 1-Naphthalenol,1,2,3,4,4a,5,8,8a-octahydro-2,2,6,8-tetramethyl- | Octalynol 967544 |
| 24 | 3-Methyl-5-(2,2,3-trimethyl-3-cyclopenten-1-yl)-4-penten-2-ol; | Ebanol |
| 25 | 4-Methyl-3-decen-5-ol | Undecavertol |
| 26 | 4-Penten-2-ol, 3,3-dimethyl-5-(2,2,3-trimethyl-3-cyclopenten-1-yl)- ; | Nirvanol® 974650; Polysantol® 974656 |
| 27 | 3-Methyl-4-phenylbutan-2-ol | Muguesia |
| 28 | 2-Methoxy-4-allylphenol | Eugenol |
| 29 | Cyclohexanepropanol, 2,2,6-trimethyl-alpha-propyl-; 1-(2,2,6-Trimethylcyclohexyl)hexan-3-ol | Norlimbanol 967412; Timberol®; |
| 30 | 5-Propenyl-2-ethoxyphenol; | Propenyl guaethol; Vanitrope |
| 31 | 1-(4-Isopropyl-cyclohexyl) ethanol; 1-(4-Isopropylcyclohexyl)-ethanol | Mugetanol |
| 32 | 2-Pentylcyclopentan-1-ol; 2-Pentylcyclopentanol | Cyclopentol HC 937165 |
| 33 | 3,7,11-Trimethyl-1,6,10-dodecatrien-3-ol | Nerolidol |
| 34 | Cedrol Crude | Cedrol |
| 35 | 3,7-Dimethyl-7-hydroxyoctan-1-al dimethyl acetal | Hydroxycitronellal dimethyl acetal |
| 36 | 4-Methyl-2-(2-methylpropyl)tetrahydro-2H-pyran-4-ol; Florol® 966458 | Florosa |

(continued)

| Number | Registry Name | Trade Name |
|---|---|---|
| 37 | 2,5,5-Trimethyl-octahydronaphthalen-2-ol; | Ambrinol 20-T |
| 38 | 2,5,5-Trimethyl-1,2,3,4,4α,5,6,7-octahydro-2-naphthalenol; | Ambrinol; Ambrinol S |
| 39 | 4-Methyl-1-isopropyl-3-cyclohexen-1-ol | Terpinen-4-ol |
| 40 | 3,7-Dimethyl-1,6-nonadien-3-ol (cis & trans) | Ethyl linalool |
| 41 | 1-Methyl-3-(2-methylpropyl)cyclohexanol | Rossitol® |
| 42 | 4-Phenyl-2-methyl-2-butanol | α,α-Dimethylphenylethylcarbinol |
| 43 | 3,7-Dimethyloctan-1,7-diol | Hydroxyol |
| 44 | 1-Methyl-4-isopropylcyclohexane-8-ol | Dihydro terpineol |
| 45 | 3,7-Dimethyl-1,6-octadiene-3-ol | Linalool |
| 46 | 3,7-Dimethyl-4,6-octadien-3-ol | Allo-Ocimenol; Muguol |
| 47 | 2-(4-Methyl-cyclohex-3-enyl)-propan-2-ol; p-Menthan-8-ol | alpha Terpineol, Lindenol™ |
| 48 | 1-Phenyl-2-methyl-2-propanol; α,α-Dimethylbenzyl carbinol; benzeneethanol, α,α-dimethyl- | 2-methyl-1-phenylpropan-2-ol |
| 49 | Cyclohexanepropanol,2,2-dimethyl- | Coranol 928130 |
| 50 | 2,6-Dimethyl-7-octen-2-ol; 2-Methyl-6-methyleneoct-7-en-2-ol, dihydro derivative; 7-Octen-2-ol, 2,6-dimethyl-; 2,6-Dimethyl-7-octen-2-ol; 3,7-Dimethyl-1-octen-7-ol; 2,6-Dimethyl-oct-7-en-2-ol; Mircenol, 6,10-dihydro | Dihydromyrcenol, Dihydro Myrcenol |
| 51 | 3,7-Dimethyloctan-3-ol | Tetrahydrolinalool |
| 52 | 2,6-Dimethyl-2-octanol | Tetrahydro myrcenol |
| 53 | 2,6-Dimethyl-2-heptanol | Dimetol , Freesiol , Lolitol |
| 54 | 2-Hexen-1-ol | 2-Hexen-1-ol |
| 55 | 3-Hexen-1-ol | Beta Gamma Hexenol |
| 56 | Ethanol, 2,2'-oxybis- | Calone 161 |
| 57 | Benzoic acid, 2-amino-, methyl ester | Methyl Anthranilate |
| 58 | 2H-Pyran, 3,6-dihydro-4-methyl-2-(2-methyl-1-propenyl)- | Nerol Oxide |
| 59 | Benzeneethanol, .beta.-methyl- | Hydratropic Alcohol |
| 60 | Benzeneethanol, .alpha.,.alpha.-dimethyl- | Dimethyl Benzyl Carbinol |
| 61 | Benzoic acid, 2-(methylamino)-, methyl ester | Dimethyl Anthranilate |
| 62 | 1-Heptanol | Heptyl Alcohol |
| 63 | Ethanol, 2-(2-methoxyethoxy)- | Veramoss Sps |
| 64 | Cyclohexaneethanol | Cyclohexyl Ethyl Alcohol |
| 65 | 3-Octen-1-ol, (Z)- | Octenol Dix |
| 66 | Ethanone, 1-(4-methylphenyl)- | Para Methyl Acetophenone |
| 67 | Linalool oxide | Linalool Oxide |
| 68 | Benzenepropanol | Phenyl Propyl Alcohol |
| 69 | Ethanol, 2-phenoxy- | Phenoxyethanol |
| 70 | 1H-Indole | Indole |
| 71 | 1,3-Dioxolane, 2-(phenylmethyl)- | Ethylene Glycol Acetal/Phenyl Acetaldehy |
| 72 | Cyclohexanol, 1-methyl-4-(1-methylethyl)- | Dihydroterpineol |

(continued)

| Number | Registry Name | Trade Name |
|---|---|---|
| 73 | 3,5-Octadien-2-ol, 2,6-dimethyl-, (?,Z)- | Muguol |
| 74 | 3-Cyclohexen-1-ol, 4-methyl-1-(1-methylethyl)- | Terpinenol |
| 75 | Bicyclo[2.2.1]heptan-2-ol, 1,3,3-trimethyl- | Fenchyl Alcohol |
| 76 | Cyclohexanol, 2-(1,1-dimethylethyl)-, cis- | Verdol |
| 77 | Bicyclo[2.2.1]heptan-2-ol, 1,7,7-trimethyl-, (1S-endo)- | Borneol Crystals |
| 78 | Cyclohexanol, 5-methyl-2-(1-methylethyl)- | Menthol |
| 79 | Cyclohexanol, 2-(1,1-dimethylethyl)-, acetate | Verdox |
| 80 | 3-Octanol | Octanol-3 |
| 81 | 2-Heptanol, 2,6-dimethyl- | Dimethyl-2, 6-Heptan-2-ol |
| 82 | 1-Octanol | Octyl Alcohol |
| 83 | 3-Octanol, 3,7-dimethyl- | Linacsol |
| 84 | Isononanol | Iso Nonyl Alcohol |
| 85 | 1-Nonanol | Nonyl Alcohol |
| 86 | 1-Octanol, 3,7-dimethyl- | Dimethyl Octanol |
| 87 | 6-Octen-1-ol, 3,7-dimethyl-, (S)- | Baranol |
| 88 | Cyclohexanol, 3,3,5-trimethyl-, cis- | Trimethylcyclohexanol |
| 89 | 1-Hexanol, 5-methyl-2-(1-methylethyl)-, (R)- | Tetrahydro Lavandulol |
| 90 | Cyclohexanol, 4-(1-methylethyl)- | Roselea |
| 91 | 7-Octen-2-ol, 2,6-dimethyl-, formate | Dimyrcetol |
| 92 | 5,7-Octadien-2-ol, 2,6-dimethyl- | Ocimenol |
| 93 | 2H-Pyran, 6-butyl-3,6-dihydro-2,4-dimethyl- | Gyrane |
| 94 | Cyclohexanol, 4-(1,1-dimethylethyl)- | Patchon |
| 95 | Cyclohexanol, 5-methyl-2-(1-methylethyl)-, [1R- (1.alpha.,2.beta.,5.alpha.)]- | Menthol Natural |
| 96 | 2-Nonanol, 6,8-dimethyl- | Nonadyl |
| 97 | Phenol, 4-(1,1-dimethylethyl)- | Para Tertiary Butyl Phenol |
| 98 | Cyclohexanol, 5-methyl-2-(1-methylethenyl)-, [1R-(1.alpha.,2.beta.,5.alpha.)]- | Iso Pulegol |
| 99 | 1-Decanol | Rhodalione |
| 100 | Phenol, 2-methyl-5-(1-methylethyl)- | Carvacrol |
| 101 | 2-Naphthalenol, decahydro- | Trans Deca Hydro Beta Naphthol |
| 102 | Phenol, 5-methyl-2-(1-methylethyl)- | Thymol Nf |
| 103 | Phenol, 2-methoxy-4-propyl- | Dihydro Eugenol |
| 104 | Benzene, 1,2-dimethoxy-4-(2-propenyl)- | Methyl Eugenol |
| 105 | Ethanol, 2-[(1,7,7-trimethylbicyclo[2.2.1]hept-2-yl)oxy]-, exo- | Arbanol |
| 106 | Phenol, 4-chloro-3,5-dimethyl- | 4-Chloro 3,5 Xylenol |

The perfumes raw materials in this specification, including the perfume raw materials listed above, can be obtained from suppliers including: International Flavors and Fragrances of New York, NY USA; Givaudan of Vernier Switzerland; Firmenich of Geneva, Switzerland; Symrise of Holzminden, Germany; Kao of Tokyo, Japan; Takasago of Tokyo, Japan; and Florasynth of Tel-Aviv, Israel.

Precursor Silicone Polymers

The present disclosure also relates to certain silicone polymers that may, for example, serve as precursors to the pro-fragrance silicone polymers according to the present disclosure. These precursor silicone polymers may be reacted, for example by way of a condensation reaction, with a perfume raw material to form the pro-fragrance silicone polymers of the present disclosure. Thus, these precursors are stand-alone polymers that may act as feedstock polymers or reactants that can be useful to form the pro-fragrance silicone polymers described herein.

Typically, the precursor silicone polymer has one or more portions that include alkanolamine and/or thiol amine moieties, which are capable of reacting with certain aldehyde- or ketone-containing PRMs to form the heterocyclic moieties described herein. The one or more portions with an alkanolamine and/or thiol amine moiety may be connected to a silicone polymer backbone via an organic linker group.

The precursor silicone polymer may comprise at least one radical selected from the group consisting of Formula VI, Formula VII, and mixtures thereof, where the radicals of Formula VI and Formula VII have the following structures:

(Si) - X - Y Formula VI;

$(Si) - X - (Y - X)_n - (Si)$ Formula VII;

and

where "(Si) -" is the bond to a silicon atom, for example a silicon atom of a silicone backbone,

where the index n is 1 or 2, preferably 1,

wherein each X group is an organic linker group, is an independently selected divalent organic group comprising from 2 to 12 chain atoms, and comprises a carbon atom bonded to a silicon atom of a silicone backbone (i.e., each X group is covalently linked to "(Si)" via a Si-C bond), and

wherein each Y group is independently a monovalent or divalent moiety that includes a nitrogen atom and a second atom, where the second atom is selected from the group consisting of an oxygen atom and a sulfur atom, and where the nitrogen atom is separated from the second atom by two atoms, three atoms, or four atoms.

Suitable organic linker / X groups are described in more detail above with regard to the pro-fragrance silicone polymer; the related disclosure above substantially applies equally with respect to the precursor silicone polymer. For example, a carbon atom of the X moiety is bonded to the silicon atom of the backbone, e.g., via the "(Si) -" bond. It is believed that configuration is more stable than if an oxygen of a linker group were to be bonded to the silicon atom. The X group is bonded to a non-terminal silicon atom.

In the radicals according to Formula VI and/or Formula VII, the Y group comprises the alkanolamine moiety and/or the thiol amine moiety. The Y groups may be selected so that they are capable of forming a Z group when reacted with an aldehyde- or ketone-containing perfume raw material, as described above.

For example, the Y group may be derivable by the removal from Formula VIII of a moiety selected from $R^1$, one or more monovalent substituents from J, or combinations thereof, where Formula VIII has the following structure:

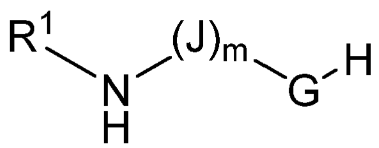

Formula VIII,

where G is selected from the group consisting of oxygen or sulfur, preferably oxygen;

where the index m is 2;

where $R^1$ is selected from H, a monovalent moiety with a molecular weight between 15 and 495 Da, or a point of attachment of Y to an X group, more preferably $R^1$ is selected from H or a monovalent moiety with a molecular weight between 15 and 101 Da, even more preferably $R^1$ is H; where each J is independently $C(R^2)_2$, where each $R^2$ is independently selected from H, a monovalent moiety with a molecular weight between 14 and 990 Da, or a point of attachment of Y to an X group, more preferably $R^2$ is selected from H and a monovalent moiety with a molecular weight

between 14 and 186 Da, even more preferably $R^2$ is H,

with the proviso that a first unit and a second unit can optionally be taken together, where feasible, as a divalent substituent, where the first unit is a first $R^2$ group, and where the second unit is selected from the group consisting of a second $R^2$ group, the $R^1$ group, and a monovalent substituent of the $R^1$ group, preferably where the divalent substituent is selected from the group consisting of a fused ring, a spirocyclic ring, an unsaturated substituent, =N($R^2$), -C(O)-, and - C(S)-; optionally, where a second moiety selected from $R^1$, a substituent of $R^1$, one or more monovalent substituents of J, or a combination thereof is replaced with a second link to an X group, preferably thereby forming a ring structure. At least one and no more than two of $R^1$, any $R^2$, or combinations thereof is a point of attachment of Y to an X group.

In Formula VIII, when $R^1$ is present as the monovalent moiety, the monovalent moiety is preferably a substituted or unsubstituted $C_1$-$C_{35}$ alkyl group, more preferably a monovalent moiety with a molecular weight of from 15 to 101 Da, even more preferably a substituted or unsubstituted $C_1$-$C_8$ alkyl, preferably wherein $R^1$ is H. In Formula VIII, when $R^2$ is present as the monovalent moiety, the monovalent moiety is preferably a substituted or unsubstituted, saturated or unsaturated $C_1$-$C_{70}$ alkyl, more preferably a monovalent moiety with a molecular weight of from 14 to 186 Da, even more preferably a substituted or unsubstituted, saturated or unsaturated $C_1$ to $C_{12}$ alkyl, preferably wherein each $R^2$ is H.

It may be preferred that when G is oxygen and when Y is derivable from the removal of $R^1$, m is not 2 or 3, as it is believed that such compounds are novel and useful for making the premixes and pro-fragrance silicone compounds described herein.

Similar to the discussions of the Z groups above, the term "derivable" in this context does not necessarily mean that the moiety is actually derived from a compound according to Formula VIII. Similarly, the site of (hypothetical) "removal" indicates where the Y moiety is connected to the X moiety.

It is believed that any of the above-described precursor silicone polymers are suitable for use in forming the presently described pro-fragrance silicone polymers, or in any of the related processes, premixes, or treatment compositions described herein. However, for patentability purposes of the precursor silicone polymer *per se,* it may be preferred that when G is an oxygen atom, and preferably when the Y group is derivable from the removal of the $R^1$ group (e.g., the Y group is connected to the X group via the nitrogen group of Formula VIII where the $R^1$ group is shown), m is not 2 or 3.

The precursor silicone polymers of the present disclosure may have a structure according to Formula IX, shown below:

$$[R^5R^6R^7SiO_{1/2}]_{(q+2r+2)}\ [R^8R^9SiO_{2/2}]_p\ [R^{10}SiO_{3/2}]_q\ [SiO_{4/2}]_r \qquad \text{Formula IX}$$

where: q is an integer from 0 to 150; p is an integer from 0 to 1500; r is an integer from 0 to 150; where q+p+r equals an integer greater than or equal to 1; where each of $R^5$, $R^6$, $R^7$, $R^8$, $R^9$ and $R^{10}$ moiety is independently selected from the group consisting of H, OH, a monovalent organic moiety, a radical according to Formula VI, or a radical according to Formula VII, where at least one of the $R^5$-$R^{10}$ moieties is a radical according to Formula VI or a radical according to Formula VII, preferably a radical according to Formula VI.

The precursor silicone polymer may be characterized by a weight average molecular weight of from 450 Da to 200000 Da, and/or the pro-fragrance silicone polymer may be characterized by a viscosity comprised in the range between 0.004 (Pa*s) and 100 (Pa*s). Methods for determining the weight average molecular weight and the viscosity of silicone polymers are provided in the Test Method section below. Such molecular weights and/or viscosities may be preferred because it is believed that they lead to improved surface deposition, formulation stability, and affinity with perfume raw materials.

The precursor silicone polymer comprises nitrogen atoms. The precursor silicone polymer may be characterized by a total amine content of from 0.05 to 2.2, preferably from 0.071 to 2.14, or from 0.071 to 1.78, or from 0.71 to 1.43, or from 0.14 to 1.07, or from 0.14 to 0.71, or from 0.21 to 0.71, or from 0.36 to 0.71. The precursor silicone polymer may be characterized by a nitrogen content of from 0.1% to 3%, or from 0.1% to 2%, or from 0.2% to 1.5%, or from 0.2% to 1.0%, or from 0.3% to 0.8%, or from 0.3% to 0.75%, reported as functional group equivalent weight %. The amine content and functional group equivalent weight percentage can be determined according to the methods provided in the Test Methods section. Nitrogen levels that are too low may lead to poor PRM loading; nitrogen levels that are too high may lead to increases in water solubility and/or poorer performance.

A precursor silicone polymer may be obtained from the combination of an appropriately functionalized homopolymer, copolymer, or higher oligomer silicone and an appropriate matching small molecule additive (e.g. amine, oxirane, alkanolamine, thiirane etc.), with or without a cosolvent catalyst (alcohol, diol, acids, bases), with or without external stimuli (e.g. pressure, heat, mechanical stirring), with or without catalyst, and with or without purification.

In the present disclosure a silicone polymer precursor may be obtained from polymerization of individual silane

compounds to reach a silicone polymer as described in Formula IX of the appropriate composition and functionality.

Method of Making a Pro-Fragrance Silicone Polymer and Related Premixes

The present disclosure also relates to methods of making pro-fragrance silicone polymers according to the present disclosures, as well as related premixes.

The present disclosure relates to a method of making a pro-fragrance silicone polymer. The method may comprise the step of combining a precursor silicone polymer as described above with a suitable perfume raw material, namely one that comprises an aldehyde moiety, a ketone moiety, or a combination thereof.

The combining step may occur at any suitable point, for example in a premix, in a base composition, or even on a surface or article, such as a fabric article, which may be accompanied by the removal of water (e.g., via a drying process).

The precursor silicone polymer and the one or more perfume raw materials may be combined in a treatment composition, for example by adding each as separate inputs to a base composition.

The precursor silicone polymer and the perfume raw material may be combined in a liquid premix composition. Thus, the present disclosure relates to such liquid premix compositions and methods of making such liquid premix compositions.

The liquid premix compositions of the present disclosure (also referred to herein as "premix compositions" or even just "premixes" or "a premix") may be useful components of treatment compositions and may help to improve perfume delivery and performance of those compositions compared to products where such a premix is not used. Further, it is believed that combining the ingredients in a premix provide more efficient perfume delivery and performance in a treatment compared to if the ingredients are added separately (e.g., not as a premix) to the consumer product, particularly in aqueous consumer products. Additionally, premix compositions may be conveniently made in advance and stored and/or transported between locations as desired, or even made by a third party prior to incorporation into a treatment composition.

The present disclosure relates to a method of making a premix composition as described herein. The method comprises the steps of: providing a precursor silicone polymer, which may comprise one or more radicals according to Formula VI, Formula VII, or mixtures thereof, as described in more detail above; and combining the precursor silicone polymer with a perfume raw material that comprises a moiety selected from the group consisting of an aldehyde moiety, a ketone moiety, and a combination thereof. As described in more detail below, the precursor silicone polymer may be combined with an emulsifier, preferably a nonionic surfactant, to form an oil-in-water emulsion, to which the PRM may be added.

The liquid premix composition may comprise: (a) a pro-fragrance silicone polymer as described herein, for example a pro-fragrance silicone polymer comprising at least one radical selected from the group consisting of Formula I, Formula II, and mixtures thereof, as described in more detail above, and optionally further comprising one or more free perfume raw materials; or (b) a precursor silicone polymer, which may comprise one or more radicals according to Formula VI, Formula VII, or combinations thereof, as described in more detail above, and a perfume raw material that comprises a moiety selected from the group consisting of an aldehyde moiety, a ketone moiety, and a combination thereof, wherein the precursor silicone polymer and the perfume raw material are capable of condensing to form the pro-fragrance silicone polymer; or (c) a mixture thereof.

The liquid premix composition may comprise, by weight of the liquid premix composition, the precursor silicone polymer present at a level of from 1% to 99%, preferably from 80% to 10%, or from 75% to 20%, or from 60% to 40%, and the perfume raw material present at a level of from 1% to 50%, preferably from 5% to 50%, or from 5% to 30%, or from 5% to 20%, or from 5% to 15%, or from 5% to 10%. The PRMs may be present, by weight of the liquid premix composition, of from 10% to 40%, or from 15% to 30%, or from 15% to 25%. An optional amount of emulsifier may be present at a level from 0% to 10%, preferably from 1% to 5%, or from 2% to 4%. The weight ratio of the precursor silicone polymer and the perfume raw material may be from 99:1 to 1:1, preferably from 50:1 to 2:1, more preferably from 10:1 to 5:1. Preferred amounts and/or ratios are desirable to achieve convenient and efficient payload delivery.

The premix compositions are liquids. The liquid premix composition may be characterized by a viscosity, for example from 10 to 10000 Pa·s, preferably from 10 to 5000 Pa·s, preferably from 10 to 1000 Pa·s, preferably from 10 to 500 Pa·s, preferably from 20 to 400 Pa·s, more preferably from 25 to 300 Pa·s, even more preferably from 100 to 300 Pa·s, measured at 0.1 rad/s and 25°C. Obtaining a liquid premix composition with the target viscosity may be desirable for processability reasons, particularly as premixes having a very high viscosity may be difficult to formulate into a product.

The liquid premix composition may be substantially free of water. The premix composition may comprise less than 10%, or less than 5%, or less than 2%, or less than 1%, or less than 0.5%, or less than 0.1%, or even comprise 0%, by weight of the premix composition of water. Such low-water premixes may be desirable, for examples, when the premix will be formulated into a low-water consumer product, such as a solid composition (particularly when water-soluble) or a composition encapsulated by water-soluble film. In such cases, the silicone polymer may be a fluid that is the bulk of the premix.

The fragrance premix composition may comprise water. The fragrance premix composition may comprise from 1% to 90%, or from 1% to 75%, or from 1% to 60%, or from 1% to 50%, or from 5% to 50%, or from 5% to 25%, or from 5% to 15%, or from 5% to 10%, by weight of the composition, of water. The fragrance premix composition may comprise from 10% to 50%, or from 25% to 50%, by weight of the composition of water. The presence of water may facilitate the formation of droplets, in view of the silicone polymer being relatively hydrophobic, which can facilitate more convenient dispersion of the premix in a treatment composition, particularly those that are aqueous.

The liquid premix composition may be in the form of an emulsion. The emulsion may preferably be an oil-in-water emulsion. The emulsion may comprise a plurality of droplets, preferably comprising droplets characterized by a volume-weighted average particle size of from 100 nm to 100 μm, more preferably from 1 μm to 10 μm, even more preferably from 1 μm to 5 μm. Without wishing to be bound by theory, it is believed that droplets of a certain minimum size are desired for efficiency of deposition onto the target surface, but that droplets that exceed a certain size can create performance and/or stability issues, such spotting on the target surface.

The liquid premix composition may comprise one or more emulsifiers. As used herein, "emulsifier(s)" and "emulsifying agent(s)" are used interchangeably. Selection of proper emulsifier can facilitate the formation of droplets of the desired size, and/or the stable incorporation of the premix into a final product. Emulsifiers may also be selected so as to not have an undesirable impact on viscosity of the emulsion, for example by increasing the viscosity to an undesirable level.

The emulsifier may be present at a level of from 0.5% to 40%, preferably from 1% to 30%, more preferably from 1% to 20%, more preferably from 2% to 20%, more preferably from 2% to 10%, by weight of the liquid premix composition. The emulsifier may be present in an amount of from 1% to 10%, more preferably from 1% to 5% by weight of the liquid premix composition. The weight ratio of the precursor silicone polymer to the emulsifier may be from 99:1 to 1:1, preferably from 50:1 to 2:1, more preferably from 15:1 to 5:1. The liquid premix composition may comprise from 20% to 40% water, and from 1% to 5% emulsifier, by weight of the liquid premix composition.

The one or more emulsifiers may comprise a nonionic surfactant. Suitable nonionic surfactant may include alkoxylated fatty alcohols. The nonionic surfactant may be selected from ethoxylated alcohols and ethoxylated alkyl phenols of the formula $R(OC_2H_4)_nOH$, wherein R is selected from the group consisting of aliphatic hydrocarbon radicals containing from 8 to 15 carbon atoms and alkyl phenyl radicals in which the alkyl groups contain from 8 to 12 carbon atoms, and the average value of n is from 5 to 15.

The one or more emulsifiers may comprise linear emulsifiers, branched emulsifiers, or mixtures thereof, preferably linear nonionic surfactants, branched nonionic surfactants, or mixtures thereof. In particular, linear emulsifiers may be useful for emulsifying the perfume raw materials, and branched emulsifiers may be useful for emulsifying the silicone polymer, particularly amino-modified silicone polymers.

The one or more emulsifiers may be substantially hydrophobic. The one or more emulsifiers may be characterized by an HLB value of from 5 to 20, or from 8 to 16. The HLB value of a nonionic surfactant may be determined according to the method provided below.

The liquid premix compositions of the present disclosure may further comprise an aminofunctional material as an additional ingredient. It is believed that the aminofunctional material can associate with the perfume raw materials and perhaps even the silicone materials described herein and facilitate the deposition and release of the perfume when used as part of a treatment composition.

The liquid premix composition may comprise from 1% to 20%, or from 2% to 15%, or from 3% to 12%, or from 4% to 10%, or from 5% to 10%, by weight of the fragrance premix composition, of the aminofunctional material.

The aminofunctional material may be characterized by a relatively low molecular weight. Relatively low molecular weights may be preferred for mass efficiency reasons (e.g. a favorable / high ratio of amine groups to molecular weight). For example, the aminofunctional material may be characterized by a molecular weight of 17 to 1000 Daltons, more preferably from 30 to 1000 Daltons, 40 to 1000 Daltons, preferably from 50 to 800 Daltons, more preferably from 60 to 600 Daltons, even more preferably from 60 to 500 Daltons.

The aminofunctional material may comprise one, two, or three amine moieties per molecule, preferably one or two amine moieties. The amine moiety may be selected from the group consisting of a primary amine moiety, a secondary amine moiety, or a combination thereof. It is believed that primary and/or secondary amine moieties may better associate with the PRMs compared to tertiary and/or quaternary amine moieties. Furthermore, two or even three amine moieties may provide improved association / loading in combination with the perfume raw materials, compared to compounds having only one amine group. However, as described in more detail below, there may be a desire to limit the number of amine groups.

It is believed that certain aminofunctional materials are likely to work better than others in the compositions of the present disclosure due to their amine moiety type and/or structure. For example, the aminofunctional material may be characterized by one of the following: (a) comprising a total of one primary amine moiety and no secondary amine moieties; or (b) comprising a total of two primary amine moieties and no secondary amine moieties; or (c) one primary amine moiety and one secondary amine moiety, preferably where the primary amine moiety and the secondary amine moiety are

separated by two carbon atoms; or (d) one primary amine moiety or secondary amine moiety that is separated by two carbon atoms from a hydroxyl group.

The aminofunctional material may be selected from: linear aliphatic aminofunctional materials, such as octylamine, nonylamine, and/or decylamine; branched aliphatic aminofunctional materials, such as 2-ethylhexylamine, branched tridecylamine, t-butylamine, 2-(diethylamino) ethylamine, neopentanediamine (2,2-dimethyl propane-1,3-diamine), 3-methoxyethylamine, trimethyl-1,6-hexanediamine, 2-aminoheptane, and/or 2-butyloctylamine; cycloaliphatic amines, including methylcyclohexane diamines such as 2-methylcyclohexane-1,3-diamine and/or 4-methylcyclohexane-1,3-diamine; aminofunctional silanes, such as trialkoxy(aminoethylaminopropyl)silane, alkyl dialkoxy(aminoethylaminopropyl)silane, dialkyl alkoxy(aminoethylaminopropyl)silane, trialkoxy(aminopropyl)silane, alkyl dialkoxy(aminopropyl)silane, and/or dialkyl alkoxy(aminopropyl)silane; aminoalcohols, such as 2-(butylamino)ethanol, 1-(cyclohexylamino) 2-propanol, 1-(dodecyloxy)-3-[(2-hydroxyethyl)amino]-2-propanol, and/or 3-(dodecylamino)-1,2-propanediol; 1,3-bis(3-aminopropyl) tetramethyldisiloxane; or mixtures thereof.

The aminofunctional material may be substantially free of aromatic amines (e.g., where an aminofunctional moiety is directly attached to an aromatic ring), or even substantially free of aromatic moieties altogether, as such moieties tend to increase the solubility of the aminofunctional material and therefore may make it less likely to associate with the hydrophobic silicone.

The liquid premix composition may be made according to any suitable process. For example, the present disclosure relates to a process of making a premix composition, such as those described herein, where the process includes the steps (preferably in order) of: providing the silicone polymer precursor; adding the aminofunctional material, if any; and adding the perfume raw materials; where each is provided in the relative amounts described above. Mixing may be provided throughout or intermittently. The resulting mixture may be mixed with sufficient mixing energy to combine the materials.

When it is desired that the fragrance premix composition is in the form of an emulsion, the process may include the steps (preferably in order) of: providing the silicone polymer precursor; adding the aminofunctional material, if any; adding an emulsifying agent; adding the water; and adding the perfume raw material; where each is provided in the relative amounts described above. Mixing may be provided throughout or intermittently. The resulting mixture may be mixed with sufficient mixing energy to combine and emulsify the materials, for example to form the droplets described above.

The process may include the steps (preferably in order) of: providing the silicone polymer precursor; adding an emulsifying agent; adding the water; adding the aminofunctional material, if any; and adding the perfume raw materials; where each is provided in the relative amounts described above. Mixing may be provided throughout or intermittently. The resulting mixture may be mixed with sufficient mixing energy to combine and emulsify the materials, for example to form the droplets described above.

Certain components may be premixed, for example, the silicone polymer precursor and an emulsifying agent, and/or the fragrance material and an emulsifying agent. The emulsifying agents for each material may be the same, or they may be different.

Treatment Compositions

The present disclosure also relates to treatment compositions that include pro-fragrance silicone polymers as described above, as well as methods of making and using such treatment compositions. The treatment compositions include a treatment adjunct.

The treatment compositions may be consumer product compositions. The consumer product compositions may be useful for treating a surface, for example to freshen and/or condition the surface, such as fabric, hair, or skin. Suitable consumer product compositions are described above. Preferred consumer product compositions may include baby care compositions, personal care compositions, fabric care compositions, home care compositions, family care compositions, and/or feminine care compositions, preferably fabric care compositions and/or home care compositions, more preferably fabric care compositions.

The treatment compositions may comprise a liquid premix composition, for example in emulsion form, according to the present disclosure and a treatment adjunct. The treatment compositions may be made by providing a premix composition according to the present disclosure, and combining the premix with a treatment adjunct. The treatment adjunct may be part of a base composition.

The treatment compositions may comprise a pro-fragrance silicone polymer according to the present disclosure at a level of from 0.01% to 99.9%, by weight of the treatment composition, preferably from 0.01% to 50%, more preferably from 0.01% to 25%, more preferably from 0.1% to 10%, more preferably from 0.1% to 5%, more preferably from 0.2% to 5%, by weight of the treatment composition. The levels may be dictated by the intended final use and form of the treatment composition.

The treatment compositions described herein may comprise from 0.1% to 20%, or from 0.1% to 15%, or from 0.1% to 10%, or from 0.1% to 5%, or from 0.1% to 3%, by weight of the treatment composition, of a liquid premix

composition according to the present disclosure.

The treatment composition may comprise the following components at one or more of the following levels, where the component(s) is provided by a liquid premix, and where the weight percentages are by weight of the treatment composition: from 0.1% to 20% of the silicone polymer, and/or from 0% to 10% of the aminofunctional material (if any), and/or from 0.05% to 20% of one or more perfume raw materials.

The present disclosure also relates to a treatment composition that comprises a treatment adjunct and a plurality of droplets, wherein the droplets comprise: a silicone polymer precursor as described above; optionally an aminofunctional material as described above; optionally emulsifiers as described above; and a perfume raw material as described above; where the components are present in the droplet in the relative amounts as described above. Additionally or alternatively, the droplets may comprise a pro-fragrance silicone polymer according to the present disclosure. The droplets may be present in the treatment composition as a result of combining a liquid premix composition as described herein with a treatment adjunct. The plurality of droplets may be characterized as having a volume-weighted average diameter of from about 1 micron to about 10 microns, preferably from about 1 micron to about 5 microns.

The treatment compositions according to the present disclosure may be in the form of a liquid composition, a granular composition, a single-compartment pouch, a multi-compartment pouch, a dissolvable sheet, particulate form where individual particles have a mass of from about 1mg to about 1 gram (such as a pastille or bead, which may preferably be water-soluble), a fibrous article, a tablet, a bar, a flake, a non-woven sheet, or a mixture thereof.

The treatment compositions of the present disclosure may be a household care composition, preferably a household care composition selected from the group consisting of a fabric and home care product, a beauty care product, or a mixture thereof.

When the treatment composition is a fabric and home care product, the fabric and home care product may preferably be selected from a laundry detergent composition, a fabric conditioning composition, a fabric pre-treatment composition, a fabric refresher composition, or a mixture thereof. The fabric conditioning composition may preferably be a liquid fabric conditioning composition.

When the treatment composition is a beauty care product, the beauty care product may preferably be selected from a hair treatment product, a skin care product, a shave care product, a personal cleansing product, a deodorant and/or antiperspirant, or a mixture thereof. The hair treatment composition preferably may preferably be a shampoo, a conditioner, or a combination thereof.

The treatment composition includes a treatment adjunct, in addition to the fragrance premix composition and/or droplets. The treatment adjunct may be any adjunct ingredient, in any amount, that is suitable for the intended product and/or intended end-use of the product. The treatment composition may be made by a method that comprises the step of combining the fragrance premix composition with the treatment adjunct.

The treatment adjunct may be part of a base composition that is combined with the liquid premix composition. For example, the present disclosure relates to a method of making a treatment composition that includes the step of combining a premix composition with a base composition, where the base composition comprises a treatment adjunct. The premix composition may be added to the base composition. Treatment adjuncts may be added to the base composition before and/or after the premix composition is added to the base composition. It is understood that as used herein, a "base composition" is an intermediate composition, to which other ingredients may be added to form a completed or final treatment composition.

Treatment adjuncts may be useful as performance aids, stability or processing aids, or both. For example, the treatment adjunct may be selected from an amine, a surfactant system, a water-binding agent, a sulfite, fatty acids and/or salts thereof, enzymes, encapsulated benefit agents, soil release polymers, hueing agents, builders, chelating agents, dye transfer inhibiting agents, dispersants, enzyme stabilizers, catalytic materials, bleaching agents, bleach catalysts, bleach activators, polymeric dispersing agents, soil removal/anti-redeposition agents, polymeric dispersing agents, polymeric grease cleaning agents, brighteners, suds suppressors, dyes, hueing agents, free perfume, structure elasticizing agents, conditioning or softening agents, carriers, fillers, hydrotropes, organic solvents, anti-microbial agents and/or preservatives, neutralizers and/or pH adjusting agents, processing aids, fillers, rheology modifiers or structurants, opacifiers, pearlescent agents, pigments, anti-corrosion and/or anti-tarnishing agents, malodor agents, and mixtures thereof. While one of ordinary skill will generally be familiar with these adjuncts, a few of the adjuncts are described in more detail below.

The treatment compositions may include surfactant. Surfactants may be useful for providing, for example, cleaning benefits. The compositions may comprise a surfactant system, which may contain one or more surfactants.

The compositions of the present disclosure may include from 1% to 70%, or from 2% to 60%, or from 5% to 50%, by weight of the composition, of a surfactant system. Liquid compositions may include from 5% to 40%, by weight of the composition, of a surfactant system. Compact formulations, including compact liquids, gels, and/or compositions suitable for a unit dose form, may include from 25% to 70%, or from 30% to 50%, by weight of the composition, of a surfactant system.

The surfactant system may include anionic surfactant, nonionic surfactant, zwitterionic surfactant, cationic

surfactant, amphoteric surfactant, or combinations thereof. The surfactant system may include linear alkyl benzene sulfonate, alkyl ethoxylated sulfate, alkyl sulfate, nonionic surfactant such as ethoxylated alcohol, amine oxide, or mixtures thereof. The surfactants may be, at least in part, derived from natural sources, such as natural feedstock alcohols.

Suitable anionic surfactants may include any conventional anionic surfactant. This may include a sulfate detersive surfactant, for e.g., alkoxylated and/or non-alkoxylated alkyl sulfate materials, and/or sulfonic detersive surfactants, e.g., alkyl benzene sulfonates. The anionic surfactants may be linear, branched, or combinations thereof. Preferred surfactants include linear alkyl benzene sulfonate (LAS), alkyl ethoxylated sulfate (AES), alkyl sulfates (AS), or mixtures thereof. Other suitable anionic surfactants include branched modified alkyl benzene sulfonates (MLAS), methyl ester sulfonates (MES), sodium lauryl sulfate (SLS), sodium lauryl ether sulfate (SLES), and/or alkyl ethoxylated carboxylates (AEC). The anionic surfactants may be present in acid form, salt form, or mixtures thereof. The anionic surfactants may be neutralized, in part or in whole, for example, by an alkali metal (e.g., sodium) or an amine (e.g., monoethanolamine).

The surfactant system may include nonionic surfactant. Suitable nonionic surfactants include alkoxylated fatty alcohols, such as ethoxylated fatty alcohols. Other suitable nonionic surfactants include alkoxylated alkyl phenols, alkyl phenol condensates, mid-chain branched alcohols, mid-chain branched alkyl alkoxylates, alkylpolysaccharides (e.g., alkylpolyglycosides), polyhydroxy fatty acid amides, ether capped poly(oxyalkylated) alcohol surfactants, and mixtures thereof. The alkoxylate units may be ethyleneoxy units, propyleneoxy units, or mixtures thereof. The nonionic surfactants may be linear, branched (e.g., mid-chain branched), or a combination thereof. Specific nonionic surfactants may include alcohols having an average of from 12 to 16 carbons, and an average of from 3 to 9 ethoxy groups, such as C12-C14 EO7 nonionic surfactant.

Suitable zwitterionic surfactants may include any conventional zwitterionic surfactant, such as betaines, including alkyl dimethyl betaine and cocodimethyl amidopropyl betaine, $C_8$ to $C_{18}$ (for example from $C_{12}$ to $C_{18}$) amine oxides (e.g., $C_{12-14}$ dimethyl amine oxide), and/or sulfo and hydroxy betaines, such as N-alkyl-N,N-dimethylammino-1-propane sulfonate where the alkyl group can be $C_8$ to $C_{18}$, or from $C_{10}$ to $C_{14}$. The zwitterionic surfactant may include amine oxide.

Depending on the formulation and/or the intended end-use, the composition may be substantially free of certain surfactants. For example, liquid fabric enhancer compositions, such as fabric softeners, may be substantially free of anionic surfactant, as such surfactants may negatively interact with cationic ingredients.

The treatment compositions may include conditioning actives. Compositions that contain conditioning actives may provide softness, anti-wrinkle, anti-static, conditioning, anti-stretch, color, and/or appearance benefits.

Conditioning actives may be present at a level of from 1% to 99%, or from 1% to 35%, or from 1% to 20%, or from 1% to 15%, or from 1% to 10%, or from 1% to 6%, by weight of the composition. The composition may include from 1%, or from 2%, or from 3%, to 99%, or to 75%, or to 50%, or to 40%, or to 35%, or to 30%, or to 25%, or to 20%, or to 15%, or to 10%, by weight of the composition, of conditioning active. The composition may include from 5% to 30%, by weight of the composition, of conditioning active.

Conditioning actives suitable for compositions of the present disclosure may include quaternary ammonium ester compounds, silicones, non-ester quaternary ammonium compounds, amines, fatty esters, sucrose esters, dispersible polyolefins, polysaccharides, fatty acids, softening or conditioning oils, polymer latexes, or combinations thereof, preferably at last quaternary ammonium ester compounds, as such materials are known to provide useful conditioning benefits while also being relatively environmentally friendly.

The composition may include a quaternary ammonium ester compound, a silicone, or combinations thereof, preferably a combination. The combined total amount of quaternary ammonium ester compound and silicone may be from 5% to 70%, or from 6% to 50%, or from 7% to 40%, or from 10% to 30%, or from 15% to 25%, by weight of the composition. The composition may include a quaternary ammonium ester compound and silicone in a weight ratio of from 1:10 to 10:1, or from 1:5 to 5:1, or from 1:3 to 1:3, or from 1:2 to 2:1, or 1:1.5 to 1.5:1, or about 1:1.

The composition may contain mixtures of different types of conditioning actives. The compositions of the present disclosure may contain a certain conditioning active but be substantially free of others. For example, the composition may be free of quaternary ammonium ester compounds, silicones, or both. The composition may comprise quaternary ammonium ester compounds but be substantially free of silicone. The composition may comprise silicone but be substantially free of quaternary ammonium ester compounds.

The compositions of the present disclosure may contain a rheology modifier and/or a structurant. Rheology modifiers may be used to "thicken" or "thin" liquid compositions to a desired viscosity. Structurants may be used to facilitate phase stability and/or to suspend or inhibit aggregation of particles or droplets in liquid compositions, such as the droplets of the emulsions as described herein. Suitable rheology modifiers and/or structurants may include non-polymeric crystalline hydroxyl functional structurants (including those based on hydrogenated castor oil), polymeric structuring agents, cellulosic fibers (for example, microfibrillated cellulose, which may be derived from a bacterial, fungal, or plant origin, including from wood), di-amido gellants, or combinations thereof.

The treatment compositions made from the presently described methods may include free perfume. To provide a

broader and more diverse scent profile, it may be desirable to include perfume raw materials in the free perfume of the treatment composition that are not present in the pro-fragrance silicone, and/or vice versa. For example, when the pro-fragrance silicone polymer comprises a fragment or residue of one or more perfume raw materials that comprise an aldehyde moiety, the free perfume of the treatment composition may comprise one or more perfume raw materials that do not comprise an aldehyde moiety. Similarly, when the pro-fragrance silicone polymer comprises a fragment or residue of one or more perfume raw materials that comprise a ketone moiety, the free perfume of the treatment composition may comprise one or more perfume raw materials that do not comprise a ketone moiety. The free perfume may include perfume raw materials that include aldehyde moieties, perfume raw materials that do not include aldehyde moieties, perfume raw materials that include ketone moieties, perfume raw materials that do not include ketone moieties, or mixtures thereof.

The treatment composition may comprise a carrier material. The carrier material may be selected from the group consisting of water, silica, zeolite, carbonate, polyvinyl alcohol, polyethylene glycol, sodium acetate, sodium bicarbonate, sodium chloride, sodium silicate, polypropylene glycol polyoxoalkylene, polyethylene glycol fatty acid ester, polyethylene glycol ether, sodium sulfate, starch, and mixtures thereof. The carrier material may be selected based on the desired final form of the consumer product; for example, a liquid product may use water as a carrier, whereas a powdered or particle product may use carbonate or polyethylene glycol (PEG).

The base composition may be in the form of a liquid. The base composition may comprise water. The base composition may comprise from 1% to 99%, preferably from 5% to 98%, or from 10% to 95%, or from 50% to 95%, or from 60% to 95%, or from 75% to 95%, by weight of the base composition, of water.

The treatment composition may be in the form of a liquid. The treatment composition may comprise water. The treatment composition may comprise from 1% to 99%, preferably from 5% to 98%, or from 10% to 95%, or from 50% to 95%, or from 60% to 95%, or from 75% to 95%, by weight of the treatment composition, of water. Certain unit dose formulations may have relatively low amounts of water so as to not dissolve the water-soluble film; for example, the composition may comprise no more than 20%, or no more than 15%, or no more than 12%, or no more than 10%, by weight of the composition, of water. The fragrance premixes of the present disclosure may be particularly useful in liquid compositions that include a relatively high amount of water, as it is believed the hydrophobicity of the silicone enables the silicone, aminofunctional material, emulsifiers, and fragrance material to partition from the water and to associate in the high-water matrix.

The liquid treatment composition may have a viscosity from 20 cps to 1000 cps (20-1000 mPa·s), preferably from 25 cps to 500 cps (25-500 mPa·s), more preferably from 50 cps to 300 cps (50 mPa·s to 300 mPa·s). The viscosity is determined using a Brookfield viscometer, No. 2 spindle, at 60 RPM/s, measured at about 22°C.

The treatment composition may be in a particulate form, such as a plurality of particulates. Individual particulates may have a mass from 1 mg to 1 g. The emulsion may be dispersed in a water-soluble carrier. The water-soluble carrier may be selected from the group consisting of polyethylene glycol, sodium acetate, sodium bicarbonate, sodium chloride, sodium silicate, polypropylene glycol polyoxoalkylene, polyethylene glycol fatty acid ester, polyethylene glycol ether, sodium sulfate, starch, and mixtures thereof. The water-soluble carrier may be a water-soluble polymer. The treatment composition, when in particulate form, may comprise from 25wt% to 99.99wt% of the water-soluble carrier, and from 0.01wt% to 50wt% by weight the silicone premix. The particulate form may be in the form of a bead or pastille.

The treatment composition may be characterized by a pH level of from 2 to 12, or from 2 to 8.5, or from 2 to 7, or from 2 to 5, preferably from 2 to 4, preferably a pH of from 2 to 3.7, more preferably a pH from 2 to 3.5, wherein pH is determined by dissolving/dispersing the treatment composition in deionized water to form a solution at 10% concentration, at about 20°C.

## Methods of Making Treatment Compositions

The present disclosure relates to processes for making any of the treatment compositions described herein.

The method may comprise the steps of: providing a base composition, wherein the base composition comprises a treatment adjunct; and combining the base composition with one or more of the following: (a) a pro-fragrance silicone polymer as described herein, for example one comprising one or more radicals according to Formula I, Formula II, or mixtures thereof; (b) a precursor silicone polymer as described herein, for example one comprising one or more radicals according to Formula VI, Formula VII, or combinations thereof, and a perfume raw material that comprises a moiety selected from the group consisting of an aldehyde moiety, a ketone moiety, and a combination thereof, preferably wherein the precursor silicone polymer and the perfume raw material are provided as a liquid premix composition, preferably in emulsion form; and/or (c) a mixture thereof. The compositions described above may optionally be combined with an aminofunctional compound.

The precursor silicone polymer and the perfume raw material may preferably be provided as a premix composition, preferably a liquid premix composition, more preferably a liquid premix in emulsion form, to the base composition. The precursor silicone polymer and the perfume raw material may preferably be provided as separate inputs to the base composition. The separate inputs may occur concurrently or sequentially. When occurring sequentially, it is

preferred that the precursor silicone polymer, which may preferably be emulsified, is added prior to the addition of the perfume raw material. The mixture may be mixed to homogenize the resulting composition.

The treatment compositions of the present disclosure can be formulated into any suitable form and prepared by any process chosen by the formulator. The materials may be combined in a batch process, in a circulation loop process, and/or by an in-line mixing process. Suitable equipment for use in the processes disclosed herein may include continuous stirred tank reactors, homogenizers, turbine agitators, recirculating pumps, paddle mixers, high shear mixers, static mixers, plough shear mixers, ribbon blenders, vertical axis granulators and drum mixers, both in batch and, where available, in continuous process configurations, spray dryers, and extruders.

## Methods of Using Treatment Compositions

The present disclosure also relates to a method of treating a surface, where the method comprises the step of contacting the surface with a treatment composition described herein, optionally in the presence of water. Preferably, the surface is a fabric, hair, or skin, more preferably a fabric, even more preferably a garment.

The processes of the present disclosure may include diluting the composition with water to form a treatment liquor, which may contact the surface to be treated. The composition may be diluted from 100-fold to 1000-fold, or from 200-fold to 900-fold, or from 300-fold to 800-fold, by water.

The contacting step may occur in the drum of an automatic washing machine. The contacting step may occur as part of, or shortly before, a wash cycle; for example, the consumer product may be a detergent composition or may be added substantially concurrently with a detergent composition. The contacting step may occur as part of a rinse cycle, which may follow a wash cycle; for example, the consumer product may be a fabric enhancer product, such as a liquid fabric enhancer product, and may contact the surface subsequent to the surface having been treated by a detergent product.

The contacting step may occur as a pretreatment step, for example prior to a wash cycle.

## Uses

The present disclosure relates to the use of a pro-fragrance silicone polymer according to the present disclosure as a perfuming agent. The pro-fragrance silicone polymer may be used as a perfuming agent on a surface, preferably on a fabric. For such uses, the pro-fragrance silicone polymer may optionally be formulated in a treatment composition, as described in more detail above.

## Article and/or Surface

The present disclosure relates to an article, preferably a fabric article, comprising a surface, where the surface comprises a pro-fragrance silicone polymer according to the present disclosure. The present disclosure also relates to an article, preferably a fabric article or a dryer sheet or an absorbant article (such as a diaper or incontinence product), that has been treated with a treatment composition according to the present disclosure, preferably according to a treatment method as described herein.

The present disclosure also relates to a surface, such as a hard surface, comprising a pro-fragrance silicone polymer according to the present disclosure. The present disclosure also relates to a surface, preferably a hard surface, that has been treated with a treatment composition according to the present disclosure, preferably according to a treatment method as described herein.

# TEST METHODS

## Preparation of a Pro-Fragrance Silicone Polymer Premix Emulsion

A pro-fragrance silicone polymer premix emulsion may be prepared as follows.

Starting with 60.0 parts by weight of the siloxane precursor compound, add Surfonic L24-9 (2.0 parts; ex Huntsman Holland BV) and Tergitol™ 15-S-40 (2.5 parts; ex The Dow Chemical Company). The mixture is mixed for 1 minute with an IKA RW 20 at 800 rpm. Water (35.5 parts in total) is added in two equal, separate additions; after each water addition, the mixture mixed with the IKA RW 20 for approximately 10-15 min.

Perfume raw material, added in an approximately equal molar equivalent to the molar amount of radicals present according to Formulas VI and/or VII in the precursor silicone polymer, is added to the emulsion and stirred for 15 minutes with an IKA RW 20 at 275 rpm.

As an illustrative example, 93.5 parts by weight of a silicone fluid as disclosed in Synthesis Example 1 below, emulsified as described above, is combined with 6.5 parts of a perfume raw material (e.g., cinnamic aldehyde).

Preparation of a Test Fabric Enhancer/Softener Composition

To an 7.5 wt % N,N di(tallowoyloxyethyl)-N,N dimethylammonium chloride in water mixture, a pro-fragrance silicone polymer premix emulsion (or neat perfume oil) is added in an amount such that the concentration of the perfume raw material or perfume raw material fragment in the fabric softener is about 0.3 wt % after the fabric softener preparation. The mixture is stirred for 5 min with an IKA RW 20 D SI Mixer, Model RW20DS1, and IKA RI 342 impeller blade at 350 rpm. A structurant and a deposition aid is added, and the mixture is stirred for 10 min. Water is added if needed to standardize the concentration of N,N di(tallowoyloxyethyl)-N,N dimethylammonium chloride amongst test legs to 7.3 wt %, and the mixture stirred for 5 min. The pH is adjusted to 2-3 with in HCl, if necessary.

Preparation of a Test Pastille Composition

To a 93.88 wt% molten PEG-8000 material in a speed mixer cup is added 6.12% the pro-fragrance silicone premix emulsion of Synthetic Example 6. The speed mixer cup is quickly placed in a Flacktek DA150.FVZ-K speed mixer for 1min at 3,500 rpm. Sample pastilles were immediately made from the mixture by pouring into blue siliconized rubber molds that were preequilibrated to 4°C and spread with a 10" plastic taping knife. The pastilles were cooled at room temperature for approximately 30 min, then the pastilles were removed from the mold and stored under ambient conditions.

Fabric Preparation Method

To prepare fabrics for Headspace analysis testing, fabric samples (100% Cotton Terry Cloth, Item Number ITL 1022-15PGP, CalderonTextiles, Inc. 6131 W. 80tA St., Indianapolis, Ind. 46278, Desized and conditioned with 3 wash cycles of Detergent and Fabric Softener) are treated with the Fabric Softener formulas in a manner consistent with North American consumers via clothes mini-washing machines, full scale machines, and clothes dryers. Fabric is equilibrated at 21.1° C. and 50% Relative Humidity for 24 hours prior to Headspace GCMS analysis (see methods below). Ballast loads are comprised of cotton and polycotton knit swatches approximately 20x20 inches (50x50cm) in size.

Wash Treatment Conditions

In the pastille composition performance tests below, the fabrics are treated with the following wash treatment conditions: North America Kenmore 600 Series top-loading washing machines are used. Each machine is set to run a Normal single cycle including a 12-minute wash agitation period, and 1 three-minute rinse. The water used is 137 ppm hardness and 30.6°C for the wash, and 15.5 $^0$C for the rinse. The water volume at each step is 64 Liters. The total fabric load weight is 3.6 kg (which included 32 test fabric hand towel terry cloths, 9 of 100% cotton ballast, and about 5 of 50/50 polycotton ballast). The detergent used is TIDE Original Scent liquid without perfume (produced by The Procter & Gamble Company). Detergent is dosed at 81 g into the wash water while the wash water is filling. After the detergent is added, 25 g of the pastilles being evaluated are also added, followed by the fabric load. After the water fill is complete, the machine enters the agitation period. This is followed by the wash agitation (Normal setting), and the rinse step (with corresponding spin cycle). After the wash process is completed, the fabrics are removed. The test fabrics are machine dried in Kenmore driers on Cotton/High setting, for 50 minutes. The test fabrics are then equilibrated for 24 hours in a 21.1°C/ 50% relative humidity controlled room.

In the fabric enhancer/softener compositions performance tests below, the fabrics are treated with the following wash treatment conditions: Wash: 12 min agitation, 30.6° C. Rinse: 2 min agitation, 15.5° C. Water Hardness: 137 ppm. Water: 7.6 pH. Fabric Load Weight: 290 g. Tumble Dry Setting: 50 min High, Cotton. Detergent Dose: 9.65 g. Fabric Softener Dose: 5.71 g.

Headspace Analysis Above Fabrics

To determine the level of perfume raw material in the headspace above a fabric, the following procedure is used.

The following equipment is used: Gas Chromatograph 7890B equipped with a Mass Selective Detector (5977B) (MSD) and Chemstation quantitation package; Gerstel Multi-Purpose sampler equipped with a solid phase micro-extraction (SPME) probe or similar system; Divinylbenzene/Carboxen/Polydimethylsiloxane SPME fiber from Supleco part #57298-U (or similar fiber); 30 mx0.25 mm nominal diameter, 0.25 m film thickness, J&W 122-5532UI DB-5; 20 mL headspace vials.

To prepare the fabric for analysis, cut three 2.54 cm x 5.08 cm cotton swatches from the cotton terry that is prepared and treated according to the above methods. Place each piece in a 20 mL headspace vial and cap.

The Gerstel auto sampler parameters are as follows: SPME-from Incubator; Incubation Temperature- 65° C.; Incubation Time-10.00 min SAMPLE PARAMETERS; Vial Penetration-22.00 mm; Extraction Time-5. 00 min; Ini.

Penetration-54.00 mm; Desorption Time- 300 s. The GC oven parameters are as follows for the Front SS Inlet He: Mode-Splitless; Heater-270° C.; GC Run Time-14.28 min. For the Oven: Initial temp.-40° C.; Hold Time-0.5 min; Heating Program-Rate of 17° C./min, Temp of 270° C., Hold Time of 0.25 min. The MSD parameters are as follows: Run in scan mode with a minimum range of 35 to 350 m/z;

Calibration curves are generated from the standards perfume material. Chemstation software (or similar quantitation software) calculates the mass amount in the headspace using the calibration curve for each perfume component.

Color Change of a Composition

A premix (e.g., a premix emulsion) or treatment composition may be tested for color changes according to the following procedure. The reflectance spectra and color measurements, including L*, a*, and b* were made using the LabScan XE reflectance spectrophotometer (HunterLabs, Reston, VA; D65 illumination, 10° observer, UV light excluded). L*, a* and b* values for premix emulsions and treatment compositions are measured at time t = 0 and 14 days after mixing in the PRM. The total color change (ΔE) of a premix emulsion or treatment composition is calculated based on the data collected at each time point t using the following equation:

$$\Delta E_t = [(L^*c - L^*s)^2 + (a^*c - a^*s)^2 + (b^*c - b^*s)^2]^{1/2}$$

wherein the subscripts c and s respectively refer to the control, i.e., the premix emulsion or treatment composition with nil PRM, and the sample, i.e., the premix emulsion or treatment composition with respective aldehyde/ketone PRM, where the values used to calculate $\Delta E_t$ are those at the corresponding time points t (0, 14 days). The desired PRM is slowly added to a sample of described functionalized silicones (for example, in relative amounts sufficient to provide 1:1 molar equivalence of amine groups in the silicone to aldehyde or ketone groups of the perfume) in ajar with overhead mixing with a fourblade IKA RW 20 impeller and gently mixed for 15 minutes. The premix emulsion mixture or treatment composition is placed into a 50 mL (25 $cm^2$) CELLSTAR® cell culture flask with standard screw cap. At t= 0 and after 14 days at room temperature, color appearance of each premix emulsion or treatment composition sample is measured on a LabScan XE 10 reflectance spectrophotometer (HunterLabs, Reston, VA; D65 illumination, 10° observer, UV light excluded).

Determination of Amine Content and % Nitrogen

Total amine content, primary amine content, and/or % nitrogen of an aminofunctional silicone is determined according to the following method. More specifically, this method is used to determine the primary, secondary and tertiary amine values (meq/g) which are defined as the milliequivalents of amine functionality (primary, secondary and tertiary) present in one gram of a sample.

The method is based on compendial method ASTM D2074-07, which should be used to supplement this method if necessary. In broad stokes, a sample is dissolved in isopropyl alcohol and is titrated to a bromophenol blue end point using a standardized HCl solution.

The following materials are used: 0.1N Hydrochloric Acid in isopropyl alcohol (CAS 7647-01-1, 67-63-0; 99.5%; ex Fisher Scientific); Isopropyl Alcohol (CAS # 67-63-0; 99%; ex EMD); Phenyl Isothiocyanate (CAS # 103-72-0; 98%; ex Sigma Aldrich); Salicylaldehyde (CAS# 90-02-8; 98%; ex Sigma Aldrich); Bromophenol Blue Indicator (0.1wt% solution in ethanol or isopropyl alcohol; ex. Fisher Scientific).

Each of the following titrations should be repeated a total of three times. Furthermore, titrant volumes must be determined empirically. Titrant volumes should be between 1 and 20mL. If titrant volumes are less than 1mL, weigh more sample. If samples are more than 20mL, weigh less sample. A buret such as Metrohm Dosimat 775 or equivalent may be used in the titrations. Regarding the yellow end point of the titrations - the yellow may fade back to green, but if it is a bright clear yellow, this is to be disregarded if additional 0.1N HCl does not change the original color.

A. Titration for Total Amine Content

Melt the sample (typically 100% active) in a water bath if it is not already a liquid. Mix thoroughly and accurately weigh out between 0.5 grams and 1.0 grams into a 250mL Erlenmeyer flask (wide mouth; alkali resistant). Record the weight to four decimal places.

To the flask, add 50mL of isopropyl alcohol. Add 0.5mL of bromophenol blue indicator. Titrate with 0.1N HCl solution while swirling until it reaches the yellow end point. Record the volume of HCl used as $V_{1,2,3}$.

B. Titration for Secondary and Tertiary Amine Content

Melt the sample (typically 100% active) in a water bath if it is not already a liquid. Mix thoroughly and accurately weigh out 1.0 grams into two 250 Erlenmeyer flasks. Record the weight to four decimal places. Mark the flasks S and T, respectively. To each flask, add 50mL of isopropyl alcohol.

To flask S, add 1mL of salicylaldehyde. Stir the solution (with a magnetic stir bar) for 30 minutes. Add 0.5mL of bromophenol blue indicator solution and titrate while stirring with 0.1N HCl to a yellow end point. Record volume of HCl used as $V_{2\&3}$.

To flask T, add 1mL of phenyl isothiocyanate. Stir the solution (with a magnetic stir bar) for 30 minutes. Add 0.5mL of bromophenol blue indicator solution and titrate while stirring with 0.1N HCl to a yellow end point. Record volume of HCl used as $V_3$.

C. Calculations for Amine Content

The variables in the calculations described below correspond to the following:

| | |
|---|---|
| V : | HCl required for titration of specimen in mL |
| N : | normality of the HCl solution |
| S : | specimen weight in grams (g) |
| meq/g : | milliequivalents/gram |
| Total : | Total Amine Value |
| AS: | Amine value of the secondary and tertiary amine groups |
| TA : | Tertiary Amine Value |

Based on the measurements obtained related to the above titrations, the following calculations are used to determine the various amine contents.

$$\text{Total Amine Value (Total)} = \frac{(V_{1,2,3} * N)\ (\text{meq/g})}{S}$$

$$\text{Secondary and Tertiary Amine Value (AS)} = \frac{(V_{2,3} * N)\ (\text{meq/g})}{S}$$

$$\text{Tertiary Amine Value (TA)} = \frac{(V_3 * N)\ (\text{meq/g})}{S}$$

$$\text{Secondary Amine Value} = (AS - TA)$$

$$\text{Primary Amine Value} = (\text{Total} - AS)$$

D. Calculation of Nitrogen wt%

To determine the wt% of nitrogen in an aminofunctional silicone based on the amine content, use the following calculation.

The weight percentage of nitrogen in a compound can be calculated from the amine value (in meq/g) as follows:

$$(\text{Amine Value} / 1000) \text{ x } (\text{MW of Nitrogen}) \text{ x } 100 = \text{wt\% Nitrogen}$$

As an example, dimethylethanolamine has an amine value of 11.2 (in meq/g). Its weight percent of nitrogen (15.7wt%) is as follows:

$$(11.2 / 1000) \text{ x } (14.01) \text{ x } 100 = 15.7 \text{ wt\% nitrogen}$$

The following table (Table D) shows wt% of nitrogen and equivalent amine values.

Table D.

| Wt% Nitrogen | Amine Value (meq/g) |
|---|---|
| 0.1 | 0.071 |
| 0.2 | 0.14 |
| 0.3 | 0.21 |
| 0.5 | 0.36 |
| 0.7 | 0.50 |
| 0.8 | 0.57 |
| 1.0 | 0.71 |
| 1.5 | 1.07 |
| 2.0 | 1.43 |
| 2.5 | 1.78 |
| 3.0 | 2.14 |
| 3.5 | 2.50 |

If the aminofunctional material contains nitrogen atoms that are not in the form of primary, secondary, and/or tertiary amines, the nitrogen content as a weight percent may be determined according to methods known to those having ordinary skill in the art.

E. Standard

To confirm quality control of the method, a suitable standard may be run - for example, dimethylethanol amine (a tertiary amine; 99.5%; available from Sigma Aldrich). For this particular amine, total amine and tertiary amine content should be 11.2 $\pm$ 0.2meq/g. Primary and Secondary amine content should be <0.1meq/g.

Particle/Droplet Size

The droplet sizes for the siloxane compounds are analyzed as the premix emulsion and in the fabric softener utilizing a Horiba, Partica, Laser Scattering, Particle Size Distribution Analyzer LA-950V2 with a static quartz cell and operated in accordance with the manufacturer's instructions.

HLB Value of Nonionic Surfactants

Nonionic surfactants can be classified by the balance between the hydrophilic and lipophilic moieties in the surfactant molecule. The hydrophile-lipophile balance (HLB) scale devised by Griffin in 1949 is a scale from 0 - 20 (20 being Hydrophilic) used to characterize the nature of surfactants. The HLB of a surfactant may be calculated as follows:

$$HLB = 20 * Mh/M$$

where *Mh* is the molecular of the hydrophilic portion of the molecule, and *M* is the molecular mass of the whole molecule, giving a result on a scale of 0 to 20. An HLB value of 0 corresponds to a completely lipophilic/hydrophobic molecule, and a value of 20 corresponds to a completely hydrophilic/lipophobic molecule. *See* Griffin, W. C. Calculation of HLB values of Nonionic Surfactants, J. Soc. Cosmet. Chem. 1954, 5, 249-256. The HLB values for commonly-used surfactants are readily available in the literature (*e.g.*, HLB Index in McCutcheon's Emulsifiers and Detergents, MC Publishing Co., 2004). The HLB value for a mixture of surfactants can be calculated as a weighted average of the HLB values of the surfactants.

Viscosity Test Method for Silicones

A preliminary estimate of the sample viscosity at 25°C is used to select the appropriate instrument geometry to be used during the final viscosity measurement analyses, which are conducted on a model AR-G2 Rheometer (manufactured by TA Instruments Corp., New Castle, Delaware, USA). A preliminary estimate of the sample viscosity may be obtained by

using a Brookfield Viscometer (Brookfield Engineering Laboratories Inc., Middleboro, Massachusetts, USA). The selection of geometry for use on the AR-G2 Rheometer is determined in accordance with the following table, Table E:

Table E. AR-G2 Geometry Selection.

| Preliminary Estimate of Sample Viscosity | AR-G2 Geometry and Plate Size |
|---|---|
| > 1000 Pa*s | 25 mm parallel plate |
| 1 to 1000 Pa*s | 40 mm parallel plate |
| > Water-thin to < 1 Pa*s | 60 mm parallel plate |
| Water-thin | Couette / Cup and Bob |

The geometry is attached to the instrument, the instrument is mapped, the gap distance is zeroed, and the instrument temperature is set to 25°C. The measurement mode is selected as Stiff Mode when using parallel plates, or to Soft mode when using the couett cup and bob geometry. Sample material is mounted into the sample holding geometry e.g., the base plate. The minimum gap distance allowable between the base plate and the selected geometry is 10x the diameter of the largest common particle present in sample. If there are common particles in the sample which have a diameter greater than 100 μm (as determined microscopically), then the gap value is set to 10x the diameter of the largest common particle, otherwise the gap distance is set to the default value of 1000 μm (i.e. 1 mm). The selected geometry is lowered to the appropriate gap and a plastic tool is used to trim off any excess sample material. The sample material is allowed to equilibrate to the temperature of the instrument. Three rheological measurement analyses are conducted, namely: Flow Curve, Stress Sweep, and Frequency Sweep, using the following selections and settings:

Flow Curve: select Stepped Flow 0.01 to 100; 10 pts/decade; shear stress; constant time 20; average last 10.

Stress Sweep: set the Stress Range as 0.01 to 100 Pa; set the Frequency at 1 rad/s.

Frequency Sweep: Set the Angular Frequency Range as 0.1 to 100.

To ensure that the analysis is conducted within the Linear Viscoelastic Region set the Stress value at a third of the stress value that was present when G' started to degrade during the prior Stress Sweep analysis.

Molecular Weight of Silicone Samples

The molecular weight of the silicone samples may be determined by size exclusion chromatography with multi-angle light scattering detection (SEC-MALS), as provided below.

The following instrumentation is used: Waters Alliance 2695 Separations Module (pump and auto-sampler) SN C055M791M equipped with a Wyatt; rEX refractive index detector SN 874-REX, Waters external column heater, and Wyatt Technology DAWN; Heleos II laser photometer SN 286-H2.

The following chromatography conditions are followed: 0.025 M tetrabutylammonium benzoate (TBAB) in toluene solution at a flow rate of 1 mL/min; Columns - Waters Styragel guard column and two Waters Styragel HR5 linear columns; refractive index detector is at 25 C°; all other components are at ambient room temperature.

To prepare the samples, samples are accurately weighed into polyseal capped vials. The samples are then diluted with 3 mL of eluent (TBAB in toluene) to achieve concentration of 8.0 mg/ml. Samples are swirled by hand and gently mixed and allowed to sit overnight to ensure complete dissolution of the sample. They are then filtered via syringe filter (PALL Acrodisc 13 mm syringe filter with 0.2 mm nylon membrane) into auto-sampler vials. Poly(dimethyl)siloxane (PDMS) reference material is prepared in the same manner.

The data collection and workup is performed with Astra 5.3.4.19 software (Wyatt Technology, Santa Barbara, CA). The dn/dc values of the silicone samples are calculated from peak data using Astra software.

EXAMPLES

The examples provided below are intended to be illustrative in nature and are not intended to be limiting.

Synthesis Examples

The following Synthetic Examples 1-20 show the synthesis of illustrative pro-fragrance silicone polymers and

their silicone precursors, according to the present disclosure. Comparative Synthetic Example A shows a comparative silicone polymer that does not include a heterocyclic moiety as provided in the present disclosure.

For consistency and illustrative/comparative purposes, each example reacts a neat silicone precursor with the same perfume raw material, cinnamic aldehyde or isocyclocitral, which have the following structures:

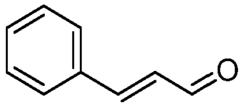

cinnamaldehyde

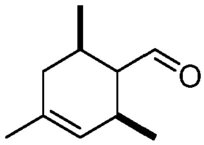

isocyclocitral

However, it is understood that other aldehyde- or ketone-containing PRMs according to the present disclosure may also lead to the formation of suitable pro-fragrance silicone polymers; some of these are exemplified and tested in the Performance Examples below. It is also understood that the Synthetic Examples may be directly formulated into a treatment composition; however, for the case of the reported performance and stability examples below, all Synthetic Examples are formulated into a treatment composition as a liquid premix emulsion as described above.

For each Synthetic Example, the silicone precursor (e.g., having the reported X-Y group) and the resulting pro-fragrance heterocyclic moiety (e.g., having the reported X-Z group) is provided below in Table F. Comparative Synthetic Example A is also shown, although it does not include a -Y moiety according to the present disclosure, nor does the resulting material include a - Z moiety according to the present disclosure.

**Comparative Synthetic Example A.**

Cinnamic aldehyde (0.35 g; available from Symrise, Holzminden, Germany) is added to an amino-modified silicone (A), KF-8003 (5g, available from Shin-Etsu Silicones of America Inc., Akron, OH). The mixture is stirred for 12h. The resulting clear fluid (A') is analyzed by $^1$H NMR.

As shown in Table F below, the resulting material of Comparative Synthetic Example A' does not include a heterocyclic moiety according to the present disclosure.

**Synthetic Example 1.**

Method A: An epoxy functionalized silicone, EMS-622 Fluid (60 g; available from Gelest, Morrisville, PA), is stirred with isopropyl alcohol (3 g, Sigma-Aldrich, St. Louis, MO) in a three-neck round bottom flask. To this fluid is added butylamine (28 g; Sigma-Aldrich, St. Louis, MO). The flask is sealed and placed under a $N_2$ atmosphere for 48h. Excess amine was removed under reduced pressure yielding a clear fluid. The independent silicone fluid **1** appeared stable for several months by $^1$H NMR.

Method B: An epoxy functionalized silicone, EMS-622 Fluid (5 g; available from Gelest, Morrisville, PA), is combined with 2-Methyl-2,4-pentanediol (0.25 g, Sigma-Aldrich, St. Louis, MO) and butylamine (2.3 g; Sigma-Aldrich, St. Louis, MO) and stirred for 24h. Excess amine was removed under reduced pressure yielding **1** as a clear fluid.

Method C: An epoxy functionalized silicone, EMS-622 Fluid (5 g; available from Gelest, Morrisville, PA), is combined with butylamine (2.3 g; Sigma-Aldrich, St. Louis, MO) and stirred for 48h. Excess amine was removed under reduced pressure yielding **1** as a clear fluid.

Regardless of method, to a 5g fraction of the independent fluid **1** is added cinnamic aldehyde (0.5g; available from Symrise, Holzminden, Germany) which is stirred for 12h. The resulting clear fluid **1'** is analyzed by $^1$H NMR.

**Synthetic Example 2.**

An epoxy functionalized silicone, EMS-622 Fluid (60 g; available from Gelest, Morrisville, PA), is combined with isopropyl alcohol (3 g, Sigma-Aldrich, St. Louis, MO) and isopropyl amine (23 g; Sigma-Aldrich, St. Louis, MO) and stirred for 24h. Excess amine was removed under reduced pressure yielding a clear fluid. The independent silicone fluid **2** appeared stable for several months by $^1$H NMR.

To a stirring fluid of **2** (5 g) is added cinnamic aldehyde (0.5 g; available from Symrise, Holzminden, Germany). After 12h the resulting clear fluid **2'** is analyzed by $^1$H NMR.

**Synthetic Example 3.**

An epoxy functionalized silicone, EMS-622 Fluid (60 g; available from Gelest, Morrisville, PA), is combined with isopropyl alcohol (3 g, Sigma-Aldrich, St. Louis, MO) and cyclohexylamine (38 g; Sigma-Aldrich, St. Louis, MO) and stirred for 24h. Excess amine was removed under reduced pressure yielding a light yellow fluid. The independent silicone fluid **3** appeared stable for several months by $^1H$ NMR.

To a stirring fluid of **3** (5 g) is added cinnamic aldehyde (0.5 g; available from Symrise, Holzminden, Germany). After 12h the resulting clear fluid **3'** is analyzed by $^1H$ NMR.

**Synthetic Example 4.**

An epoxy functionalized silicone, EMS-622 Fluid (10 g; available from Gelest, Morrisville, PA), is combined with isopropyl alcohol (0.5 g, Sigma-Aldrich, St. Louis, MO) and tert-butylamine (4.6 g; Sigma-Aldrich, St. Louis, MO) and stirred for 24h. Excess amine was removed under reduced pressure yielding a clear fluid. The independent silicone fluid **4** appeared stable for several months by $^1H$ NMR.

To a stirring fluid of **4** (5 g) is added cinnamic aldehyde (0.5 g; available from Symrise, Holzminden, Germany). After 12h the resulting clear fluid **4'** is analyzed by $^1H$ NMR.

**Synthetic Example 5.**

An epoxy functionalized silicone, EMS-622 Fluid (10 g; available from Gelest, Morrisville, PA), is combined with isopropyl alcohol (0.5 g, Sigma-Aldrich, St. Louis, MO) and methyl amine (2.0 g; Sigma-Aldrich, St. Louis, MO), the flask is sealed and stirred for 24h. Excess amine was removed under reduced pressure yielding **5** as clear fluid.

To a stirring fluid of **5** (5 g) is added cinnamic aldehyde (0.5 g; available from Symrise, Holzminden, Germany). After 12h the resulting in the clear fluid **5'** is analyzed by $^1H$-NMR.

**Synthetic Example 6.**

Method A: An epoxy functionalized silicone, EMS-622 Fluid (45 g; available from Gelest, Morrisville, PA), is combined with $NH_3$ (50 ml, 2M IPA solution, Sigma-Aldrich, St. Louis, MO) in a round bottom flask. The flask is sealed and stirred at room temperature for 72h. Excess amine was removed under reduced pressure yielding a clear fluid. The independent silicone fluid **6** appeared stable for several months by $^1H$ NMR.

Method B: An epoxy functionalized silicone, EMS-622 Fluid (10 g; available from Gelest, Morrisville, PA), is combined with isopropyl alcohol (0.5 g, Sigma-Aldrich, St. Louis, MO) to which $NH_3$ is bubbled through the fluid for 12h (2.0 g; Sigma-Aldrich, St. Louis, MO), the flask is then sealed and stirred for an additional 24h. Excess amine was removed under reduced pressure yielding **6** as clear fluid.

To a stirring fluid of **6** (5 g) is added cinnamic aldehyde (0.55 g; available from Symrise, Holzminden, Germany). After 12h the resulting clear fluid **6'** is analyzed by $^1H$ NMR.

**Synthetic Example 7.**

An epoxy functionalized silicone, X-22-343 (15 g; available from Shin-Etsu Silicones of America Inc., Akron, OH), is combined with 2-Methyl-2,4-pentanediol (0.95 g, Sigma-Aldrich, St. Louis, MO) and octylamine (4.0 g; Sigma-Aldrich, St. Louis, MO) and stirred via overhead stirrer at 800rpm for 16h. The independent silicone fluid 7 appeared stable for several months by $^1H$ NMR.

To a stirring fluid of **7** (5 g) is added cinnamic aldehyde (0.5 g; available from Symrise, Holzminden, Germany). After 12h the resulting clear fluid **7'** is analyzed by $^1H$ NMR.

**Synthetic Examples 8.**

An amino-modified silicone, KF-8003 (500 g; available from Shin-Etsu Silicones of America Inc., Akron, OH), is combined with 2-Methyl-2,4-pentanediol (26g, Sigma-Aldrich, St. Louis, MO) in a three-neck round bottom flask. The solution is stirred until homogenous and placed under a $N_2$ atmosphere. To this solution is added 1,2-Propylene oxide (18.15 g; Sigma-Aldrich, St. Louis, MO) and the mixture is stirred at 80°C for 48h. The independent fluid **8** appears stable for several months by $^1H$ NMR.

To a 5g fraction of the independent fluid **8** is added cinnamic aldehyde (0.32 g; available from Symrise, Holzminden, Germany) which is stirred for 12h. The resulting clear fluid **8'** is analyzed by $^1H$ NMR.

**Synthetic Example 9.**

An amino-modified silicone, KF-8003 (500 g; available from Shin-Etsu Silicones of America Inc., Akron, OH), is combined with 2-Methyl-2,4-pentanediol (27g, Sigma-Aldrich, St. Louis, MO) in a three-neck round bottom flask. The solution is stirred until homogenous and placed under a $N_2$ atmosphere. To this solution is added butyl glycidyl ether (40.7 g; Sigma-Aldrich, St. Louis, MO) and the mixture is stirred at 80°C for 48h. The independent fluid **9** appears stable for several months by $^1$H NMR.

To a 5g fraction of the independent fluid **9** is added 2,4,6-trimethylcyclohex-3-ene-1-carbaldehyde (0.35 g; available from IFF, New York, NY) which is stirred for 12h. The resulting clear fluid **9'** is analyzed by $^1$H NMR.

**Synthetic Example 10.**

An amino-modified silicone, KF-8003 (100 g; available from Shin-Etsu Silicones of America Inc., Akron, OH), is combined with 2-Methyl-2,4-pentanediol (5.5 g, Sigma-Aldrich, St. Louis, MO) in a three-neck round bottom flask. The solution is stirred until homogenous and placed under a $N_2$ atmosphere. To this solution is added cyclohexene sulfide (8.9 g; Sigma-Aldrich, St. Louis, MO) and the mixture is stirred at 80°C for 48h. The independent fluid **10** appears stable for months in a sealed container by $^1$H NMR.

To a 5g fraction of **10** the independent fluid above is added cinnamic aldehyde (0.31 g; available from Symrise, Holzminden, Germany) which is stirred for 12h. The resulting light-yellow fluid **10'** is analyzed by $^1$H NMR.

**Synthetic Example 11.**

An amino-modified silicone, AMS-152 (100 g; available from Shin-Etsu Silicones of America Inc., Akron, OH), is combined with 2-Methyl-2,4-pentanediol (10.5 g, Sigma-Aldrich, St. Louis, MO) in a three-neck round bottom flask. The solution is stirred until homogenous and placed under a $N_2$ atmosphere. To this solution is added ethylene sulfide (4.25 g; Sigma-Aldrich, St. Louis, MO) and the mixture is stirred at 50°C for 48h. The independent fluid **11** appears stable for months in a sealed container by $^1$H NMR.

To a 5g fraction of the independent fluid **11** is added cinnamic aldehyde (0.35 g; available from Symrise, Holzminden, Germany) which is stirred for 12h. The resulting light-yellow fluid **11'** is analyzed by $^1$H NMR.

**Synthetic Example 12.**

An epoxy functionalized silicone, XF-22-343 (50 g; available from Shin-Etsu Silicones of America Inc., Akron, OH), is stirred with 1,2-hexanediol (7 g, Sigma-Aldrich, St. Louis, MO) in a three-neck round bottom flask. To this fluid is added isomeric mixture of a cyclic diamine Baxxodur® (98 g; BASF, Ludwigshafen, Germany). The flask is sealed and placed under a $N_2$ atmosphere for 24h. Excess amine was removed under reduced pressure yielding a light-yellow fluid. The independent silicone fluid **12** darkened upon standing over several months.

To a 5g fraction of freshly prepared independent fluid **12** is added cinnamic aldehyde (2.15 g; available from Symrise, Holzminden, Germany) which is stirred for 12h. The resulting light-yellow fluid **12'** is analyzed by $^1$H NMR.

**Synthetic Example 13.**

An epoxy functionalized silicone, XF-22-343 (50 g; available from Shin-Etsu Silicones of America Inc., Akron, OH), is stirred with 1,2-hexanediol (2.8 g, Sigma-Aldrich, St. Louis, MO) in a three-neck round bottom flask. To this fluid is added isomeric mixture of a cyclic diamine Baxxodur® (6.7 g; BASF, Ludwigshafen, Germany). The flask is sealed and placed under a $N_2$ atmosphere for 24h. The independent fluid **13** appears stable for months in a sealed container by $^1$H NMR.

To a 5g fraction of freshly prepared independent fluid **13** is added cinnamic aldehyde (2.15 g; available from Symrise, Holzminden, Germany) which is stirred for 12h. The resulting fluid **13'** is analyzed by $^1$H NMR.

**Synthetic Example 14.**

An epoxy functionalized silicone, XF-22-343 (100 g; available from Shin-Etsu Silicones of America Inc., Akron, OH), is stirred with 1,2-hexanediol (8.5 g; Sigma-Aldrich, St. Louis, MO) in a three-neck round bottom flask. To this fluid is added ethylene diamine (71 g; Sigma-Aldrich, St. Louis, MO). The flask is placed under a $N_2$ atmosphere and heated to 30°C for 24h. Excess amine was removed under reduced pressure yielding **14** a light-yellow fluid. Cross-linking is facile with ethylene diamine; therefore, excess amine is critical in the preparation of **14.**

To a 5g fraction of the independent fluid **14** is added 2,4,6-trimethylcyclohex-3-ene-1-carbaldehyde (0.35 g;

available from IFF, New York, NY) which is stirred for 12h. The resulting clear fluid **14'** is analyzed by $^1$H NMR. The predominate structural motif is oxazolidine, however, trace amounts of imine were observed.

**Synthetic Example 15.**

An epoxy functionalized silicone, KF-102 (50 g; available from Shin-Etsu Silicones of America Inc., Akron, OH), is combined with isopropyl alcohol (5 g, Sigma-Aldrich, St. Louis, MO) and butylamine (5 g; Sigma-Aldrich, St. Louis, MO) the flask is then placed under a $N_2$ atmosphere. Once homogenous, the flask is heated to 30°C and stirred for 72h. The course of the reaction is monitored by $^1$H NMR. Excess amine was removed under reduced pressure yielding a clear fluid. The independent silicone fluid **15** appeared stable for several months by $^1$H NMR.

To a stirring fluid of **15** (5 g) is added cinnamic aldehyde (0.2 g; available from Symrise, Holzminden, Germany). After 12h the resulting clear fluid **15'** is analyzed by $^1$H NMR.

**Synthetic Example 16.**

An amino-modified silicone, KF-8003 (10 g; available from Shin-Etsu Silicones of America Inc., Akron, OH), is combined with 2-Methyl-2,4-pentanediol (0.5 g, Sigma-Aldrich, St. Louis, MO) in a three-neck round bottom flask. The solution is stirred until homogenous and placed under a $N_2$ atmosphere. To this solution is added methyl glycolate (0.6 g; Sigma-Aldrich, St. Louis, MO) and the mixture is stirred at 120°C for 48h. The resulting fluid **16** appears stable for several months by $^1$H NMR.

To a 5g fraction of the fluid **16** is added cinnamic aldehyde (0.34 g; available from Symrise, Holzminden, Germany) which is stirred at 50°C for 12h. The resulting fluid **16'** is analyzed by $^1$H NMR.

**Synthetic Example 17.**

An acrylate functionalized silicone, UMS-182 fluid (50 g; available from Gelest, Morrisville, PA), is combined with ethanolamine (6.25 g; Sigma-Aldrich, St. Louis, MO) and placed under a $N_2$ atmosphere. The mixture is stirred at room temperature until the reaction is complete, monitored by $^1$H NMR. The independent fluid **17** appears stable for months in a sealed container by $^1$H NMR.

To a 5g fraction of the independent fluid **17** is added cinnamic aldehyde (1.25 g; available from Symrise, Holzminden, Germany) which is stirred for 12h. The resulting clear fluid **17'** was analyzed by $^1$H NMR.

**Synthetic Example 18.**

An amino-modified silicone, DMS-A15 (10 g; available from Gelest, Morrisville, PA), is combined with isopropyl alcohol (5 g, Sigma-Aldrich, St. Louis, MO) and 1,4-Butanediol diglycidyl ether (10 g; Sigma-Aldrich, St. Louis, MO) the flask is then placed under a $N_2$ atmosphere. Once homogenous, the flask is heated to 30°C and stirred for 72h. The course of the reaction is monitored by $^1$H NMR. Excess oxirane was removed under reduced pressure yielding a clear fluid. The independent silicone fluid **18** appeared stable for several months by $^1$H NMR.

To a stirring fluid of **18** (5 g) is added cinnamic aldehyde (0.4 g; available from Symrise, Holzminden, Germany). After 12h the resulting clear fluid **18'** is analyzed by $^1$H NMR.

**Synthetic Example 19.**

A halogenated-modified silicone, LMS-152 fluid (10 g; available from Gelest, Morrisville, PA), is combined with ethanolamine (6.25 g; Sigma-Aldrich, St. Louis, MO) and placed under a $N_2$ atmosphere. The mixture is stirred at 30°C until the reaction is complete monitored by $^1$H NMR. The independent fluid **19** appears stable for months in a sealed container by $^1$H NMR.

To a 5g fraction of the independent fluid **19** is added cinnamic aldehyde (1.25 g; available from Symrise, Holzminden, Germany) which is stirred for 12h. The resulting fluid **19'** was analyzed by $^1$H NMR.

**Synthetic Example 20.**

An epoxy functionalized silicone, EMS-622 fluid (10 g; available from Gelest, Morrisville, PA), is combined with isopropyl alcohol (0.5 g, Sigma-Aldrich, St. Louis, MO), butylamine (2.5 g; Sigma-Aldrich, St. Louis, MO), propylamine (2.2 g; Sigma-Aldrich, St. Louis, MO) and stirred for 48h. Excess amine was removed under reduced pressure yielding a clear fluid. The independent silicone fluid **20** appeared stable for several months by $^1$H NMR.

To a stirring fluid of **20** (5 g) is added cinnamic aldehyde (0.5 g; available from Symrise, Holzminden, Germany).

After 12h the resulting clear fluid **20’** is analyzed by $^1$H NMR.

**Structures of the Synthetic Examples.**

Table F below shows the X-Y and the X-Z structures of the Synthetic Examples described above. The X-Y column shows, for example, the (Si)-X-Y moiety, etc., of the precursor silicone polymer, e.g., the radical according to Formula VI or Formula VII; in the above examples are labeled with a number, e.g., **1.** The X-Z column shows, for example, the (Si)-X-Z moiety, etc., of the pro-fragrance silicone polymer, e.g., the radical according to Formula I or Formula II; in the above Synthesis Examples, these are labeled with a number having a "prime" notation, e.g., **1’.**

Table F also includes Comparative Synthetic Example **A** (and **A’**), which does not include (Si)-X-Y or (Si)-X-Z radicals as described in the present disclosure (e.g., no heterocyclic ring is formed), but they are included in the table for convenience / comparative purposes. This comparative example is marked with an asterisk (*).

**Table F.** Structural representation of the Synthesis Examples

| No. | Illustrative X-Y moiety (e.g., of precursor silicone polymer according to Formula VI or VII) | No.’ | Illustrative X-Z moiety (e.g., of pro-fragrance silicone polymer according to Formula I or II) |
|---|---|---|---|
| A* | 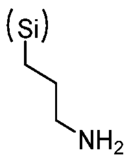 | A’ * | 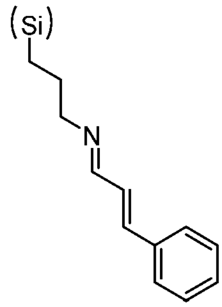 |
| I | 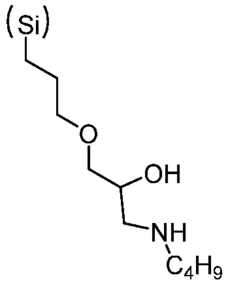 | I’ | 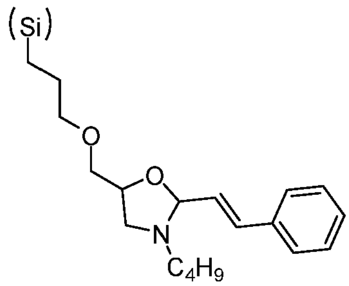 |
| 2 | 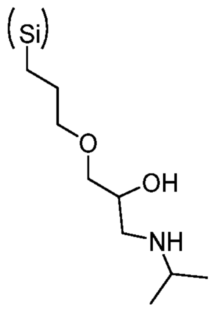 | 2’ | 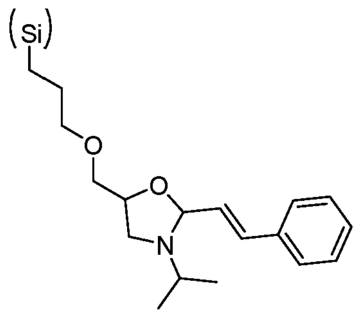 |

(continued)

| No. | Illustrative X-Y moiety (e.g., of precursor silicone polymer according to Formula VI or VII) | No.' | Illustrative X-Z moiety (e.g., of pro-fragrance silicone polymer according to Formula I or II) |
|---|---|---|---|
| 3 | 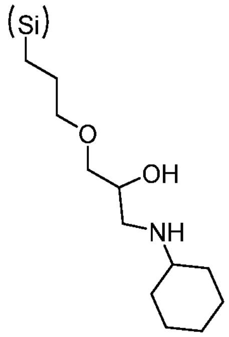 | 3' | 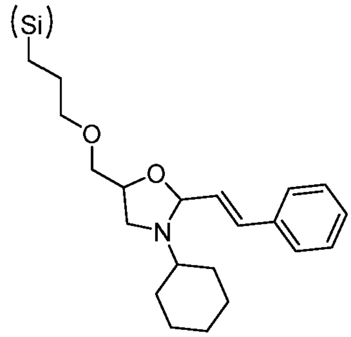 |
| 4 | 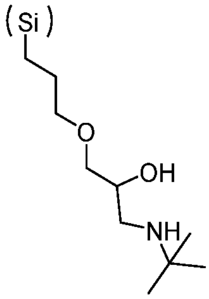 | 4' | 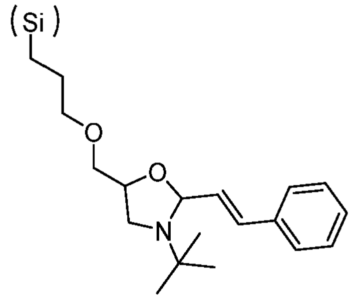 |
| 5 | 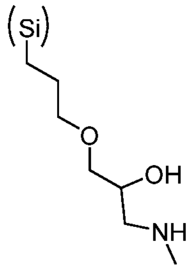 | 5' | 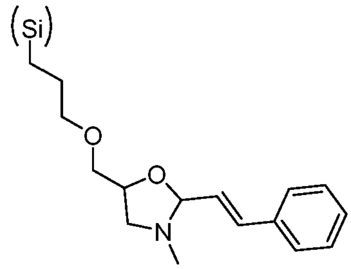 |
| 6 | 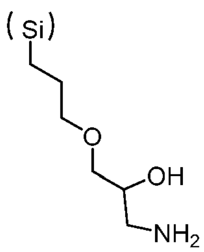 | 6' | 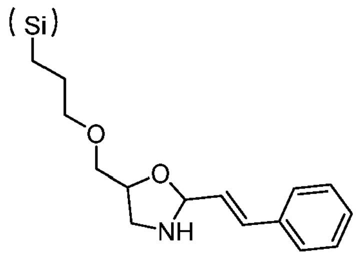 |
| 7 | 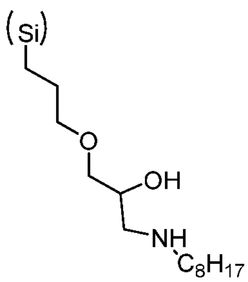 | 7' | 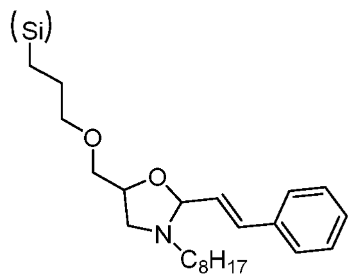 |

(continued)

| No. | Illustrative X-Y moiety (e.g., of precursor silicone polymer according to Formula VI or VII) | No.' | Illustrative X-Z moiety (e.g., of pro-fragrance silicone polymer according to Formula I or II) |
|---|---|---|---|
| 8 | 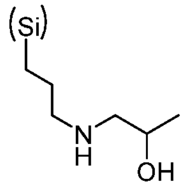 | 8' | 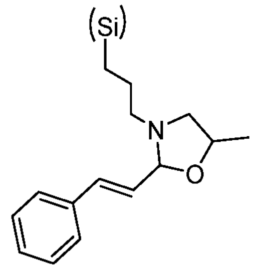 |
| 9 | 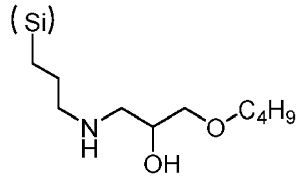 | 9' | 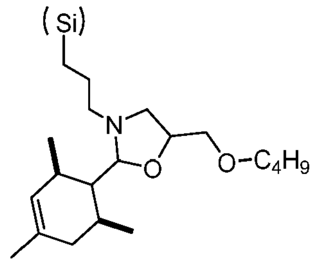 |
| 10 | 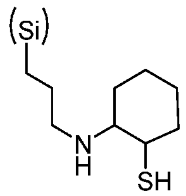 | 10' | 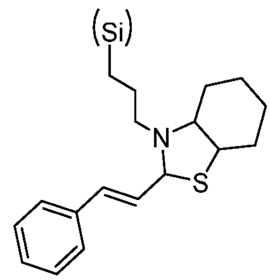 |
| 11 | 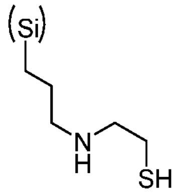 | 11' | 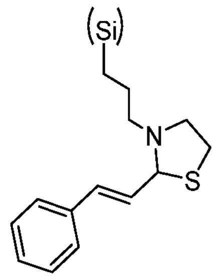 |

(continued)

| No. | Illustrative X-Y moiety (e.g., of precursor silicone polymer according to Formula VI or VII) | No.' | Illustrative X-Z moiety (e.g., of pro-fragrance silicone polymer according to Formula I or II) |
|---|---|---|---|
| 12 | 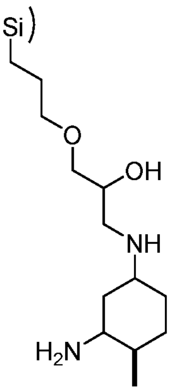 | 12' | 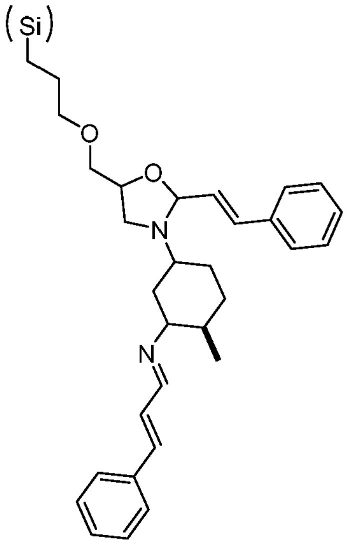 |
| 13 | 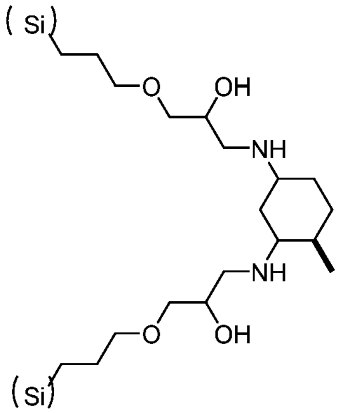 | 13' | 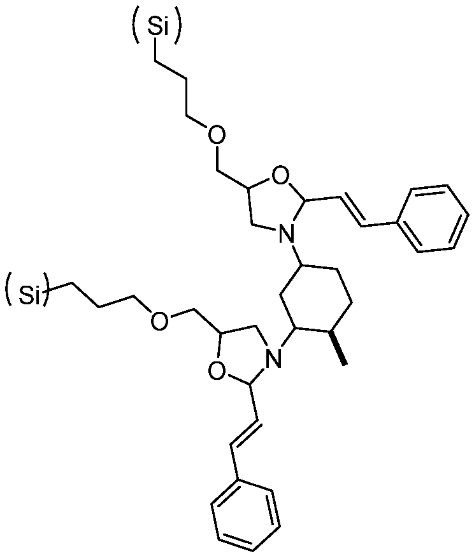 |
| 14 | 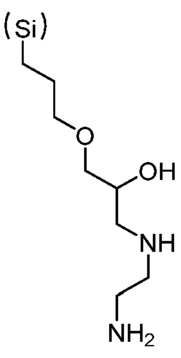 | 14' | 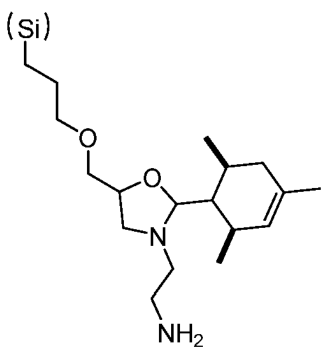 |

(continued)

| No. | Illustrative X-Y moiety (e.g., of precursor silicone polymer according to Formula VI or VII) | No.' | Illustrative X-Z moiety (e.g., of pro-fragrance silicone polymer according to Formula I or II) |
|---|---|---|---|
| **15** | 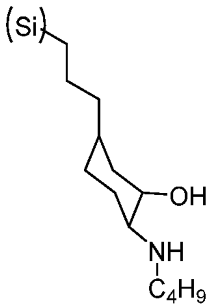 | **15'** | 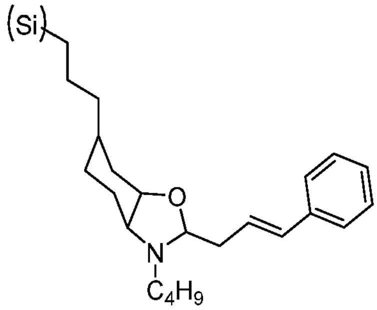 |
| **16** | 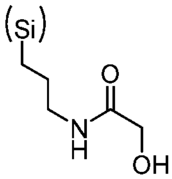 | **16'** | 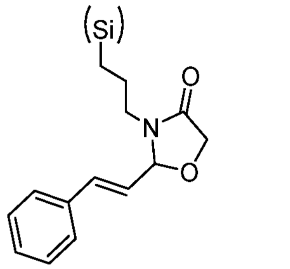 |
| **17** | 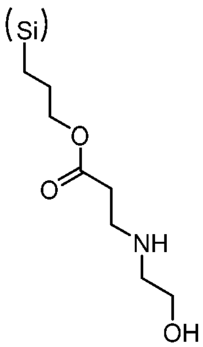 | **17'** | 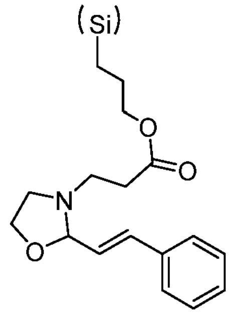 |
| **18** | 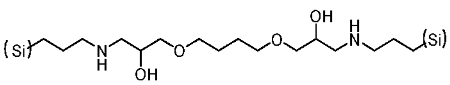 | **18'** | 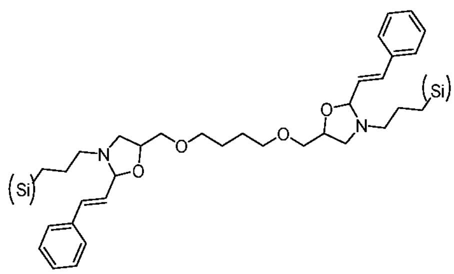 |
| **19** | 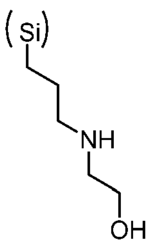 | **19'** | 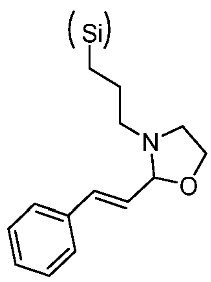 |

(continued)

| No. | Illustrative X-Y moiety (e.g., of precursor silicone polymer according to Formula VI or VII) | No.' | Illustrative X-Z moiety (e.g., of pro-fragrance silicone polymer according to Formula I or II) |
|---|---|---|---|
| **20** | 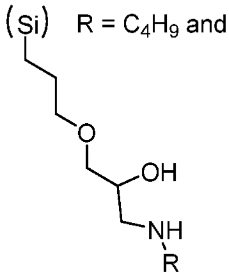 R = $C_4H_9$ and $C_3H_7$ | **20'** | 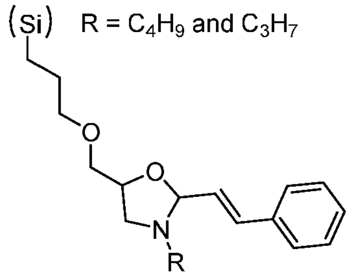 R = $C_4H_9$ and $C_3H_7$ |

In the above Synthesis Examples, precursor silicone polymers (or comparative silicones, such as KF-8003) and the indicated perfume raw materials are combined / reacted to form a fluid comprising the pro-fragrance silicone polymers according to present disclosure.

In the following Performance Examples and Stability Examples, precursor silicone polymers (or comparative silicones, such as KF-8003) and the indicated perfume raw materials are made into an emulsion, substantially following a procedure aligned with the method found in the Test Methods section above ("Preparation of a Pro-Fragrance Silicone Polymer Premix Emulsion"). Despite the different methods of preparation between the Synthesis Examples and the Performance and Stability Examples, the inputs and outputs, in terms of the pro-fragrance silicone polymers, are substantially the same.

Performance Examples

In Performance Examples 1-5 below, treatment compositions comprising neat perfume oil, a liquid premix emulsion comprising the precursor silicone polymers according to the present disclosure (e.g., based on alkanolamine or thiol amine silicone precursors described above) and PRMs as indicated, or premix emulsions comprising comparative silicone polymers are compared via treatment cycles in an automatic washing machine according to the Fabric Treatment methods provided above. After treatment, the fabrics are tested for Headspace Analysis according to the test methods provided above. The data below shows the benefits afforded by heterocyclic moieties, such as oxazolidine and thiazolidine motifs, in delivering benefit agents.

**Performance Example 1.** Benefits of pro-fragrance silicone polymers

Initially, two silicone scaffolds are selected to examine perfume benefit. The first stems from traditional amino-modified silicones, which can then react with oxiranes to yield alkanolamines (Synthetic Example **8**). The second is derived from epoxy silicones that form alkanolamines after nucleophilic attack from an amine (Synthetic Example **1**). To evaluate these materials, a range of perfume raw materials are examined.

In the example below, equal molar concentrations of four aldehydic perfume raw materials are provided to a premix emulsion of the respective test legs, then formulated into a Test Fabric Enhancer/Softener Composition, prepared as provided in the test methods above. Test fabrics are prepared, wash treated, and tested for headspace analysis above the fabrics according to the test methods above.

It is understood that for the rows reading "Synthetic Example 1," etc., both in Table 1 and in subsequent tables, the sample was prepared substantially in accordance with the method and silicone precursor provided in the listed Synthetic Example, but with the perfume raw materials listed in the example (in equal molar concentrations) rather than with just cinnamic aldehyde or isocyclocitral, and formulated as a premix emulsion as detailed above.

Results of the Headspace Analysis Above Fabrics testing is provided below in Table 1.

Table 1.

| Average headspace concentration of aldehyde perfume raw materials[a] above fabrics | | | | | |
|---|---|---|---|---|---|
| Compound | Amount of Isocyclocitral released (nmol/L) | Amount of Cymal released (nmol/L) | Amount of Cinnamic aldehyde released (nmol/L) | Amount of Floralozone released (nmol/L) | Total Headspace Amount (nmol/L) |
| Neat raw materials | 0.01 | 0.30 | 0.04 | 0.15 | 0.51 |
| Synthetic Example **1** | 5.82 | 2.48 | 0.09 | 3.90 | 12.29 |
| Synthetic Example **8** | 3.57 | 3.18 | 0.49 | 2.43 | 9.67 |
| Comp. Synthetic Example A | 2.64 | 0.72 | 0.43 | 3.49 | 7.28 |

[a]All aldehyde raw materials were combined in equal molar concentrations.
[b]The silicone used in Comparative Synthetic Example A (KF-8003) is a product of Shin-Etsu Silicones of America Inc., Akron, OH. The silicone premix emulsion solution of Comp. Synthetic Sample A is prepared using substantially the same method as for the premix emulsions of Synthetic Examples **1** and **8.**

As shown in Table 1, the alkanolamine silicones **1, 8,** and comparative amino-modified Comparative Synthetic Example A, showed an improvement in total headspace over neat raw materials. Furthermore, the alkanolamine silicones **1** and **8** showed an increase in total headspace over the amino-modified Comparative Synthetic Example A. Example fluid **8,** which was synthetically derived from KF-8003, had an affinity for sterically unencumbered neat raw materials versus the parent KF-8003 silicone (see Comparative Synthetic Example A) . Fluid **1** had the highest total headspace with a more balanced reactivity toward non-allylic aldehydes.

**Performance Example 2.** Effect of steric hinderance

This example shows the effect of stearic hinderance at the nitrogen atom of the heterocyclic moiety of the pro-fragrance silicone polymers. The tests are run substantially the same as in Performance Example 1, with the PRMs and Synthetic Examples provided below in Table 2; as with Performance Example 1, it is understood that the pro-fragrance silicone polymers are made with the listed PRMs rather than with just cinnamic aldehyde or isocyclocitral and reacted in the form of a premix emulsion as detailed above.
Results of the Headspace Analysis Above Fabrics are provided in Table 2.

Table 2.

| Average headspace concentration of aldehyde perfume raw materials[a] above fabrics | | | | | |
|---|---|---|---|---|---|
| Compound | Amount of Methyl nonyl acetaldehyde released (nmol/L) | Amount of P.T. Bucinal released (nmol/L) | Amount of Precyclemone B released (nmol/L) | Amount of Floralozone released (nmol/L) | Total Headspace Amount (nmol/L) |
| Neat raw materials | 5.64 | 7.91 | 4.93 | 0.89 | 19.37 |
| Synthetic Example **1** | 42.90 | 28.42 | 29.42 | 12.98 | 113.72 |
| Synthetic Example **2** | 17.25 | 17.00 | 6.13 | 1.83 | 42.21 |
| Synthetic Example **3** | 27.65 | 22.57 | 6.30 | 2.06 | 58.58 |
| Synthetic Example **4** | 9.12 | 10.58 | 5.92 | 1.40 | 27.02 |

[a]*The formulation of the accord is as follows: 10 wt% Methyl nonyl acetaldehdye, 40 wt% P.T.Bucinal, 20 wt% Precyclemone B, and 30 wt% Floralozone.*

As shown in Table 2, each of the example pro-fragrance silicones delivered a higher total headspace over neat raw material. Total headspace concentration for the Synthetic Example silicones is **1** > **3** > **2** > **4.** Without wishing to be bound by theory, it is believed that this trend is rationalized based on sterics, where less steric bulk at the nitrogen leads to improved total headspace - e.g., *n*-butylamine > cyclohexylamine > iso-propylamine > tert-butylamine. The steric influence is highlighted upon examination of Synthetic Examples **2** and **3.** The $\alpha$-carbon to the nitrogen atom in both Synthetic Examples **2** and **3** are secondary in substitution in respect to carbon atoms; however, in Synthetic Example **3** the

α-carbon is in a ring structure resulting in a smaller steric influence. This subtle difference led to a noted difference in the total quantity of delivered perfume raw materials.

**Example 3.** Effect of amine substitution

This example shows the effect of primary vs. secondary substitution at the nitrogen atom of the heterocyclic moiety of the pro-fragrance silicone polymer. The tests are run substantially the same as in Performance Example 1, with the PRMs and Synthetic Examples provided below in Table 3; as with previous examples, it is understood that the pro-fragrance silicone polymers are made with the listed PRMs rather than with just cinnamic aldehyde or isocyclocitral, and reacted in the form of a premix emulsion as detailed above.

Results of the Headspace Analysis Above Fabrics are provided in Table 3.

Table 3.

| Average headspace concentration of aldehyde perfume raw materials[a] above fabrics | | | | | |
|---|---|---|---|---|---|
| Compound | Amount of Methyl nonyl acetaldehyde released (nmol/L) | Amount of P.T. Bucinal released (nmol/L) | Amount of Precyclemone B released (nmol/L) | Amount of Floralozone released (nmol/L) | Total Headspace Amount (nmol/L) |
| Neat raw materials | 1.65 | 3.66 | 0.67 | 0.61 | 6.59 |
| Synthetic Example **1** | 16.22 | 31.49 | 42.22 | 18.27 | 108.20 |
| Synthetic Example **6** | 4.36 | 18.71 | 16.39 | 12.90 | 52.36 |

[a]*The formulation of the accord is as follows: 10 wt% Methyl nonyl acetaldehdye, 40 wt% P.T.Bucinal, 20 wt% Precyclemone B, and 30 wt% Floralozone.*

As shown in Table 3, alkanolamine silicones of Synthetic Examples **1** and **6** had an increased total headspace over neat raw material. Furthermore, Synthetic Example **1,** which contained a secondary amine, resulted in a higher total headspace than the primary amine in Synthetic Example **6.** Thus, alkyl substitution of the amine may be more preferred in specific applications.

**Example 4.** Additional examples of pro-fragrance silicone polymers

This example shows additional example of pro-fragrance silicone polymers. Synthetic Example **11** contains a thiol amine in the precursor and forms a thiazolidine ring when reacted with the perfume raw material. Synthetic Example **12** forms an oxazolidine ring, but because the precursor also includes a cyclic amine in the pendant group that can also react with a perfume raw material, the pendant group may ultimately be loaded with two PRM residues.

The tests are run substantially the same as in Performance Example 1, with the PRMs and Synthetic Examples provided below in Table 4; as with previous examples, it is understood that the pro-fragrance silicone polymers are made with the listed PRMs rather than with just cinnamic aldehyde or isocyclocitral, and reacted in the form of a premix emulsion as detailed above.

Results of the Headspace Analysis Above Fabrics are provided in Table 4.

Table 4.

| Average headspace concentration of aldehyde and ketone perfume raw materials[a] above fabrics | | | | | | |
|---|---|---|---|---|---|---|
| Compound | Amount of Iso cyclo citral released (nmol/L) | Amount of Cymal released (nmol/L) | Amount of Cinnamic aldehyde released (nmol/L) | Amount of Floralozone released (nmol/L) | Amount of δ-Damascone released (nmol/L) | Total Headspace Amount (nmol/L) |
| Neat raw materials | 0.034 | 0.061 | 0.002 | 0.077 | 0.030 | 0.204 |
| Synthetic Example **11** | 3.682 | 0.064 | 0.067 | 4.183 | 3.862 | 11.858 |
| Synthetic Example **12** | 12.429 | 1.419 | 0.313 | 0.938 | 7.073 | 22.172 |

[a]All aldehyde raw materials were combined in equal molar concentrations.

Synthetic Example **11,** which forms a thiazolidine ring in the presence of the raw materials, had an increase in total headspace over neat raw material through the wash (see Table 4). Fluid **11** had a strong interaction with sterically hindered aldehydes and ketones. Oxazolidine-forming silicone **12** reacted strongly with each of the raw materials.

**Example 5.** Use in a particulate laundry additive

In this example, the formulation of material are prepared in a dry-formed particles application (e.g., a pastille comprising polyethylene glycol as a carrier; similar in size and shape to those sold as DOWNY UNSTOPABLES™ by The Procter & Gamble Company). The formulations of the particles for each leg are provided in Table 5A, where Synthetic Example 6 is introduced as a premix emulsion as detailed above. Amounts are provided by % weight of the composition.

Table 5A.

| Leg | PEG-8000 | Silicone premix | Neat PRMs |
|---|---|---|---|
| Neat raw materials[a] | 99.34% | 0.00% | 0.66% |
| Synthetic Example **6**[a] | 89.86% | 10.14% | 0.00% |

[a]The cumulative raw materials equated 0.66 wt% in both legs.

In the example below, equal molar concentrations of the perfume raw materials are provided to a Test Fabric Pastille Composition, prepared as provided in the Test Methods above. Test fabrics are prepared, wash treated, and tested for headspace analysis above the fabrics according to the Test Methods above. The performance test results are provided in Table 5B.

Table 5B.

| Average headspace concentration of aldehyde and ketone perfume raw materials[a] above fabrics | | | | | | |
|---|---|---|---|---|---|---|
| Leg | Amount of Iso cyclo citral released (nmol/L) | Amount of Cymal released (nmol/L) | Amount of Intereleven aldehyde released (nmol/L) | Amount of Ligustral-1 released (nmol/L) | Amount of δ-Damascone released (nmol/L) | Total Headspace (nmol/L) |
| Neat raw material | 0.001 | 0.353 | 0.817 | 0.144 | 0.007 | 1.322 |
| Synthetic Example **6** | 0.057 | 0.443 | 4.289 | 0.284 | 0.217 | 5.290 |

[a]All aldehyde raw materials were combined in equal molar concentrations.

As shown in Table 5B, the Synthetic Example **6** in a dry particles formulation showed an advantage over neat raw materials through the wash.

Stability Example

In Stability Example 1, silicone premix emulsions and treatment compositions are tested for color stability upon storage.

**Stability Example 1.** Color stability in silicone premix

Pro-perfume silicone premixes, specifically premixes in the form of emulsions, and related fabric softener products formed from such premix emulsions are prepared. Color measurements for silicone premix emulsions of Synthetic Examples **1** and **8** are provided and are measured as described in Test Methods above. Aldehyde-containing perfume raw materials (Cymal, Isocyclocitral, Cinnamic aldehyde, and Floralozone) are combined with the silicones in equal molar concentrations. As with previous examples, it is understood that the pro-fragrance silicone polymers are made with the listed PRMs rather than just cinnamic aldehyde or isocyclocitral, and reacted with the PRMs in the form of a premix emulsion as detailed above. Comparative compositions are made with a KF-8003 silicone premix emulsion and the same PRMs.

Color stability of the premix emulsions and the fabric softener products upon storage for two weeks is assessed by the Color Change of a Composition test method provided above. The results are provided in Table 6 below.

Table 6.

| Average ($\Delta E_t{}^a$) absorption change upon storing at 20°C for 2 weeks. | | |
|---|---|---|
| Compound | $\Delta E_t$ [L*, a*, b*] Premix Emulsion (a.u.) | $\Delta E_t$ [L*, a*, b*]] Fabric Softener Formulation |
| Synthetic Example **1** | 11.5 [89.3, -2.9, 10.8] | 15.8 [74.0, -2.4, -5.6] |
| Synthetic Example **8** | 16.3 [76.0, 9.8, 13.3] | 27.2 [64.4, 1.9, 11.0] |
| Comp. Synthetic Example A[b] | 28.6 [71.0, 12.3, 17.4] | 37.9 [54.5, 15.7, -1.9] |

[a] $\Delta E_t$ is calculated as defined in test methods.
[b] Made with KF-8003, product of Shin-Etsu Silicones of America Inc., Akron, OH. The silicone premix emulsion solution of Comp. Synthetic Sample A is prepared using substantially the same method as for the premix emulsions of Synthetic Examples **1** and **8.**

As shown in Table 6, Synthetic Examples **1** and **8,** each of which forms heterocyclic moieties in combination with the PRM residues, result in lower $\Delta E_t$ in both a premix emulsion and treatment composition compared to the solutions formed from the imine-forming silicone KF-8003. Without wishing to be bound by theory, it is believed that π-bonds are likely the origin of color intensity for imines represented in Comparative Synthetic Example A. The influence of π-bond conjugation is further highlighted using cinnamic aldehyde, where the extended conjugation upon imine formation leads to significant coloring. Additionally or alternatively, it is believed the nature of bonding in heterocycles precludes static π-bonds, resulting in Synthetic Examples **1** and **8** having improved color stability with time due to reduced conjugation and diminished gelling.

Exemplary Formulations

The section provides exemplary, non-limiting formulations of premixes and treatment compositions.

**Formulation Example 1.** Exemplary premixes

Table 7 provides exemplary fragrance premix formulations that may be incorporated into consumer products. Amounts are provided as weight percent, by weight of the premix.

Table 7.

| **Ingredient** | **A** | **B** |
|---|---|---|
| Silicone Polymer[1] | 80 | 50 |
| Aminofunctional material[2] | 5 | 0 |
| Perfume raw materials[3] | 15 | 9 |
| Emulsifier | -- | 6 |
| Water | -- | 35 |

[1] Precursor silicone polymer of Synthetic Example **1**
[2] Methyl cyclohexane diamine (Baxxodur EC210, ex BASF)
[3] Contains ethyl vanillin, vanillin, heliotropin, and lilial, each selected so that each PRM is present in an amount to provide equal moles of aldehyde moieties.

**Formulation Example 2.** Particulate treatment composition

A particulate treatment composition may be made according to the following procedure; such compositions may be useful as laundry additive consumer products.

The Pro-Fragrance Silicone Polymer Premix Emulsion made according to the method provided in the Test Method Section, and made according to Synthetic Example **1,** made with equal molar amounts of the following PRMs: Cymal, Cinnamic Aldehyde, Acetophenone, and Benzaldehyde, (113.3 grams) is combined with 1756.6 grams of molten polyethylene glycol (Pluriol E 8000 Prill supplied by BASF Corporation) and 119.8 grams of free fragrance. The blend is mixed and solidified into consumer product particles having an average diameter of about 0.3cm to about 1.5cm, and/or an average mass of from about 1mg to about 1g.

The resulting consumer product is a plurality of particles that are suitable for addition to the wash cycle of an automatic fabric washing machine, optionally in combination with a laundry detergent.

**Formulation Example 3.** Liquid fabric enhancers

Table 8 shows exemplary formulations of compositions according to the present disclosure. Specifically, the following compositions are liquid fabric enhancer products.

Table 8.

| Ingredient | % Active (w/w) | | |
|---|---|---|---|
| | Composition 1 | Composition 2 | Composition 3 |
| Quaternary ammonium ester material | 5% (Ester Quat 1)[1] | 7% (Ester Quat 2)[2] | 8% (Ester Quat 3)[3] |
| Pro-fragrance silicone polymer* | 0.25% | 0.25% | 0.25% |
| Formic Acid | 0.045% | 0.045% | 0% |
| Hydrochloric acid | 0.01% | 0% | 0% |
| Preservative | 0.0045% | 0% | 0% |
| Chelant | 0.0071% | 0.0071% | 0% |
| Structurant | 0.10% | 0.30% | 0.1% |
| Antifoam | 0.008% | 0.00% | 0% |
| Water | Balance | Balance | Balance |

[1] Ester Quat 1: Mixture of bis-(2-hydroxypropyl)-dimethylammonium methylsulfate fatty acid ester, (2-hydroxypropyl)-(1-methyl-2-hydroxyethyl)-dimethylammonium methylsulfate fatty acid ester, and bis-(1-methyl-2-hydroxyethyl)-dimethylammonium methylsulfate fatty acid ester, where the fatty acid esters are produced from a C12-C18 fatty acid mixture (REWOQUAT DIP V 20 M Conc, ex Evonik)
[2] Ester Quat 2: N,N-bis(hydroxyethyl)-N,N-dimethyl ammonium chloride fatty acid ester, produced from C12-C18 fatty acid mixture (REWOQUAT CI-DEEDMAC, ex Evonik)
[3] Ester Quat 3: Esterification product of fatty acids (C16-18 and C18 unsaturated) with triethanolamine, quaternized with dimethyl sulphate (REWOQUAT WE 18, ex Evonik)
* Pro-fragrance silicone polymer premix emulsion according to Synthesis Example **1,** made with equal amounts of the following PRMs: Cymal, Cinnamic Aldehyde, Acetophenone, and Benzaldehyde

**Formulation Example 4.** Liquid laundry detergents

Table 9 shows exemplary formulations of heavy-duty liquid laundry detergent compositions that may be made according to the present disclosure. Amounts provided are by weight % of active, unless otherwise indicated.

Table 9.

| Ingredients | 1 | 2 | 3 | 4 | 5 | 6 | 7 |
|---|---|---|---|---|---|---|---|
| AE18S | 6.77 | 2.16 | 1.36 | 1.30 | | | |
| AE3S | | 3.0 | | | 0.45 | | |
| LAS | 0.86 | 2.06 | 2.72 | 0.68 | 0.95 | | |
| HSAS | 1.85 | 2.63 | 1.02 | | | | |
| AE9 | 6.32 | 9.85 | 10.2 | 7.92 | | | |
| AE8 | | | | | | | 35.45 |
| AE7 | | | | | 8.40 | 12.44 | |
| C1214 dimethyl Amine | 0.3 | 0.73 | 0.23 | 0.37 | | | |
| C1218 Fatty Acid | 0.80 | 1.90 | 0.60 | 0.99 | 1.20 | | 15.00 |
| Citric Acid | 2.50 | 3.96 | 1.88 | 1.98 | 0.9 | 2.5 | 0.6 |

(continued)

| Ingredients | 1 | 2 | 3 | 4 | 5 | 6 | 7 |
|---|---|---|---|---|---|---|---|
| Optical Brightener 1 | 1.0 | 0.8 | 0.1 | 0.3 | 0.05 | 0.5 | 0.001 |
| Optical Brightener 3 | 0.001 | 0.05 | 0.01 | 0.2 | 0.5 | | |
| Sodium formate | 1.6 | 0.09 | 1.2 | 0.04 | 1.6 | 1.2 | 0.2 |
| DTI 1 | 0.32 | 0.05 | | 0.6 | 0.1 | 0.6 | 0.01 |
| DTI2 | 0.32 | 0.1 | 0.6 | 0.6 | 0.05 | 0.4 | 0.2 |
| Sodium hydroxide | 2.3 | 3.8 | 1.7 | 1.9 | 1.7 | 2.5 | 2.3 |
| Monoethanolamine | 1.4 | 1.49 | 1.00 | 0.7 | | | |
| Diethylene glycol | 5.5 | | 4.1 | | | | |
| Chelant 1 | 0.15 | 0.15 | 0.11 | 0.07 | 0.5 | 0.11 | 0.8 |
| 4-formyl-phenylboronic acid | | 0.05 | 0.02 | 0.01 | | | |
| Sodium tetraborate | 1.43 | 1.50 | 1.10 | 0.75 | | 1.07 | |
| Ethanol | 1.54 | 1.77 | 1.15 | 0.89 | | 3.00 | 7.00 |
| Polymer 1 | 0.1 | | | | | | 2.00 |
| Polymer 2 | 0.3 | 0.33 | 0.23 | 0.17 | | | |
| Polymer 3 | | | | | | | 0.8 |
| Polymer 4 | 0.8 | 0.81 | 0.60 | 0.40 | 1.0 | 1.0 | |
| 1,2-Propanediol | | 6.6 | | 3.3 | 0.5 | 2.0 | 8.0 |
| Structurant | 0.1 | | | | | | 0.1 |
| Perfume (neat oil) | 1.6 | 1.1 | 1.0 | 0.8 | 0.9 | 1.5 | 1.6 |
| Pro-fragrance silicone polymer | 0.1 | 0.1 | 0.2 | 0.1 | 0.1 | 0.1 | 0.3 |
| Protease | 0.8 | 0.6 | 0.7 | 0.9 | 0.7 | 0.6 | 1.5 |
| Mamanase | 0.7 | 0.05 | 0.045 | 0.06 | 0.04 | 0.045 | 0.1 |
| Amylase 1 | | | 0.3 | 0.1 | | | |
| Amylase 2 | | 0.1 | 0.1 | | 0.07 | | |
| Amylase4 | 0.3 | 0.1 | | 0.15 | 0.03 | 0.4 | 0.1 |
| Isoamylase | 0.3 | 0.2 | 0.1 | 0.07 | 0.2 | 0.02 | 0.3 |
| Xyloglucanase | 0.2 | 0.1 | | | 0.05 | 0.05 | 0.2 |
| Lipase | 0.4 | 0.2 | 0.3 | 0.1 | 0.2 | | |
| Polishing enzyme | | 0.04 | | 0.004 | | | |
| Dispersin B | | | | 0.05 | 0.03 | 0.001 | 0.001 |
| Acid Violet 50 | 0.05 | | | | | | 0.05 |
| Direct Violet 9 | | | | | | 0.05 | |
| Violet DD | | 0.035 | 0.02 | 0.037 | 0.04 | | |
| Water insoluble plant fiber | 0.2 | | | | 1.2 | | |
| Dye control agent | | 0.3 | | 0.5 | 0.3 | | |
| Alkoxylated polyaryl polyalkyl phenol | | | 1.2 | | | | 3.1 |
| Water, dyes & minors | Balance | | | | | | |

(continued)

| Ingredients | 1 | 2 | 3 | 4 | 5 | 6 | 7 |
|---|---|---|---|---|---|---|---|
| pH | 8.2 | | | | | | |

• AE1.85 is C1215 alkyl ethoxy (1.8) sulfate
• AE3S is C1215 alkyl ethoxy (3) sulfate
• AE7 is C1213 alcohol ethoxylate, with an average degree of ethoxylation of 7
• AE8 is C1213 alcohol ethoxylate, with an average degree of ethoxylation of 8
• AE9 is C1213 alcohol ethoxylate, with an average degree of ethoxylation of 9
• Alkoxylated polyaryl is, for example, EMULSOGEN® T5160, HOSTAPAL®BV conc., SAPOGENAT® T11O, and/or SAPOGENAT® T139, all from Clariant
• Amylase 1 is STAINZYME®, 15 mg active/g
• Amylase 2 is NATALASE®, 29 mg active/g
• Amylase 3 is STAINZYME PLUS®, 20 mg active/g
• Isoamylase has glycogen-debranching activity
• AS is C1214 alkylsulfate
• Cellulase 2 is CELLUCLEAN®, 15.6 mg active/g
• Xyloglucanase is WHITEZYME®, 20 mg active/g
• Chelant 1 is diethylene triamine pentaacetic acid (DTPA)
• Chelant 2 is 1-hydroxyethane 1,1-diphosphonic acid (HEDP)
• Chelant 3 is sodium salt of ethylenediamine-N,N'-disuccinic acid, (S,S) isomer (EDDS)
• Dispersin B is a glycoside hydrolase, reported as 1000 mg active/g
• DTI 1 is poly(4-vinylpyridine-1-oxide), such as CHROMABOND S-403E®),
• DTI 2 is poly(1-vinylpyrrolidone-co-l-vinylimidazole) (such as SOKALAN HP56 ©).
• Dye control agent is, for example, SUPAREX® O.IN (MI), NYLOFIXAN® P (M2), NYLOFIXAN® PM (M3), or NYLOFIXAN® HF (M4)
• HSAS is mid-branched alkyl sulfate as disclosed in U.S. Pat. No. 6,020,303 and U.S. Pat. No. 6,060,443
• LAS is linear alkylbenzenesulfonate having an average aliphatic carbon chain length C9-C15 (HLAS is acid form)
• Lipase is LIPEX®, 18mg active/g
• Mannanase is MANNAWAY®, 25 mg active/g
• Optical Brightener 1 is disodium 4,4'-bis{[4-anilino-6-morpholino-s-triazin-2-yl]-amino}-2,2-stilbenedisulfonate
• Optical Brightener 2 is disodium 4,4'-bis-(2-sulfostyryl)biphenyl (sodium salt)
• Optical Brightener 3 is OPTIBLANC SPL1O® from 3 V Sigma
• Photobleach is a sulfonated zinc phthalocyanine
• Polishing enzyme is Para-nitrobenzyl esterase, reported as 1000 mg active/g
• Polymer 1 is bis((C2H5O)(C2H4O)n)(CH3) -N -CH----N'----(CH3)-bis((C2H50)(C2H40)n), wherein n = 20-30, x = 3 to 8, or sulphated or sulfonated variants thereof
• Polymer 2 is ethoxylated (EO15) tetraethylene pentamine
• Polymer 3 is ethoxylated polyethylenimine (PEI600 EO20)
• Polymer 4 is ethoxylated hexamethylene diamine
• Polymer 5 is ACUSOL® 305, provided by Rohm&Haas
• Polymer 6 is a polyethylene glycol polymer grafted with vinyl acetate side chains, provided by BASF
• Pro-fragrance silicone polymer premix emulsion is Synthetic Example 6, made with equal amounts of the following PRMs: Cymal, Cinnamic Aldehyde, Acetophenone, and Benzaldehyde
• Protease is PURAFECT PRIME®, 40.6 mg active/g
• Protease 2 is SAVINASE®, 32.89 mg active/g
• Protease 3 is PURAFECT®, 84 mg active/g
• Quaternary ammonium is C1214 Dimethylhydroxyethyl ammonium chloride
• S-ACMC is Reactive Blue 19 Azo-CM-Cellulose, provided by Megazyme
• Soil release agent is REPEL-O-TEX® SF2
• Structurant is Hydrogenated Castor Oil
• Violet DD is a thiophene azo dye provided by Milliken
• Water insoluble plant material is, for example, Herbacel AQ+ Type N, supplied by Herbafood Ingredients GmbH, Werder, Germany

**Formulation Example 5.** Unit dose articles

Table 10 shows formulations of various unit dose detergent articles in the form of pouches. Multi-compartment pouches can contain a plurality of benefit agents. By way of a non-limiting example, a two- or three-component pouch may contain the formulations presented in Table 10 in separate enclosures, where dosage is the amount of the formulation in the respective enclosure. The pouch may be formed from a water-soluble film, such as polyvinyl alcohol films available from MonoSol, LLC (Indiana, USA).

Table 10.

| | A | | | B | | C | | |
|---|---|---|---|---|---|---|---|---|
| | 3 compartments | | | 2 compartments | | 3 compartments | | |
| Compartment # | 1 | 2 | 3 | 1 | 2 | 1 | 2 | 3 |
| Dosage (g) | 34.0 | 3.5 | 3.5 | 30.0 | 5.0 | 25.0 | 1.5 | 4.0 |
| Ingredients | Weight % | | | | | | | |
| Alkylbenzene sulfonic acid | 20.0 | 20.0 | 20.0 | 10.0 | 20.0 | 20.0 | | |
| Alkyl sulfate | | | | 2.0 | | | | |
| C12-14 alkyl 7-ethoxylate | 17.0 | 17.0 | 17.0 | | 17.0 | 17.0 | | |
| Cationic surfactant | | | | 1.0 | | | | |
| Zeolite A | | | | 10.0 | | | | |
| C12-18 Fatty acid | 13.0 | 13.0 | 13.0 | | 18.0 | 18.0 | | |
| Sodium acetate | | | | 4.0 | | | | |
| enzymes | 0-3 | 0-3 | 0-3 | 0-3 | | 0-3 | | |
| Sodium Percarbonate | | | | 11.0 | | | | |
| TAED | | | | 4.0 | | | | |
| Organic catalyst [1] | | | | 1.0 | | | | |
| PAP granule [2] | | | | | | | | 50 |
| Polycarboxylate | | | | 1.0 | | | | |
| Polyethyleneimine ethoxylate [3] | 2.2 | 2.2 | 2.2 | | | | | |
| Hydroxyethane diphosphonic acid | 0.6 | 0.6 | 0.6 | 0.5 | | | | |
| Ethylene diamine tetra(methylene phosphonic) acid | | | | | | 0.4 | | |
| Brightener | 0.2 | 0.2 | 0.2 | 0.3 | | 0.3 | | |
| Mineral oil | | | | | | | | |
| Hueing dye [4] | | | 0.05 | | 0.035 | | 0.12 | |
| Perfume (neat) | 1.7 | 1.7 | | 0.6 | | 1.5 | | |
| Fragrance Premix* | 3 | | | 2.5 | | 3 | | |
| Water and minors (antioxidant, aesthetics, etc.) | 10.0 | 10.0 | 10.0 | 4.0 | | | | |
| Buffers (sodium carbonate, monoethanolamine) [5] | To pH 8.0 for liquids<br>To RA > 5.0 for powders | | | | | | | |

(continued)

| | A | B | C |
|---|---|---|---|
| | 3 compartments | 2 compartments | 3 compartments |
| Solvents (1,2 propanediol, ethanol) for liquids, sodium sulfate for powders | To 100% | | |

[1] Sulfuric acid mono-[2-(3,4-dihydro-isoquinolin-2-yl)-1-(2-ethyl-hexyloxymethyl)-ethyl]ester as described in US7169744
[2] PAP = Phtaloyl-Amino-Peroxycaproic acid, as a 70% active wet cake
[3] Polyethylenimine (MW = 600) with 20 ethoxylate groups per -NH.
[4] Ethoxylated thiophene, EO ($R_1$+$R_2$) = 5
[5] RA = Reserve Alkalinity (g NaOH/dose)
* A silicone/PRM premix emulsion according to the present disclosure. Wt% provided in table is the amount of fragrance premix provided, not the amount of perfume delivered by the premix; premix is substantially free of water. The premix is made with about 20% of perfume raw materials, by weight of the premix.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

**1.** A treatment composition comprising a treatment adjunct and a pro-fragrance silicone polymer,
wherein the pro-fragrance silicone polymer comprises a silicone backbone, an organic linker group comprising a carbon atom bonded to a silicon atom of the silicone backbone, and a heterocyclic moiety bonded to the organic linker group,
the heterocyclic moiety comprising five ring members, the ring members comprising:

a first ring member that is a nitrogen atom;
a second ring member that is a carbon atom,

wherein the second ring member is part of a residue of a perfume raw material ("PRM"),
wherein the PRM that formed the residue comprises a moiety selected from the group consisting of an aldehyde moiety, a ketone moiety, and a combination thereof;

a third ring member selected from the group consisting of an oxygen atom or a sulfur atom, preferably an oxygen atom,
wherein the second ring member is directly bonded to the first ring member and to the third ring member;
and wherein the pro-fragrance silicone polymer comprises at least one radical selected from the group consisting of Formula I, Formula II, and mixtures thereof,

wherein the radicals of Formula I and Formula II have the following structures:

$$(Si) - X - Z \qquad \text{Formula I}$$

$$(Si) - X - (Z - X)_n - (Si) \qquad \text{Formula II}$$

wherein "(Si) -" represents the bond to an Si atom,
wherein each X group is the organic linker group and is an independently selected divalent organic moiety group comprising from two to twelve chain atoms, wherein the X group is bonded to a non-terminal silicon atom;
wherein the Z group comprises the heterocyclic moiety, and

wherein the index n is 1 or 2, preferably 1;
preferably wherein the at least one radical has the structure of Formula I;

wherein each Z is a monovalent or divalent five-membered heterocyclic moiety according to Formula IV, wherein Formula IV has the following structure:

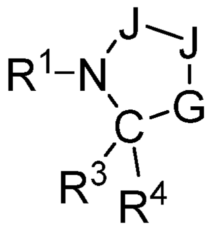

Formula IV,

wherein G is selected from the group consisting of oxygen or sulfur,
$R^1$ is selected from H, a monovalent moiety with a molecular weight of from 15 to 495 Da, or a point of attachment of Z to an X group, preferably $R^1$ is H or a point of attachment of Z to an X group,
and the -C($R^3$)($R^4$)- moiety is the residue of the PRM, preferably a PRM that comprises from 3 to 34 carbon atoms,

preferably wherein G is oxygen,
and wherein each J is independently $C(R^2)_2$, wherein each $R^2$ is independently selected from H, a monovalent moiety with a molecular weight of from 14 to 990 Da, or a point of attachment of Z to an X group, preferably $R^2$ is H or a point of attachment of Z to an X group;

with the proviso that a first unit and a second unit can optionally be taken together, where feasible, as a divalent substituent, where the first unit is a first $R^2$ group, and where the second unit is selected from the group consisting of a second $R^2$ group, the $R^1$ group, and a monovalent substituent of the $R^1$ group, provided that at least one and no more than two of $R^1$, any $R^2$, or mixtures thereof are a point of attachment of Z to an X group.

**2.** The treatment composition according to claim 1, wherein the PRM that formed the residue comprises an aldehyde moiety,
preferably wherein the PRM is selected from the group consisting of: methyl nonyl acetaldehyde: benzaldehyde; floralozone; isocyclocitral; triplal (ligustral); precylcemone B; lilial; decyl aldehyde; undecylenic aldehyde; cyclamen homoaldehyde; cyclamen aldehyde; dupical; oncidal; adoxal; melonal; calypsone; anisic aldehyde; heliotropin; cuminic aldehyde; scentenal; 3,6-dimethylcyclohex-3-ene-1-carbaldehyde; satinaldehyde; canthoxal; vanillin; ethyl vanillin; cinnamic aldehyde; and mixtures thereof.

**3.** The treatment composition according to claim 1 or claim 2, wherein the PRM that formed the residue comprises a ketone moiety,
preferably wherein the PRM is selected from the group consisting of: nerolione; 4-(4-methoxyphenyl)butan-2-one; 1-naphthalen-2-ylethanone; nectaryl; trimofix O; fleuramone; delta-damascone; beta-damascone; alpha-damascone; methyl ionone; 2-hexylcyclopent-2-en-1-one; galbascone; and mixtures thereof.

**4.** The treatment composition according to any preceding claim, wherein each X group independently comprises from two to nine chain atoms, more preferably from two to six chain atoms, even more preferably wherein at least one of the chain atoms is an oxygen atom, even more preferably wherein the X group is a four-carbon ether group, most preferably wherein the X group is $-CH_2-CH_2-CH_2-O-CH_2-$.

**5.** The treatment composition according to any preceding claim, wherein when $R^1$ is present as the monovalent moiety, the monovalent moiety is a substituted or unsubstituted $C_1$-$C_{35}$ alkyl group, preferably a monovalent moiety with a molecular weight of from 15 to 101 Da, more preferably a substituted or unsubstituted $C_1$-$C_8$ alkyl.

**6.** The treatment composition according to any preceding claim, wherein when $R^2$ is present as the monovalent moiety, the monovalent moiety is preferably a substituted or unsubstituted, saturated or unsaturated $C_1$-$C_{70}$ alkyl, more preferably a monovalent moiety with a molecular weight of from 14 to 186 Da, even more preferably a substituted or unsubstituted, saturated or unsaturated $C_1$ to $C_{12}$ alkyl.

**7.** The treatment composition according to any preceding claim, wherein the divalent substituent is selected from the

group consisting of a fused ring, a spirocyclic ring, an unsaturated substituent, =N($R^1$), =O, and =S.

**8.** The treatment composition according to any preceding claim, wherein $R^3$ is independently selected from a monovalent organic moiety, and preferably wherein $R^4$ is independently selected from the group consisting of hydrogen and a monovalent organic moiety.

**9.** The treatment composition according to any preceding claim, wherein at least one $R^2$ is a point of attachment of Z to an X group,
preferably wherein $R^1$ is H.

**10.** A liquid premix composition comprising:

a) a pro-fragrance silicone polymer as described in any of claims 1 to 9,
optionally, further comprising one or more free perfume raw materials; or
b) a precursor silicone polymer, and a perfume raw material that comprises a moiety selected from the group consisting of an aldehyde moiety, a ketone moiety, and a combination thereof,
wherein the precursor silicone polymer and the perfume raw material are capable of condensing to form the pro-fragrance silicone polymer as described in any of claims 1 to 9; or
c) a mixture thereof.

**11.** The liquid premix composition according to claim 10, wherein the liquid premix composition is in the form of an oil-in-water emulsion,
preferably comprising droplets **characterized by** a volume-weighted average particle size of from 100 nm to 100 μm, more preferably from 1 μm to 10 μm,
even more preferably from 1 μm to 5 μm.

**12.** The liquid premix composition according to claim 10 or claim 11, wherein, by weight of the liquid premix composition, the precursor silicone polymer is present at a level of from 1% to 99%, preferably from 80% to 10%, or from 75% to 20%, or from 60% to 40%, and
the perfume raw material is present at a level of from 1% to 50%, preferably from 5% to 50%, or from 5% to 40%, or from 5% to 30%, or from 5% to 20%, or from 5% to 15%, or from 5% to 10%,
preferably wherein the weight ratio of the precursor silicone polymer and the perfume raw material is from 99:1 to 1:1, preferably from 50:1 to 2:1, more preferably from 10:1 to 5:1.

**13.** The liquid premix composition according to any of claims 10 to 12, wherein the precursor silicone polymer comprises at least one radical selected from the group consisting of Formula VI, Formula VII, and mixtures thereof,

wherein the radicals of Formula VI and Formula VII have the following structures:

(Si) - X - Y     Formula VI;

(Si) - X - $(Y - X)_n$ - (Si)     Formula VII;

wherein "(Si) -" is the bond to an Si atom,
wherein the index n is 1 or 2, preferably 1,
wherein each X group is covalently linked to "(Si)" via a Si-C bond and is an independently selected divalent organic group comprising from two to twelve chain atoms, and
wherein each Y is independently a monovalent or divalent moiety according to Formula VIII,

wherein Formula VIII has the following structure:

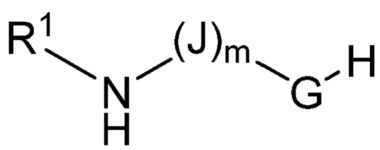

Formula VIII,

wherein G is selected from the group consisting of oxygen or sulfur, preferably oxygen,

wherein the index m is 2,
wherein $R^1$ is selected from H, a monovalent moiety with a molecular weight between 15 and 495 Da, or a point of attachment of Y to an X group,
wherein when $R^1$ is present as the monovalent moiety, the monovalent moiety is preferably a substituted or unsubstituted $C_1$-$C_{35}$ alkyl group, more preferably a monovalent moiety with a molecular weight of from 15 to 101 Da, even more preferably a substituted or unsubstituted $C_1$-$C_8$ alkyl,
preferably wherein $R^1$ is H,
wherein each J is independently $C(R^2)_2$

wherein each $R^2$ is independently selected from the group consisting of H, a monovalent moiety with a molecular weight between 14 and 990 Da, or a point of attachment of Y to an X group,
wherein when $R^2$ is present as the monovalent moiety, the monovalent moiety is preferably a substituted or unsubstituted, saturated or unsaturated $C_1$-$C_{70}$ alkyl, more preferably a monovalent moiety with a molecular weight of from 14 to 186 Da, even more preferably a substituted or unsubstituted, saturated or unsaturated $C_1$ to $C_{12}$ alkyl,
preferably wherein each $R^2$ is H,
with the proviso that a first unit and a second unit can optionally be taken together, where feasible, as a divalent substituent,

where the first unit is a first $R^2$ group, and
where the second unit is selected from the group consisting of a second $R^2$ group, the $R^1$ group, and a monovalent substituent of the $R^1$ group,
preferably where the divalent substituent is selected from the group consisting of a fused ring, a spirocyclic ring, an unsaturated substituent, $=N(R^2)$, =O, and =S,

provided that at least one and no more than two of $R^1$, any $R^2$, or combinations thereof is a point of attachment of Y to an X group.

**14.** A method of making a treatment composition, the method comprising the steps of:

providing a base composition, wherein the base composition comprises a treatment adjunct;
combining the base composition with one or more of the following:

a) a pro-fragrance silicone polymer as described in any of claims 1 to 9;
b) a precursor silicone polymer as described in any of claims 10 to 13, and a perfume raw material that comprises a moiety selected from the group consisting of an aldehyde moiety, a ketone moiety, and a combination thereof,
preferably wherein the precursor silicone polymer and the perfume raw material are provided together as a liquid premix composition, preferably a liquid premix composition in the form of an emulsion; or
c) a mixture thereof.

**15.** Use of a pro-fragrance silicone polymer as described in any of claims 1 to 9 as a perfuming agent, optionally on a surface, preferably on a fabric, optionally being formulated in a treatment composition, where the treatment composition may be a treatment composition according to any of claims 1 to 9.

**Patentansprüche**

**1.** Behandlungszusammensetzung, umfassend ein Behandlungshilfsmittel und ein Pro-Duftstoff-Silikonpolymer,

wobei das Pro-Duft-Silikonpolymer ein Silikongrundgerüst, eine organische Linkergruppe, umfassend ein Kohlenstoffatom, das an ein Siliziumatom des Silikongrundgerüsts gebunden ist, und eine heterocyclische Einheit, die an die organische Linkergruppe gebunden ist, umfasst,
die heterocyclische Einheit, umfassend fünf Ringglieder, die Ringglieder umfassend:

ein erstes Ringglied, das ein Stickstoffatom ist;
ein zweites Ringglied, das ein Kohlenstoffatom ist,
wobei das zweite Ringglied Teil eines Rückstands eines Duftstoffrohmaterials ("PRM") ist,

wobei das PRM, das den Rückstand bildete, eine Einheit, ausgewählt aus der Gruppe bestehend aus einer Aldehydeinheit, einer Ketoneinheit und einer Kombination davon, umfasst;
ein drittes Ringglied, ausgewählt aus der Gruppe bestehend aus einem Sauerstoffatom oder einem Schwefelatom, vorzugsweise einem Sauerstoffatom,
wobei das zweite Ringglied an das erste Ringglied und an das dritte Ringglied direkt gebunden ist;
und wobei das Pro-Duftstoff-Silikonpolymer wenigstens ein Radikal, ausgewählt aus der Gruppe bestehend aus Formel I, Formel II und Mischungen davon, umfasst,
wobei die Radikale von Formel I und Formel II die folgenden Strukturen aufweisen:

(Si) - X - Z    Formel I

$(Si) - X - (Z - X)_n - (Si)$    Formel II

wobei "(Si) -" die Bindung an ein Si-Atom darstellt,
wobei jede X-Gruppe die organische Linkergruppe ist und eine unabhängig voneinander ausgewählte zweiwertige organische Einheitsgruppe ist, umfassend zwei bis zwölf Kettenatome, wobei die X-Gruppe an ein nicht endständiges Siliziumatom gebunden ist;
wobei die Z-Gruppe die heterocyclische Einheit umfasst, und
wobei der Index n 1 oder 2, vorzugsweise 1, ist;
vorzugsweise wobei das wenigstens eine Radikal die Struktur von Formel I aufweist;
wobei jedes Z eine einwertige oder zweiwertige fünfgliedrige heterocyclische Einheit gemäß Formel IV ist,
wobei Formel IV die folgende Struktur aufweist:

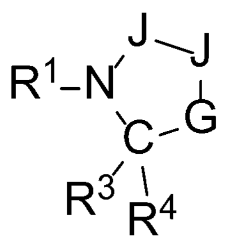

Formel IV,

wobei G ausgewählt ist aus der Gruppe bestehend aus Sauerstoff oder Schwefel,
$R^1$ ausgewählt ist aus H, einer einwertigen Einheit mit einem Molekulargewicht von 15 bis 495 Da, oder einem Bindungspunkt von Z an eine X-Gruppe, vorzugsweise $R^1$ H oder ein Bindungspunkt von Z an eine X-Gruppe ist,
und die $-C(R^3)(R^4)$-Einheit der Rückstand des PRM, ist, vorzugsweise eines PRM, das 3 bis 34 Kohlenstoffatome umfasst,
vorzugsweise wobei G Sauerstoff ist,
und wobei jedes J unabhängig voneinander $C(R^2)_2$ ist, wobei jedes $R^2$ unabhängig voneinander ausgewählt ist aus H, einer einwertigen Einheit mit einem Molekulargewicht von 14 bis 990 Da, oder einem Bindungspunkt von Z an eine X-Gruppe, vorzugsweise $R^2$ H oder ein Bindungspunkt von Z an eine X-Gruppe ist;
mit der Maßgabe, dass eine erste Einheit und eine zweite Einheit wahlweise zusammengenommen werden können, wo durchführbar, als ein zweiwertiger Substituent, wobei die erste Einheit eine erste $R^2$-Gruppe ist, und wobei die zweite Einheit ausgewählt ist aus der Gruppe bestehend aus einer zweiten $R^2$-Gruppe, der $R^1$-Gruppe und einem einwertigen Substituenten der $R^1$-Gruppe,
mit der Maßgabe, dass wenigstens eines und nicht mehr als zwei von $R^1$, ein beliebiges $R^2$ oder Mischungen davon ein Bindungspunkt von Z an eine X-Gruppe sind.

**2.** Behandlungszusammensetzung nach Anspruch 1, wobei das PRM, das den Rückstand bildete, eine Aldehydeinheit umfasst,
vorzugsweise wobei das PRM ausgewählt ist aus der Gruppe bestehend aus: Methylnonylacetaldehyd: Benzaldehyd; Floralozon; Isocyclocitral; Triplal (Ligustral); Precylcemon B; Lilial; Decylaldehyd; Undecylenaldehyd; Cyclamen-Homoaldehyd; Cyclamenaldehyd; Dupical; Oncidal; Adoxal; Melonal; Calypson; Anisaldehyd; Heliotropin; Cuminaldehyd; Scentenal; 3,6-Dimethylcyclohex-3-en-1-carbaldehyd; Satinaldehyd; Canthoxal; Vanillin; Ethylvanillin; Zimtaldehyd; und Mischungen davon.

3. Behandlungszusammensetzung nach Anspruch 1 oder 2, wobei das PRM, das den Rückstand bildete, eine Ketoneinheit umfasst,
vorzugsweise wobei das PRM ausgewählt ist aus der Gruppe bestehend aus: Nerolion; 4-(4-Methoxyphenyl) butan-2-on; 1-Naphthalen-2-ylethanon; Nectaryl; Trimofix O; Fleuramon; Delta-Damascon; Beta-Damascon; Alpha-Damascon; Methylionon; 2-Hexylcyclopent-2-en-1-on; Galbascon; und Mischungen davon.

4. Behandlungszusammensetzung nach einem der vorstehenden Ansprüche, wobei jede X-Gruppe unabhängig voneinander von zwei bis neun Kettenatome, mehr bevorzugt von zwei bis sechs Kettenatome, noch mehr bevorzugt, wobei wenigstens eines der Kettenatome ein Sauerstoffatom ist, noch mehr bevorzugt, wobei die X-Gruppe eine Vier-Kohlenstoff-Ethergruppe ist, am meisten bevorzugt, wobei die X-Gruppe $-CH_2-CH_2-CH_2-O-CH_2-$ ist, umfasst.

5. Behandlungszusammensetzung nach einem der vorstehenden Ansprüche, wobei, wenn $R^1$ als die einwertige Einheit vorhanden ist, die einwertige Einheit eine substituierte oder nicht-substituierte $C_1$-$C_{35}$-Alkylgruppe ist, vorzugsweise eine einwertige Einheit mit einem Molekulargewicht von 15 bis 101 Da, mehr bevorzugt ein substituiertes oder nicht-substituiertes $C_1$-$C_8$-Alkyl.

6. Behandlungszusammensetzung nach einem der vorstehenden Ansprüche, wobei, wenn $R^2$ als die einwertige Einheit vorhanden ist, die einwertige Einheit vorzugsweise ein substituiertes oder nicht-substituiertes, gesättigtes oder ungesättigtes $C_1$-$C_{70}$-Alkyl ist, mehr bevorzugt eine einwertige Einheit mit einem Molekulargewicht von 14 bis 186 Da, noch mehr bevorzugt ein substituiertes oder nicht-substituiertes, gesättigtes oder ungesättigtes $C_1$- bis $C_{12}$-Alkyl.

7. Behandlungszusammensetzung nach einem der vorstehenden Ansprüche, wobei der zweiwertige Substituent ausgewählt ist aus der Gruppe bestehend aus einem kondensierten Ring, einem spirocyclischen Ring, einem ungesättigten Substituenten, $=N(R^1)$, =O und =S.

8. Behandlungszusammensetzung nach einem der vorstehenden Ansprüche, wobei $R^3$ unabhängig voneinander ausgewählt ist aus einer einwertigen organischen Einheit, und vorzugsweise wobei $R^4$ unabhängig voneinander ausgewählt ist aus der Gruppe bestehend aus Wasserstoff und einer einwertigen organischen Einheit.

9. Behandlungszusammensetzung nach einem der vorstehenden Ansprüche, wobei wenigstens ein $R^2$ ein Bindungspunkt von Z an eine X-Gruppe ist,
vorzugsweise wobei $R^1$ H ist.

10. Flüssige Vormischungszusammensetzung, umfassend:

a) ein Pro-Duftstoff-Silikonpolymer, wie in einem der Ansprüche 1 bis 9 beschrieben,
wahlweise ferner umfassend einen oder mehrere freie Duftstoffrohmaterialien; oder
b) ein Vorläufersilikonpolymer und ein Duftstoffrohmaterial, das eine Einheit, ausgewählt aus der Gruppe bestehend aus einer Aldehydeinheit, einer Ketoneinheit und einer Kombination davon, umfasst,
wobei das Vorläufersilikonpolymer und das Duftstoffrohmaterial in der Lage sind, zu kondensieren, um des Pro-Duftstoff-Silikonpolymer nach einem der Ansprüche 1 bis 9 zu bilden; oder
c) eine Mischung davon.

11. Flüssige Vormischungszusammensetzung nach Anspruch 10, wobei die flüssige Vormischungszusammensetzung in der Form einer Öl-in-Wasser-Emulsion vorliegt,

vorzugsweise umfassend Tröpfchen, **gekennzeichnet durch** eine volumengewichtete durchschnittliche Teilchengröße von 100 nm bis 100 $\mu$m,
mehr bevorzugt von 1 $\mu$m bis 10 $\mu$m,
noch mehr bevorzugt von 1 $\mu$m bis 5 $\mu$m.

12. Flüssige Vormischungszusammensetzung nach Anspruch 10 oder 11, wobei, bezogen auf das Gewicht der flüssigen Vormischungszusammensetzung, das Vorläufersilikonpolymer in einem Gehalt von 1 % bis 99 %, vorzugsweise von 80 % bis 10 %, oder von 75 % bis 20 %, oder von 60 % bis 40 %, vorhanden ist, und

das Duftstoffrohmaterial in einem Gehalt von 1 % bis 50 %, vorzugsweise von 5 % bis 50 %, oder von 5 % bis 40 %,

oder von 5 % bis 30 %, oder von 5 % bis 20 %, oder von 5 % bis 15 %, oder von 5 % bis 10 %, vorhanden ist, vorzugsweise wobei das Gewichtsverhältnis des Vorläufersilikonpolymers und des Duftstoffrohmaterials von 99 : 1 bis 1 : 1, vorzugsweise von 50 : 1 bis 2 : 1, mehr bevorzugt von 10 : 1 bis 5 : 1, beträgt.

**13.** Flüssige Vormischungszusammensetzung nach einem der Ansprüche 10 bis 12, wobei das Vorläufersilikonpolymer wenigstens ein Radikal, ausgewählt aus der Gruppe bestehend aus Formel VI, Formel VII und Mischungen davon, umfasst,
wobei die Radikale von Formel VI und Formel VII die folgenden Strukturen aufweisen:

(Si) - X - Y Formel VI;

(Si) - X - $(Y - X)_n$ - (Si) Formel VII;

wobei "(Si) -" die Bindung an ein Si-Atom ist,
wobei der Index n 1 oder 2, vorzugsweise 1, ist,
wobei jede X-Gruppe über eine Si-C-Bindung mit "(Si)" kovalent verknüpft ist und eine unabhängig voneinander ausgewählte zweiwertige organische Gruppe ist, umfassend von zwei bis zwölf Kettenatome, und
wobei jedes Y unabhängig voneinander eine einwertige oder zweiwertige Einheit gemäß Formel VIII ist,
wobei Formel VIII die folgende Struktur aufweist:

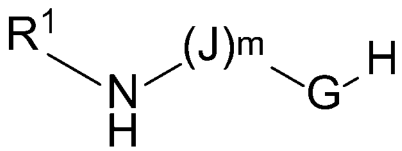

Formel VIII,

wobei G ausgewählt ist aus der Gruppe bestehend aus Sauerstoff oder Schwefel, vorzugsweise Sauerstoff,
wobei der Index m 2 ist,
wobei $R^1$ ausgewählt ist aus H, einer einwertigen Einheit mit einem Molekulargewicht zwischen 15 und 495 Da, oder einem Bindungspunkt von Y an eine X-Gruppe,
wobei, wenn $R^1$ als die einwertige Einheit vorhanden ist, die einwertige Einheit vorzugsweise eine substituierte oder nicht-substituierte $C_1$-$C_{35}$-Alkylgruppe ist, mehr bevorzugt eine einwertige Einheit mit einem Molekulargewicht von 15 bis 101 Da, noch mehr bevorzugt ein substituiertes oder nicht-substituiertes $C_1$-$C_8$-Alkyl,
vorzugsweise wobei $R^1$ H ist,
wobei jedes J unabhängig voneinander $C(R^2)_2$ ist,
wobei $R^2$ unabhängig voneinander ausgewählt ist aus der Gruppe bestehend aus H, einer einwertigen Einheit mit einem Molekulargewicht zwischen 14 und 990 Da, oder einem Bindungspunkt von Y an eine X-Gruppe,
wobei, wenn $R^2$ als die einwertige Einheit vorhanden ist, die einwertige Einheit vorzugsweise ein substituiertes oder nicht-substituiertes, gesättigtes oder ungesättigtes $C_1$-$C_{70}$-Alkyl ist, mehr bevorzugt eine einwertige Einheit mit einem Molekulargewicht von 14 bis 186 Da, noch mehr bevorzugt ein substituiertes oder nicht-substituiertes, gesättigtes oder ungesättigtes $C_1$- bis $C_{12}$-Alkyl,
vorzugsweise wobei jedes $R^2$ H ist,
mit der Maßgabe, dass eine erste Einheit und eine zweite Einheit wahlweise zusammengenommen werden können, wo durchführbar, als ein zweiwertiger Substituent,
wobei die erste Einheit eine erste $R^2$-Gruppe ist, und
wobei die zweite Einheit ausgewählt ist aus der Gruppe, bestehend aus einer zweiten $R^2$-Gruppe, der $R^1$-Gruppe und einem einwertigen Substituenten der $R^1$-Gruppe,
vorzugsweise wobei der zweiwertige Substituent ausgewählt ist aus der Gruppe bestehend aus einem kondensierten Ring, einem spirocyclischen Ring, einem ungesättigten Substituenten, $=N(R^2)$, =O und =S,
mit der Maßgabe, dass wenigstens eines und nicht mehr als zwei von $R^1$, ein beliebiges $R^2$ oder Kombinationen davon ein Bindungspunkt von Y an eine X-Gruppe ist.

**14.** Verfahren zum Herstellen einer Behandlungszusammensetzung, das Verfahren umfassend die Schritte:

Bereitstellen einer Basiszusammensetzung, wobei die Basiszusammensetzung ein Behandlungshilfsmittel umfasst;
Kombinieren der Basiszusammensetzung mit einem oder mehreren der folgenden:

a) einem Pro-Duftstoff-Silikonpolymer, wie in einem der Ansprüche 1 bis 9 beschrieben;
b) einem Vorläufersilikonpolymer, wie in einem der Ansprüche 10 bis 13 beschrieben, und einem Duftstoffrohmaterial, das eine Einheit, ausgewählt aus der Gruppe bestehend aus einer Aldehydeinheit, einer Ketoneinheit und einer Kombination davon, umfasst,
vorzugsweise wobei das Vorläufersilikonpolymer und das Duftstoffrohmaterial zusammen als eine flüssige Vormischungszusammensetzung, vorzugsweise als eine flüssige Vormischungszusammensetzung in der Form einer Emulsion, bereitgestellt werden; oder
c) einer Mischung davon.

**15.** Verwendung eines Pro-Duftstoff-Silikonpolymers, wie in einem der Ansprüche 1 bis 9 beschrieben, als ein Duftstoffmittel, wahlweise auf einer Oberfläche, vorzugsweise auf einem Stoff, wahlweise formuliert in einer Behandlungszusammensetzung, wobei die Behandlungszusammensetzung eine Behandlungszusammensetzung nach einem der Ansprüche 1 bis 9 sein kann.

## Revendications

**1.** Composition de traitement comprenant un adjuvant de traitement et un polymère de silicone pro-fragrance,

dans laquelle le polymère de silicone pro-fragrance comprend un squelette de silicone, un groupe lieur organique comprenant un atome de carbone lié à un atome de silicium du squelette de silicone, et une fraction hétérocyclique liée au groupe lieur organique,
la fraction hétérocyclique comprenant cinq chaînons de cycle, les chaînons de cycle comprenant :

un premier chaînon de cycle qui est un atome d'azote ;
un deuxième chaînon de cycle qui est un atome de carbone,
dans laquelle le deuxième chaînon de cycle fait partie d'un résidu d'une matière première de parfum (« PRM »),
dans laquelle la PRM qui a formé le résidu comprend une fraction choisie dans le groupe constitué d'une fraction aldéhyde, une fraction cétone, et une combinaison de celles-ci ;
un troisième chaînon de cycle choisi dans le groupe constitué d'un atome d'oxygène ou un atome de soufre, de préférence un atome d'oxygène,
dans laquelle le deuxième chaînon de cycle est directement lié au premier chaînon de cycle et au troisième chaînon de cycle ;
et dans laquelle le polymère de silicone pro-fragrance comprend au moins un radical choisi dans le groupe constitué de la formule (I), formule (II), ou un mélange de celles-ci,
dans laquelle les radicaux de formule I et de formule II ont les structures suivantes :

$$(Si) - X - Z \qquad \text{Formule I}$$

$$(Si) - X - (Z - X)_n - (Si) \qquad \text{Formule II}$$

dans laquelle « (Si) - » représente la liaison à un atome de Si,
dans laquelle chaque groupe X est le groupe lieur organique et est un groupe fraction organique divalente choisi indépendamment comprenant deux à douze atomes de chaîne, où le groupe X est lié à un atome de silicium non terminal ;
dans laquelle le groupe Z comprend la fraction hétérocyclique, et
dans laquelle l'indice n est 1 ou 2, de préférence 1 ;
de préférence dans laquelle l'au moins un radical a la structure de formule I ;
dans laquelle chaque Z est une fraction hétérocyclique à cinq chaînons monovalente ou divalente selon la formule IV,
dans laquelle la formule IV a la structure suivante :

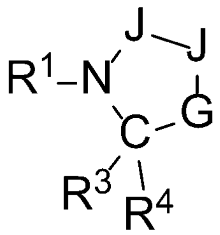

Formule IV,

dans laquelle G est choisi indépendamment dans le groupe constitué d'oxygène ou soufre, $R^1$ est choisi parmi H, une fraction monovalente d'un poids moléculaire de 15 à 495 Da, ou un point d'attache de Z à un groupe X, de préférence $R^1$ est H ou un point d'attache de Z à un groupe X, et la fraction -$C(R^3)(R^4)$- est le résidu de la PRM, de préférence une PRM qui comprend de 3 à 34 atomes de carbone,

de préférence où G est oxygène,

et dans laquelle chaque J est indépendamment $C(R^2)_2$, où chaque $R^2$ est choisi indépendamment parmi H, une fraction monovalente d'un poids moléculaire de 14 à 990 Da, ou un point d'attache de Z à un groupe X, de préférence $R^2$ est H ou un point d'attache de Z à un groupe X ;

à condition qu'une première unité et une deuxième unité puissent éventuellement être prises ensemble, lorsque cela est possible, en tant que substituant divalent, la première unité étant un premier groupe $R^2$, et la deuxième unité étant choisie dans le groupe constitué d'un deuxième groupe $R^2$, le groupe $R^1$, et un substituant monovalent du groupe $R^1$,

à condition qu'au moins l'un et pas plus de deux parmi $R^1$, un quelconque $R^2$, ou des mélanges de ceux-ci soient un point d'attache de Z à un groupe X.

**2.** Composition de traitement selon la revendication 1, dans laquelle la PRM qui a formé le résidu comprend une fraction aldéhyde,
de préférence dans laquelle la PRM est choisie dans le groupe constitué de : méthyl nonyl acétaldéhyde ; benzaldéhyde ; floralozone ; isocyclocitral ; triplal (ligustral) ; précylcemone B ; lilial ; décylaldéhyde ; aldéhyde undécylénique ; homoaldéhyde de cyclamen ; aldéhyde de cyclamen ; dupical ; oncidal ; adoxal ; melonal ; calypsone ; aldéhyde anisique ; héliotropine ; aldéhyde cuminique ; scenténal ; 3,6-diméthylcyclohex-3-ène-1-carbaldéhyde ; satinaldéhyde ; canthoxal ; vanilline ; éthyl-vanilline ; aldéhyde cinnamique ; et mélanges de ceux-ci.

**3.** Composition de traitement selon la revendication 1 ou la revendication 2, dans laquelle la PRM qui a formé le résidu comprend une fraction cétone,
de préférence dans laquelle la PMR est choisie dans le groupe constitué de : nérolione ; 4-(4-méthoxyphényl)butan-2-one ; 1-naphtalèn-2-yléthanone ; nectaryle ; trimofix O ; fleuramone ; delta-damascone ; bêta-damascone ; alpha-damascone ; méthyl ionone ; 2-hexylcyclopent-2-èn-1-one ; galbascone ; et mélanges de ceux-ci.

**4.** Composition de traitement selon l'une quelconque revendication précédente, dans laquelle chaque groupe X comprend indépendamment deux à neuf atomes de chaîne, plus préférablement deux à six atomes de chaîne, encore plus préférablement dans laquelle au moins l'un des atomes de chaîne est un atome d'oxygène, encore plus préférablement dans laquelle le groupe X est un groupe éther à quatre atomes de carbone, le plus préférablement dans laquelle le groupe X est $-CH_2-CH_2-CH_2-O-CH_2-$.

**5.** Composition de traitement selon l'une quelconque revendication précédente, dans laquelle lorsque $R^1$ est présent en tant que fraction monovalente, la fraction monovalente est un groupe alkyle en $C_1$ à $C_{35}$ substitué ou non substitué, de préférence une fraction monovalente d'un poids moléculaire de 15 à 101 Da, plus préférablement un alkyle en $C_1$ à $C_8$ substitué ou non substitué.

**6.** Composition de traitement selon l'une quelconque revendication précédente, dans laquelle lorsque $R^2$ est présent en tant que fraction monovalente, la fraction monovalente est de préférence un alkyle en $C_1$ à $C_{70}$ substitué ou non substitué, saturé ou insaturé, plus préférablement une fraction monovalente d'un poids moléculaire de 14 à 186 Da, encore plus préférablement un alkyle en $C_1$ à $C_{12}$ substitué ou non substitué, saturé ou insaturé.

**7.** Composition de traitement selon l'une quelconque revendication précédente, dans laquelle le substituant divalent est choisi dans le groupe constitué d'un cycle fusionné, un cycle spirocyclique, un substituant insaturé, $=N(R^1)$, =O, et =S.

**8.** Composition de traitement selon l'une quelconque revendication précédente, dans laquelle $R^3$ est choisi indépendamment parmi une fraction organique monovalente, et de préférence dans laquelle $R^4$ est choisi indépendamment dans le groupe constitué d'hydrogène et une fraction organique monovalente.

**9.** Composition de traitement selon l'une quelconque revendication précédente, dans laquelle au moins un $R^2$ est un point d'attache de Z à un groupe X,
de préférence dans laquelle $R^1$ est H.

**10.** Composition de prémélange liquide comprenant :

a) un polymère de silicone pro-fragrance tel que décrit dans l'une quelconque des revendications 1 à 9, éventuellement, comprenant en outre une ou plusieurs matières premières de parfum libres ; ou
b) un polymère de silicone précurseur, et une matière première de parfum qui comprend une fraction choisie dans le groupe constitué d'une fraction aldéhyde, une fraction cétone, et une combinaison de celles-ci,
dans laquelle le polymère de silicone précurseur et la matière première de parfum sont capables de se condenser pour former le polymère de silicone pro-fragrance tel que décrit dans l'une quelconque des revendications 1 à 9 ; ou
c) un mélange de ceux-ci.

**11.** Composition de prémélange liquide selon la revendication 10, dans laquelle la composition de prémélange liquide se présente sous la forme d'une émulsion huile-dans-eau,

de préférence comprenant des gouttelettes **caractérisées par** une taille moyenne de particule pondérée en volume de 100 nm à 100 $\mu$m,
plus préférablement de 1 $\mu$m à 10 $\mu$m,
encore plus préférablement de 1 $\mu$m à 5 $\mu$m.

**12.** Composition de prémélange liquide selon la revendication 10 ou la revendication 11, dans laquelle, en poids de la composition de prémélange liquide,

le polymère de silicone précurseur est présent à un taux de 1 % à 99 %, de préférence de 80 % à 10 %, ou de 75 % à 20 %, ou de 60 % à 40 %, et
la matière première de parfum est présente à un taux de 1 % à 50 %, de préférence de 5 % à 50 %, ou de 5 % à 40 %, ou de 5 % à 30 %, ou de 5 % à 20 %, ou de 5 % à 15 %, ou de 5 % à 10 %,
de préférence dans laquelle le rapport en poids entre le polymère de silicone précurseur et la matière première de parfum est de 99:1 à 1:1, de préférence de 50:1 à 2:1, plus préférablement de 10:1 à 5:1.

**13.** Composition de prémélange liquide selon l'une quelconque des revendications 10 à 12, dans laquelle le polymère de silicone précurseur comprend au moins un radical choisi dans le groupe constitué de la formule VI, la formule VII, et des mélanges de celles-ci,
dans laquelle les radicaux de formule VI et de formule VII ont les structures suivantes :

(Si) - X - Y    Formule VI ;

(Si) - X - $(Y - X)_n$ - (Si)    Formule VII ;

dans laquelle « (Si) - » est la liaison à un atome de Si,
dans laquelle l'indice n est 1 ou 2, de préférence 1,
dans laquelle chaque groupe X est lié de manière covalente à « (Si) » par l'intermédiaire d'une liaison Si-C et est un groupe organique divalent choisi indépendamment comprenant de deux à douze atomes de chaîne, et dans lequel chaque Y est indépendamment une fraction monovalente ou divalente selon la formule VIII,
dans laquelle la formule VIII a la structure suivante :

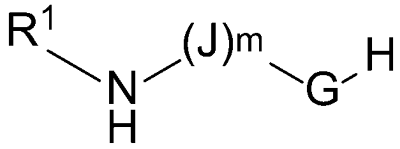

Formule VIII,

dans laquelle G est choisi dans le groupe constitué d'oxygène ou soufre, de préférence oxygène,
dans laquelle l'indice m est 2,
dans laquelle $R^1$ est choisi parmi H, une fraction monovalente d'un poids moléculaire compris entre 15 et 495 Da, ou un point d'attache de Y à un groupe X,
dans laquelle, lorsque $R^1$ est présent sous la forme d'une fraction monovalente, la fraction monovalente est de préférence un groupe alkyle en $C_1$ à $C_{35}$ substitué ou non substitué, plus préférablement une fraction monovalente d'un poids moléculaire de 15 à 101 Da, encore plus préférablement un alkyle en $C_1$ à $C_8$ substitué ou non substitué,
de préférence où $R^1$ est H,
dans laquelle chaque J est indépendamment $C(R^2)_2$
dans laquelle chaque $R^2$ est choisi indépendamment dans le groupe constitué de H, une fraction monovalente d'un poids moléculaire compris entre 14 et 990 Da, ou un point d'attache de Y à un groupe X,
dans laquelle, lorsque $R^2$ est présent sous la forme de la fraction monovalente, la fraction monovalente est de préférence un alkyle en $C_1$ à $C_{70}$ substitué ou non substitué, saturé ou insaturé, plus préférablement une fraction monovalente d'un poids moléculaire de 14 à 186 Da, encore plus préférablement un alkyle en $C_1$ à $C_{12}$ substitué ou non substitué, saturé ou insaturé,
de préférence dans laquelle chaque $R^2$ est H,
à condition qu'une première unité et une deuxième unité puissent éventuellement être prises ensemble, lorsque cela est possible, en tant que substituant divalent,
la première unité étant un premier groupe $R^2$, et
la deuxième unité étant choisie dans le groupe constitué d'un deuxième groupe $R^2$, le groupe $R^1$, et un substituant monovalent du groupe $R^1$,
de préférence le substituant divalent étant choisi dans le groupe constitué d'un cycle fusionné, un cycle spirocyclique, un substituant insaturé, $=N(R^2)$, =O, et =S,
à condition qu'au moins un et pas plus de deux de $R^1$, un quelconque $R^2$, ou des combinaisons de ceux-ci soit un point d'attache de Y à un groupe X.

**14.** Procédé de fabrication d'une composition de traitement, le procédé comprenant les étapes consistant à :

la fourniture d'une composition de base, dans lequel la composition de base comprend un adjuvant de traitement ;
la combinaison de la composition de base avec un ou plusieurs des éléments suivants :

a) un polymère de silicone pro-fragrance tel que décrit dans l'une quelconque des revendications 1 à 9 ;
b) un polymère de silicone précurseur tel que décrit dans l'une quelconque des revendications 10 à 13, et une matière première de parfum qui comprend une fraction choisie dans le groupe constitué d'une fraction aldéhyde, une fraction cétone, et une combinaison de celles-ci,
de préférence dans lequel le polymère de silicone précurseur et la matière première de parfum sont fournis ensemble sous la forme d'une composition de prémélange liquide, de préférence une composition de prémélange liquide sous la forme d'une émulsion ; ou
c) un mélange de ceux-ci.

**15.** Utilisation d'un polymère de silicone pro-fragrance tel que décrit dans l'une quelconque des revendications 1 à 9 en tant qu'agent parfumant, éventuellement sur une surface, de préférence sur une étoffe, éventuellement formulé dans une composition de traitement, la composition de traitement pouvant être une composition de traitement selon l'une quelconque des revendications 1 à 9.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9946318 A1
- US 6020303 A
- US 6060443 A
- US 7169744 B


### Non-patent literature cited in the description


- Perfume and Flavor Chemicals. Steffen Arctander Allured Pub. Co, 1994, vol. I,II
- **MILLER, P. M** ; **LAMPARSKY, D.** Perfumes: Art, Science and Technology. Blackie Academic and Professional, 1994
- *CHEMICAL ABSTRACTS*, 7647-01-1
- *CHEMICAL ABSTRACTS*, 67-63-0
- *CHEMICAL ABSTRACTS*, 103-72-0
- *CHEMICAL ABSTRACTS*, 90-02-8
- **GRIFFIN, W. C**. Calculation of HLB values of Nonionic Surfactants. *J. Soc. Cosmet. Chem*, 1954, vol. 5, 249-256
- McCutcheon's Emulsifiers and Detergents. MC Publishing Co, 2004